# EUROPEAN PATENT SPECIFICATION

(11) **EP 1 301 605 B1**
(45) Date of publication and mention of the grant of the patent: **30.11.2005**
(21) Application number: 01956747.8
(22) Date of filing: 19.07.2001
(51) Int. Cl.: C12N 15/62, C12N 15/12, C07K 14/05, C07K 16/18, A61K 38/17, G01N 33/50

(54) **NK CELLS ACTIVATING RECEPTORS AND THEIR THERAPEUTIC AND DIAGNOSTIC USES**
NK ZELLEN-AKTIVIERENDE REZEPTOREN UND DEREN THERAPEUTISCHE UND DIAGNOSTISCHE VERWENDUNGEN
RECEPTEURS ACTIVANT LES CELLULES NK ET LEURS EMPLOIS THERAPEUTIQUES ET DIAGNOSTIQUES

(30) Priority: 20.07.2000 IL 13741900
(43) Date of publication of application: 16.04.2003
(73) Proprietor: YISSUM RESEARCH DEVELOPMENT COMPANY OF THE HEBREW UNIVERSITY OF JERUSALEM, 91390 Jerusalem (IL); BEN-GURION UNIVERSITY OF THE NEGEV, 84105 Beer-Sheva (IL)
(72) Inventor: MANDELBOIM, Ofer, 73142 Shoam (IL); PORGADOR, Angel, 85338 Lehavim (IL)
(74) Representative: Vossius & Partner
(86) International application number: PCT/IL2001/000664
(87) International publication number: WO 2002/008287

(56) References cited:
- WO-A-95/09011
- WO-A-96/25953
- WO-A-96/26265
- WO-A-96/36720
- WO-A-99/23867
- PESSINO A ET AL: "MOLECULAR CLONING OF NKP46: A NOVEL MEMBER OF THE IMMUNOGLOBULIN SUPERFAMILY INVOLVED IN TRIGGERING OF NATURAL CYTOTOXICITY" JOURNAL OF EXPERIMENTAL MEDICINE, TOKYO, JP, vol. 188, no. 5, 7 September 1998 (1998-09-07), pages 953-960, XP000953037 ISSN: 0022-1007 cited in the application
- CANTONI C ET AL: "NKp44, a triggering receptor involved in tumor cell lysis by activated human natural killer cells, is a novel member of the immunoglobulin superfamily" JOURNAL OF EXPERIMENTAL MEDICINE, TOKYO, JP, vol. 189, no. 5, 1 March 1999 (1999-03-01), pages 787-795, XP002167574 ISSN: 0022-1007 cited in the application
- MANDELBOIM OFER ET AL: "Human CD16 as a lysis receptor mediating direct natural killer cell cytotoxicity." PROCEEDINGS OF THE NATIONAL ACADEMY OF SCIENCES OF THE UNITED STATES, vol. 96, no. 10, 11 May 1999 (1999-05-11), pages 5640-5644, XP002191492 May 11, 1999 ISSN: 0027-8424 cited in the application
- DATABASE EMBL [Online] 22 September 1998 (1998-09-22) BIASSONI R.: " Homo sapiens mRNA for NK receptor (NKp46), isoform b" retrieved from EBI Database accession no. AJ006121 XP002191532
- PENDE D ET AL: "Identification and molecular characterization of NKp30, a novel triggering receptor involved in natural cytotoxicity mediated by human natural killer cells" JOURNAL OF EXPERIMENTAL MEDICINE, TOKYO, JP, vol. 190, no. 10, 15 November 1999 (1999-11-15), pages 1505-1516, XP002167575 ISSN: 0022-1007
- MAZANEC MARY B ET AL: "Intracellular neutralization of influenza virus by immunoglobulin A anti-hemagglutinin monoclonal antibodies." JOURNAL OF VIROLOGY, vol. 69, no. 2, 1995, pages 1339-1343, XP002201819 ISSN: 0022-538X
- MAZANEC M B ET AL: "INTRACELLULAR NEUTRALIZATION OF VIRUS BY IMMUNOGLOBULIN A ANTIBODIES" PROCEEDINGS OF THE NATIONAL ACADEMY OF SCIENCES OF THE UNITED STATES, vol. 89, no. 15, 1992, pages 6901-6905, XP002201820 1992 ISSN: 0027-8424
- DATABASE EMBL [Online] 22 September 1998 (1998-09-22) BIASSONI R.: "Homo sapiens mRNA for NK receptor (NKp46) isoform c" retrieved from EBI Database accession no. AJ006122 XP002191533
- DATABASE EMBL [Online] 22 September 1998 (1998-09-22) BIASSONI R.: " Homo sapiens mRNA for NK receptor (NKp46), isoform d" retrieved from EBI Database accession no. AJ006123 XP002191534
- SIVORI SIMONA ET AL: "NKp46 is the major triggering receptor involved in the natural cytotoxicity of fresh or cultured human NK cells. Correlation between surface density of NKp46 and natural cytotoxicity against autologous, allogeneic or xenogeneic target cells." EUROPEAN JOURNAL OF IMMUNOLOGY, vol. 29, no. 5, May 1999 (1999-05), pages 1656-1666, XP001064097 ISSN: 0014-2980
- FALCO, MICHELA ET AL: "Identification of the rat homologue of the human NKp46 triggering receptor" IMMUNOL. LETT. (1999), 68(2,3), 411-414, XP001064058
- MANDELBOIM OFER ET AL: "Recognition of haemagglutinins on virus-infected cells by NKp46 activates lysis by human NK cells." NATURE (LONDON), vol. 409, no. 6823, 2001, pages 1055-1060, XP002191494 ISSN: 0028-0836

### SEQUENCE LISTING

<110> Yissum research&BGU
<120> sialicacid based binding of NKp46 and NKp44 to viral HA
<130> 11454b
<140>
   <141>
<170> PatentIn Ver. 2.1
<210> 1
   <211> 762
   <212> DNA
   <213> homo sapiens
<400> 1
   atgtcttcca cactccctgc cctgctctgc gtcgggctgt gtctgagtca gaggatcagc 60
   gcccagcagc agactctccc aaaaccgttc atctgggccg agccccattt catggttcca 120
   aaggaaaagc aagtgaccat ctgttgccag ggaaattatg ggctgttgaa ataccagctg 180
   cactttgaag gaagcctttt tgccgtggac agaccaaaac cccctgagcg gattaacaaa 240
   gtcaaattct acatcccgga catgaactcc cgcatggcag ggcaatacag ctgcatctac 300
   cgggttgggg agctctggtc agagcccagc aacttgctgg atctggtggt aacagaaatg 360
   tatgacacac ccaccctctc ggttcatcct ggacccgaag tgatctcggg agagaaggtg 420
   accttctact gccgtctaga cactgcaaca agcatgttct tactgctcaa ggagggaaga 480
   tccagccacg tacagcgcgg atacgggaag gtccaggcgg agttccccct gggccctgtg 540
   accacagccc accgagggac ataccgatgt tttggctcct ataacaacca tgcctggtct 600
   ttccccagtg agccagtgag gctcctggtc acaggcgaca ttgagaacac cagccttgca 660
   cctgaagacc ccacctttcc tgcagacact tggggcacct accttttaag cacagagacg 720
   ggactccaga aagaccatgc cctctgggat cacactgccc ag                    762
<210> 2
   <211> 705
   <212> DNA
   <213> homo sapiens
<400> 2
   gatccggagc ccaaatcttc tgacaaaact cacacatgcc caccgtgccc agcacctgaa 60
   ttcgagggtg caccgtcagt cttcctcttc cccccaaaac ccaaggacac cctcatgatc 120
   tcccggaccc ctgaggtcac atgcgtggtg gtggacgtga gccacgaaga ccctgaggtc 180
   aagttcaact ggtacgtgga cggcgtggag gtgcataatg ccaagacaaa gccgcgggag 240
   gagcagtaca acagcacgta ccgtgtggtc agcgtcctca ccgtcctgca ccaggactgg 300
   ctgaatggca aggagtacaa gtgcaaggtc tccaacaaag ccctcccagc ccccatcgag 360
   aaaaccatct ccaaagccaa agggcagccc cgagagccac aggtgtacac cctgccccca 420
   tcccgggatg agctgaccaa gaaccaggtc agcctgacct gcctggtcaa aggcttctat 480
   cccagcgaca tcgccgtgga gtgggagagc aatgggcagc cggagaacaa ctacaagacc 540
   acgcctcccg tgctggactc cgacggctcc ttcttcctct acagcaagct caccgtggac 600
   aagagcaggt ggcagcaggg gaacgtcttc tcatgctccg tgatgcatga ggctctgcac 660
   aaccactaca cgcagaagag cctctccctg tctccgggta aatga 705
<210> 3
   <211> 1467
   <214> DNA
   <213> homo sapiens
<400> 3
   atgtcttcca cactccctgc cctgctctgc gtcgggctgt gtctgagtca gaggatcagc 60
   gcccagcagc agactctccc aaaaccgttc atctgggccg agccccattt catggttcca 120
   aaggaaaagc aagtgaccat ctgttgccag ggaaattatg gggctgttga ataccagctg 180
   cactttgaag gaagcctttt tgccgtggac agaccaaaac cccctgagcg gattaacaaa 240
   gtcaaattct acatcccgga catgaactcc cgcatggcag ggcaatacag ctgcatctat 300
   cgggttgggg agctctggtc agagcccagc aacttgctgg atctggtggt aacagaaatg 360
   tatgacacac ccaccctctc ggttcatcct ggacccgaag tgatctcggg agagaaggtg 420
   accttctact gccgtctaga cactgcaaca agcatgttct tactgctcaa ggagggaaga 480
   tccagccacg tacagcgcgg atacgggaag gtccaggcgg agttccccct gggccctgtg 540
   accacagccc accgagggac ataccgatgt tttggctcct ataacaacca tgcctggtct 600
   ttccccagtg agccagtgaa gctcctggtc acaggcgaca ttgagaacac cagccttgca 660
   cctgaagacc ccacctttcc tgcagacact tggggcacct accttttaac cacagagacg 720
   ggactccaga aagaccatgc cctctgggat cacactgccc aggatccgga gcccaaatct 780
   tctgacaaaa ctcacacatg cccaccgtgc ccagcacctg aattcgaggg tgcaccgtca 840
   gtcttcctct tccccccaaa acccaaggac accctcatga tctcccggac ccctgaggtc 900
   acatgcgtgg tggtggacgt gagccacgaa gaccctgagg tcaagttcaa ctggtacgtg 960
   gacggcgtgg aggtgcataa tgccaagaca aagccgcggg aggagcagta caacagcacg 1020
   taccgtgtgg tcagcgtcct caccgtcctg caccaggact ggctgaatgg caaggagtac 1080
   aagtgcaagg tctccaacaa agccctccca gcccccatcg agaaaaccat ctccaaagcc 1140
   aaagggcagc cccgagagcc acaggtgtac accctgcccc catcccggga tgagctgacc 1200
   aagaaccagg tcagcctgac ctgcctggtc aaaggcttct atcccagcga catcgccgtg 1260
   gagtgggaga gcaatgggca gccggagaac aactacaaga ccacgcctcc cgtgctggac 1320
   tccgacggct ccttcttcct ctacagcaag ctcaccgtgg acaagagcag gtggcagcag 1380
   gggaacgtct tctcatgctc cgtgatgcat gaggctctgc acaaccacta cacgcagaag 1440
   agcctctccc tgtctccggg taaatga 1467
<210> 4
   <211> 254
   <212> PRT
   <213> homo sapiens
<400> 4
   Met Ser Ser Thr Leu Pro Ala Leu Leu Cys Val Gly Leu Cys Leu Ser
   1 5 10 15
   Gln Arg Ile Ser Ala Gln Gln Gln Thr Leu Pro Lys Pro Phe Ile Trp
   20 25 30
   Ala Glu Pro His Phe Met Val Pro Lys Glu Lys Gln Val Thr Ile Cys
   35 40 45
   Cys Gln Gly Asn Tyr Gly Ala Val Glu Tyr Gln Leu His Phe Glu Gly
   50 55 60
   Ser Leu Phe Ala Val Asp Arg Pro Lys Pro Pro Glu Arg Ile Asn Lys
   65 70 75 80
   Val Lys Phe Tyr Ile Pro Asp Met Asn Ser Arg Met Ala Gly Gln Tyr
   85 90 95
   Ser Cys Ile Tyr Arg Val Gly Glu Leu Trp Ser Glu Pro Ser Asn Leu
   100 105 110
   Leu Asp Leu Val Val Thr Glu Met Tyr Asp Thr Pro Thr Leu Ser Val
   115 120 125
   His Pro Gly Pro Glu Val Ile Ser Gly Glu Lys Val Thr Phe Tyr Cys
   130 135 140
   Arg Leu Asp Thr Ala Thr Ser Met Phe Leu Leu Leu Lys Glu Gly Arg
   145 150 155 160
   Ser Ser His Val Gln Arg Gly Tyr Gly Lys Val Gln Ala Glu Phe Pro
   165 170 175
   Leu Gly Pro Val Thr Thr Ala His Arg Gly Thr Tyr Arg Cys Phe Gly
   180 185 190
   Ser Tyr Asn Asn His Ala Trp Ser Phe Pro Ser Glu Pro Val Lys Leu
   195 200 205
   Leu Val Thr Gly Asp Ile Glu Asn Thr Ser Leu Ala Pro Glu Asp Pro
   210 215 220
   Thr Phe Pro Ala Asp Thr Trp Gly Thr Tyr Leu Leu Thr Thr Glu Thr
   225 230 235 240
   Gly Leu Gln Lys Asp His Ala Leu Trp Asp His Thr Ala Gln
   245 250
<210> 5
   <211> 234
   <212> PRT
   <213> homo sapiens
<400> 5
   Asp Pro Glu Pro Lys Ser Ser Asp Lys Thr His Thr Cys Pro Pro Cys
    1               5                  10                  15
   Pro Ala Pro Glu Phe Glu Gly Ala Pro Ser Val Phe Leu Phe Pro Pro
               20                  25                  30
   Lys Pro Lys Asp Thr Leu Met Ile Ser Arg Thr Pro Glu Val Thr Cys
           35                  40                  45
   Val Val Val Asp Val Ser His Glu Asp Pro Glu Val Lys Phe Asn Trp
       50                  55                  60
   Tyr Val Asp Gly Val Glu Val His Asn Ala Lys Thr Lys Pro Arg Glu
   65                  70                  75                  80
   Glu Gln Tyr Asn Ser Thr Tyr Arg Val Val Ser Val Leu Thr Val Leu
                   85                  90                  95
   His Gln Asp Trp Leu Asn Gly Lys Glu Tyr Lys Cys Lys Val Ser Asn
               100                 105                 110
   Lys Ala Leu Pro Ala Pro Ile Glu Lys Thr Ile Ser Lys Ala Lys Gly
           115                 120                 125
   Gln Pro Arg Glu Pro Gln Val Tyr Thr Leu Pro Pro Ser Arg Asp Glu
       130                 135                 140
   Leu Thr Lys Asn Gln Val Ser Leu Thr Cys Leu Val Lys Gly Phe Tyr
   145                 150                 155                 160
   Pro Ser Asp Ile Ala Val Glu Trp Glu Ser Asn Gly Gln Pro Glu Asn
                   165                 170                 175
   Asn Tyr Lys Thr Thr Pro Pro Val Leu Asp Ser Asp Gly Ser Phe Phe
               180                 185                 190
   Leu Tyr Ser Lys Leu Thr Val Asp Lys Ser Arg Trp Gln Gln Gly Asn
           195                 200                 205
   Val Phe Ser Cys Ser Val Met His Glu Ala Leu His Asn His Tyr Thr
       210                 215                 220
   Gln Lys Ser Leu Ser Leu Ser Pro Gly Lys
   225                 230
<210> 6
   <211> 488
   <212> PRT
   <213> homo sapiens
<400> 6
   Met Ser Ser Thr Leu Pro Ala Leu Leu Cys Val Gly Leu Cys Leu Ser
    1               5                  10                  15
   Gln Arg Ile Ser Ala Gln Gln Gln Thr Leu Pro Lys Pro Phe Ile Trp
               20                  25                  30
   Ala Glu Pro His Phe Met Val Pro Lys Glu Lys Gln Val Thr Ile Cys
           35                  40                  45
   Cys Gln Gly Asn Tyr Gly Ala Val Glu Tyr Gln Leu His Phe Glu Gly
       50                  55                  60
   Ser Leu Phe Ala Val Asp Arg Pro Lys Pro Pro Glu Arg Ile Asn Lys
   65                  70                  75                  80
   Val Lys Phe Tyr Ile Pro Asp Met Asn Ser Arg Met Ala Gly Gln Tyr
                   85                  90                  95
   Ser Cys Ile Tyr Arg Val Gly Glu Leu Trp Ser Glu Pro Ser Asn Leu
   100 105 110
   Leu Asp Leu Val Val Thr Glu Met Tyr Asp Thr Pro Thr Leu Ser Val
   115 120 125
   His Pro Gly Pro Glu Val Ile Ser Gly Glu Lys Val Thr Phe Tyr Cys
   130 135 140
   Arg Leu Asp Thr Ala Thr Ser Met Phe Leu Leu Leu Lys Glu Gly Arg
   145 150 155 160
   Ser Ser His Val Gln Arg Gly Tyr Gly Lys Val Gln Ala Glu Phe Pro
   165 170 175
   Leu Gly Pro Val Thr Thr Ala His Arg Gly Thr Tyr Arg Cys Phe Gly
   180 185 190
   Ser Tyr Asn Asn His Ala Trp Ser Phe Pro Ser Glu Pro Val Lys Leu
   195 200 205
   Leu Val Thr Gly Asp Ile Glu Asn Thr Ser Leu Ala Pro Glu Asp Pro
   210 215 220
   Thr Phe Pro Ala Asp Thr Trp Gly Thr Tyr Leu Leu Thr Thr Glu Thr
   225 230 235 240
   Gly Leu Gln Lys Asp His Ala Leu Trp Asp His Thr Ala Gln Asp Pro
   245 250 255
   Glu Pro Lys Ser Ser Asp Lys Thr His Thr Cys Pro Pro Cys Pro Ala
   260 265 270
   Pro Glu Phe Glu Gly Ala Pro Ser Val Phe Leu Phe Pro Pro Lys Pro
   275 280 285
   Lys Asp Thr Leu Met Ile Ser Arg Thr Pro Glu Val Thr Cys Val Val
   290 295 300
   Val Asp Val Ser His Glu Asp Pro Glu Val Lys Phe Asn Trp Tyr Val
   305 310 315 320
   Asp Gly Val Glu Val His Asn Ala Lys Thr Lys Pro Arg Glu Glu Gln
   325 330 335
   Tyr Asn Ser Thr Tyr Arg Val Val Ser Val Leu Thr Val Leu His Gln
   340 345 350
   Asp Trp Leu Asn Gly Lys Glu Tyr Lys Cys Lys Val Ser Asn Lys Ala
   355 360 365
   Leu Pro Ala Pro Ile Glu Lys Thr Ile Ser Lys Ala Lys Gly Gln Pro
   370 375 380
   Arg Glu Pro Gln Val Tyr Thr Leu Pro Pro Ser Arg Asp Glu Leu Thr
   385 390 395 400
   Lys Asn Gln Val Ser Leu Thr Cys Leu Val Lys Gly Phe Tyr Pro Ser
   405 410 415
   Asp Ile Ala Val Glu Trp Glu Ser Asn Gly Gln Pro Glu Asn Asn Tyr
   420 425 430
   Lys Thr Thr Pro Pro Val Leu Asp Ser Asp Gly Ser Phe Phe Leu Tyr
   435 440 445
   Ser Lys Leu Thr Val Asp Lys Ser Arg Trp Gln Gln Gly Asn Val Phe
   450 455 460
   Ser Cys Ser Val Met His Glu Ala Leu His Asn His Tyr Thr Gln Lys
   465 470 475 480
   Ser Leu Ser Leu Ser Pro Gly Lys
   485
<210> 7
   <211> 570
   <212> DNA
   <213> homo sapiens
<400> 7
   atggcctggc gagccctaca cccactgcta ctgctgctgc tgctgttccc aggctctcag 60
   gcacaatcca aggctcaggt acttcaaagt gtggcagggc agacgctaac cgtgagatgc 120
   cagtacccgc ccacgggcag tctctacgag aagaaaggct ggtgtaagga ggcttcagca 180
   cttgtgtgca tcaggttagt caccagctcc aagcccagga cgatggcttg gacctctcga 240
   ttcacaatct gggacgaccc tgatgctggc ttcttcactg tcaccatgac tgatctgaga 300
   gaggaagact caggacatta ctggtgtaga atctaccgcc cttctgacaa ctctgtctct 360
   aagtccgtca gattctatct ggtggtatct ccagcctctg cctccacaca gaccccctgg 420
   actccccgcg acctggtctc ttcacagacc cagacccaga gctgtgtgcc tcccactgca 480
   ggagccagac aagcccctga gtctccatct accatccctg tcccttcaca gccacagaac 540
   tccacgctcc gccctggccc tgcagccccc 570
<210> 8
   <211> 1275
   <212> DNA
   <213> homo sapiens
<400> 8
   atggcctggc gagccctaca cccactgcta ctgctgctgc tgctgttccc aggctctcag 60
   gcacaatcca aggctcaggt acttcaaagt gtggcagggc agacgctaac cgtgagatgc 120
   cagtacccgc ccacgggcag tctctacgag aagaaaggct ggtgtaagga ggcttcagca 180
   cttgtgtgca tcaggttagt caccagctcc aagcccagga cgatggcttg gacctctcga 240
   ttcacaatct gggacgaccc tgatgctggc ttcttcactg tcaccatgac tgatctgaga 300
   gaggaagact caggacatta ctggtgtaga atctaccgcc cttctgacaa ctctgtctct 360
   aagtccgtca gattctatct ggtggtatct ccagcctctg cctccacaca gaccccctgg 420
   actccccgcg acctggtctc ttcacagacc cagacccaga gctgtgtgcc tcccactgca 480
   ggagccagac aagcccctga gtctccatct accatccctg tcccttcaca gccacagaac 540
   tccacgctcc gccctggccc tgcagccccc gatccggagc ccaaatcttc tgacaaaact 600
   cacacatgcc caccgtgccc agcacctgaa ttcgagggtg caccgtcagt cttcctcttc 660
   cccccaaaac ccaaggacac cctcatgatc tcccggaccc ctgaggtcac atgcgtggtg 720
   gtggacgtga gccacgaaga ccctgaggtc aagttcaact ggtacgtgga cggcgtggag 780
   gtgcataatg ccaagacaaa gccgcgggag gagcagtaca acagcacgta ccgtgtggtc 840
   agcgtcctca ccgtcctgca ccaggactgg ctgaatggca aggagtacaa gtgcaaggtc 900
   tccaacaaag ccctcccagc ccccatcgag aaaaccatct ccaaagccaa agggcagccc 960
   cgagagccac aggtgtacac cctgccccca tcccgggatg agctgaccaa gaaccaggtc 1020
   agcctgacct gcctggtcaa aggcttctat cccagcgaca tcgccgtgga gtgggagagc 1080
   aatgggcagc cggagaacaa ctacaagacc acgcctcccg tgctggactc cgacggctcc 1140
   ttcttcctct acagcaagct caccgtggac aagagcaggt ggcagcaggg gaacgtcttc 1200
   tcatgctccg tgatgcatga ggctctgcac aaccactaca cgcagaagag cctctccctg 1260
   tctccgggta aatga                                                  1275
<210> 9
   <211> 190
   <212> PRT
   <213> homo sapiens
<400> 9
   Met Ala Trp Arg Ala Leu His Pro Leu Leu Leu Leu Leu Leu Leu Phe
   1               5                   10                  15
   Pro Gly Ser Gln Ala Gln Ser Lys Ala Gln Val Leu Gln Ser Val Ala
               20                  25                  30
   Gly Gln Thr Leu Thr Val Arg Cys Gln Tyr Pro Pro Thr Gly Ser Leu
           35                  40                  45
   Tyr Glu Lys Lys Gly Trp Cys Lys Glu Ala Ser Ala Leu Val Cys Ile
       50                  55                  60
   Arg Leu Val Thr Ser Ser Lys Pro Arg Thr Met Ala Trp Thr Ser Arg
   65                  70                  75                  80
   Phe Thr Ile Trp Asp Asp Pro Asp Ala Gly Phe Phe Thr Val Thr Met
                   85                  90                  95
   Thr Asp Leu Arg Glu Glu Asp Ser Gly His Tyr Trp Cys Arg Ile Tyr
               100                 105                 110
   Arg Pro Ser Asp Asn Ser Val Ser Lys Ser Val Arg Phe Tyr Leu Val
           115                 120                 125
   Val Ser Pro Ala Ser Ala Ser Thr Gln Thr Pro Trp Thr Pro Arg Asp
       130                 135                 140
   Leu Val Ser Ser Gln Thr Gln Thr Gln Ser Cys Val Pro Pro Thr Ala
   145                 150                 155                 160
   Gly Ala Arg Gln Ala Pro Glu Ser Pro Ser Thr Ile Pro Val Pro Ser
                   165                 170                 175
   Gln Pro Gln Asn Ser Thr Leu Arg Pro Gly Pro Ala Ala Pro
               180                 185                 190
<210> 10
   <211> 424
   <212> PRT
   <213> homo sapiens
<400> 10
   Met Ala Trp Arg Ala Leu His Pro Leu Leu Leu Leu Leu Leu Leu Phe
   1               5                   10                  15
   Pro Gly Ser Gln Ala Gln Ser Lys Ala Gln Val Leu Gln Ser Val Ala
               20                  25                  30
   Gly Gln Thr Leu Thr Val Arg Cys Gln Tyr Pro Pro Thr Gly Ser Leu
           35                  40                  45
   Tyr Glu Lys Lys Gly Trp Cys Lys Glu Ala Ser Ala Leu Val Cys Ile
       50                  55                  60
   Arg Leu Val Thr Ser Ser Lys Pro Arg Thr Met Ala Trp Thr Ser Arg
   65                  70                  75                  80
   Phe Thr Ile Trp Asp Asp Pro Asp Ala Gly Phe Phe Thr Val Thr Met
                   85                  90                  95
   Thr Asp Leu Arg Glu Glu Asp Ser Gly His Tyr Trp Cys Arg Ile Tyr
               100                 105                 110
   Arg Pro Ser Asp Asn Ser Val Ser Lys Ser Val Arg Phe Tyr Leu Val
           115                 120                 125
   Val Ser Pro Ala Ser Ala Ser Thr Gln Thr Pro Trp Thr Pro Arg Asp
       130                 135                 140
   Leu Val Ser Ser Gln Thr Gln Thr Gln Ser Cys Val Pro Pro Thr Ala
   145                 150                 155                 160
   Gly Ala Arg Gln Ala Pro Glu Ser Pro Ser Thr Ile Pro Val Pro Ser
                   165                 170                 175
   Gln Pro Gln Asn Ser Thr Leu Arg Pro Gly Pro Ala Ala Pro Asp Pro
               180                 185                 190
   Glu Pro Lys Ser Ser Asp Lys Thr His Thr Cys Pro Pro Cys Pro Ala
           195                 200                 205
   Pro Glu Phe Glu Gly Ala Pro Ser Val Phe Leu Phe Pro Pro Lys Pro
       210                 215                 220
   Lys Asp Thr Leu Met Ile Ser Arg Thr Pro Glu Val Thr Cys Val Val
   225                 230                 235                 240
   Val Asp Val Ser His Glu Asp Pro Glu Val Lys Phe Asn Trp Tyr Val
                   245                 250                 255
   Asp Gly Val Glu Val His Asn Ala Lys Thr Lys Pro Arg Glu Glu Gln
               260                 265                 270
   Tyr Asn Ser Thr Tyr Arg Val Val Ser Val Leu Thr Val Leu His Gln
           275                 280                 285
   Asp Trp Leu Asn Gly Lys Glu Tyr Lys Cys Lys Val Ser Asn Lys Ala
       290                 295                 300
   Leu Pro Ala Pro Ile Glu Lys Thr Ile Ser Lys Ala Lys Gly Gln Pro
   305                 310                 315                 320
   Arg Glu Pro Gln Val Tyr Thr Leu Pro Pro Ser Arg Asp Glu Leu Thr
                   325                 330                 335
   Lys Asn Gln Val Ser Leu Thr Cys Leu Val Lys Gly Phe Tyr Pro Ser
               340                 345                 350
   Asp Ile Ala Val Glu Trp Glu Ser Asn Gly Gln Pro Glu Asn Asn Tyr
           355                 360                 365
   Lys Thr Thr Pro Pro Val Leu Asp Ser Asp Gly Ser Phe Phe Leu Tyr
       370                 375                 380
   Ser Lys Leu Thr Val Asp Lys Ser Arg Trp Gln Gln Gly Asn Val Phe
   385                 390                 395                 400
   Ser Cys Ser Val Met His Glu Ala Leu His Asn His Tyr Thr Gln Lys
                   405                 410                 415
   Ser Leu Ser Leu Ser Pro Gly Lys
               420
<210> 11
   <211> 711
   <212> DNA
   <213> homo sapiens
<400> 11
   atgtcttcca cactccctgc cctgctctgc gtcgggctgt gtctgagtca gaggatcagc 60
   gcccagcagc agactctccc aaaaccgttc atctgggccg agccccattt catggttcca 120
   aaggaaaagc aagtgaccat ctgttgccag ggaaattatg gggctgttga ataccagctg 180
   cactttgaag gaagcctttt tgccgtggac agaccaaaac cccctgagcg gattaacaaa 240
   gtcaaattct acatcccgga catgaactcc cgcatggcag ggcaatacag ctgcatctat 300
   cgggttgggg agctctggtc agagcccagc aacttgctgg atctggtggt aacagaaatg 360
   tatgacacac ccaccctctc ggttcatcct ggacccgaag tgatctcggg agagaaggtg 420
   accttctact gccgtctaga cactgcaaca agcatgttct tactgctcaa ggagggaaga 480
   tccagccacg tacagcgcgg atacgggaag gtccaggcgg agttccccct gggccctgtg 540
   accacagccc accgagggac ataccgatgt tttggctcct ataacaacca tgcctggtct 600
   ttccccagtg agccagtgaa gctcctggtc acaggcgaca ttgagaacac cagccttgca 660
   cctgaagacc ccacctttcc tgaccatgcc ctctgggatc acactgccca g          711
<210> 12
   <211> 1416
   <212> DNA
   <213> homo sapiens
<400> 12
   atgtcttcca cactccctgc cctgctctgc gtcgggctgt gtctgagtca gaggatcagc 60
   gcccagcagc agactctccc aaaaccgttc atctgggccg agccccattt catggttcca 120
   aaggaaaagc aagtgaccat ctgttgccag ggaaattatg gggctgttga ataccagctg 180
   cactttgaag gaagcctttt tgccgtggac agaccaaaac cccctgagcg gattaacaaa 240
   gtcaaattct acatcccgga catgaactcc cgcatggcag ggcaatacag ctgcatctat 300
   cgggttgggg agctctggtc agagcccagc aacttgctgg atctggtggt aacagaaatg 360
   tatgacacac ccaccctctc ggttcatcct ggacccgaag tgatctcggg agagaaggtg 420
   accttctact gccgtctaga cactgcaaca agcatgttct tactgctcaa ggagggaaga 480
   tccagccacg tacagcgcgg atacgggaag gtccaggcgg agttccccct gggccctgtg 540
   accacagccc accgagggac ataccgatgt tttggctcct ataacaacca tgcctggtct 600
   ttccccagtg agccagtgaa gctcctggtc acaggcgaca ttgagaacac cagccttgca 660
   cctgaagacc ccacctttcc tgaccatgcc ctctgggatc acactgccca ggatccggag 720
   cccaaatctt ctgacaaaac tcacacatgc ccaccgtgcc cagcacctga attcgagggt 780
   gcaccgtcag tcttcctctt ccccccaaaa cccaaggaca ccctcatgat ctcccggacc 840
   cctgaggtca catgcgtggt ggtggacgtg agccacgaag accctgaggt caagttcaac 900
   tggtacgtgg acggcgtgga ggtgcataat gccaagacaa agccgcggga ggagcagtac 960
   aacagcacgt accgtgtggt cagcgtcctc accgtcctgc accaggactg gctgaatggc 1020
   aaggagtaca agtgcaaggt ctccaacaaa gccctcccag cccccatcga gaaaaccatc 1080
   tccaaagcca aagggcagcc ccgagagcca caggtgtaca ccctgccccc atcccgggat 1140
   gagctgacca agaaccaggt cagcctgacc tgcctggtca aaggcttcta tcccagcgac 1200
   atcgccgtgg agtgggagag caatgggcag ccggagaaca actacaagac cacgcctccc 1260
   gtgctggact ccgacggctc cttcttcctc tacagcaagc tcaccgtgga caagagcagg 1320
   tggcagcagg ggaacgtctt ctcatgctcc gtgatgcatg aggctctgca caaccactac 1380
   acgcagaaga gcctctccct gtctccgggt aaatga 1416
<210> 13
   <211> 237
   <212> PRT
   <213> homo sapiens
<400> 13
   Met Ser Ser Thr Leu Pro Ala Leu Leu Cys Val Gly Leu Cys Leu Ser
   1 5 10 15
   Gln Arg Ile Ser Ala Gln Gln Gln Thr Leu Pro Lys Pro Phe Ile Trp
   20 25 30
   Ala Glu Pro His Phe Met Val Pro Lys Glu Lys Gln Val Thr Ile Cys
   35 40 45
   Cys Gln Gly Asn Tyr Gly Ala Val Glu Tyr Gln Leu His Phe Glu Gly
   50 55 60
   Ser Leu Phe Ala Val Asp Arg Pro Lys Pro Pro Glu Arg Ile Asn Lys
   65 70 75 80
   Val Lys Phe Tyr Ile Pro Asp Met Asn Ser Arg Met Ala Gly Gln Tyr
   85 90 95
   Ser Cys Ile Tyr Arg Val Gly Glu Leu Trp Ser Glu Pro Ser Asn Leu
   100 105 110
   Leu Asp Leu Val Val Thr Glu Met Tyr Asp Thr Pro Thr Leu Ser Val
   115 120 125
   His Pro Gly Pro Glu Val Ile Ser Gly Glu Lys Val Thr Phe Tyr Cys
   130 135 140
   Arg Leu Asp Thr Ala Thr Ser Met Phe Leu Leu Leu Lys Glu Gly Arg
   145 150 155 160
   Ser Ser His Val Gln Arg Gly Tyr Gly Lys Val Gln Ala Glu Phe Pro
   165 170 175
   Leu Gly Pro Val Thr Thr Ala His Arg Gly Thr Tyr Arg Cys Phe Gly
   180 185 190
   Ser Tyr Asn Asn His Ala Trp Ser Phe Pro Ser Glu Pro Val Lys Leu
   195 200 205
   Leu Val Thr Gly Asp Ile Glu Asn Thr Ser Leu Ala Pro Glu Asp Pro
   210 215 220
   Thr Phe Pro Asp His Ala Leu Trp Asp His Thr Ala Gln
   225 230 235
<210> 14
   <211> 471
   <212> PRT
   <213> homo sapiens
<400> 14
   Met Ser Ser Thr Leu Pro Ala Leu Leu Cys Val Gly Leu Cys Leu Ser
   1 5 10 15
   Gln Arg Ile Ser Ala Gln Gln Gln Thr Leu Pro Lys Pro Phe Ile Trp
   20 25 30
   Ala Glu Pro His Phe Met Val Pro Lys Glu Lys Gln Val Thr Ile Cys
   35 40 45
   Cys Gln Gly Asn Tyr Gly Ala Val Glu Tyr Gln Leu His Phe Glu Gly
   50 55 60
   Ser Leu Phe Ala Val Asp Arg Pro Lys Pro Pro Glu Arg Ile Asn Lys
   65 70 75 80
   Val Lys Phe Tyr Ile Pro Asp Met Asn Ser Arg Met Ala Gly Gln Tyr
   85 90 95
   Ser Cys Ile Tyr Arg Val Gly Glu Leu Trp Ser Glu Pro Ser Asn Leu
   100 105 110
   Leu Asp Leu Val Val Thr Glu Met Tyr Asp Thr Pro Thr Leu Ser Val
   115 120 125
   His Pro Gly Pro Glu Val Ile Ser Gly Glu Lys Val Thr Phe Tyr Cys
   130 135 140
   Arg Leu Asp Thr Ala Thr Ser Met Phe Leu Leu Leu Lys Glu Gly Arg
   145 150 155 160
   Ser Ser His Val Gln Arg Gly Tyr Gly Lys Val Gln Ala Glu Phe Pro
   165 170 175
   Leu Gly Pro Val Thr Thr Ala His Arg Gly Thr Tyr Arg Cys Phe Gly
   180 185 190
   Ser Tyr Asn Asn His Ala Trp Ser Phe Pro Ser Glu Pro Val Lys Leu
   195 200 205
   Leu Val Thr Gly Asp Ile Glu Asn Thr Ser Leu Ala Pro Glu Asp Pro
   210 215 220
   Thr Phe Pro Asp His Ala Leu Trp Asp His Thr Ala Gln Asp Pro Glu
   225 230 235 240
   Pro Lys Ser Ser Asp Lys Thr His Thr Cys Pro Pro Cys Pro Ala Pro
   245 250 255
   Glu Phe Glu Gly Ala Pro Ser Val Phe Leu Phe Pro Pro Lys Pro Lys
   260 265 270
   Asp Thr Leu Met Ile Ser Arg Thr Pro Glu Val Thr Cys Val Val Val
   275 280 285
   Asp Val Ser His Glu Asp Pro Glu Val Lys Phe Asn Trp Tyr Val Asp
   290 295 300
   Gly Val Glu Val His Asn Ala Lys Thr Lys Pro Arg Glu Glu Gln Tyr
   305 310 315 320
   Asn Ser Thr Tyr Arg Val Val Ser Val Leu Thr Val Leu His Gln Asp
   325 330 335
   Trp Leu Asn Gly Lys Glu Tyr Lys Cys Lys Val Ser Asn Lys Ala Leu
   340 345 350
   Pro Ala Pro Ile Glu Lys Thr Ile Ser Lys Ala Lys Gly Gln Pro Arg
   355 360 365
   Glu Pro Gln Val Tyr Thr Leu Pro Pro Ser Arg Asp Glu Leu Thr Lys
   370 375 380
   Asn Gln Val Ser Leu Thr Cys Leu Val Lys Gly Phe Tyr Pro Ser Asp
   385                 390                 395                 400
   Ile Ala Val Glu Trp Glu Ser Asn Gly Gln Pro Glu Asn Asn Tyr Lys
                   405                 410                 415
   Thr Thr Pro Pro Val Leu Asp Ser Asp Gly Ser Phe Phe Leu Tyr Ser
               420                 425                 430
   Lys Leu Thr Val Asp Lys Ser Arg Trp Gln Gln Gly Asn Val Phe Ser
           435                 440                 445
   Cys Ser Val Met His Glu Ala Leu His Asn His Tyr Thr Gln Lys Ser
       450                 455                 460
   Leu Ser Leu Ser Pro Gly Lys
   465                 470
<210> 15
   <211> 405
   <212> DNA
   <213> homo sapiens
<400> 15
   atggcctgga tgctgttgct catcttgatc atggtccatc caggatcctg tgctctctgg 60
   gtgtcccagc cccctgagat tcgtaccctg gaaggatcct ctgccttcct gccctgctcc 120
   ttcaatgcca gccaagggag actggccatt ggctccgtca cgtggttccg agatgaggtg 180
   gttccaggga aggaggtgag gaatggaacc ccagagttca ggggccgcct ggccccactt 240
   gcttcttccc gtttcctcca tgaccaccag gctgagctgc acatccggga cgtgcgaggc 300
   catgacgcca gcatctacgt gtgcagagtg gaggtgctgg gccttggtgt cgggacaggg 360
   aatgggactc ggctggtggt ggagaaagaa catcctcagc taggg                405
<210> 16
   <211> 1110
   <212> DNA
   <213> homo sapiens
<400> 16
   atggcctgga tgctgttgct catcttgatc atggtccatc caggatcctg tgctctctgg 60
   gtgtcccagc cccctgagat tcgtaccctg gaaggatcct ctgccttcct gccctgctcc 120
   ttcaatgcca gccaagggag actggccatt ggctccgtca cgtggttccg agatgaggtg 180
   gttccaggga aggaggtgag gaatggaacc ccagagttca ggggccgcct ggccccactt 240
   gcttcttccc gtttcctcca tgaccaccag gctgagctgc acatccggga cgtgcgaggc 300
   catgacgcca gcatctacgt gtgcagagtg gaggtgctgg gccttggtgt cgggacaggg 360
   aatgggactc ggctggtggt ggagaaagaa catcctcagc taggggatcc ggagcccaaa 420
   tcttctgaca aaactcacac atgcccaccg tgcccagcac ctgaattcga gggtgcaccg 480
   tcagtcttcc tcttcccccc aaaacccaag gacaccctca tgatctcccg gacccctgag 540
   gtcacatgcg tggtggtgga cgtgagccac gaagaccctg aggtcaagtt caactggtac 600
   gtggacggcg tggaggtgca taatgccaag acaaagccgc gggaggagca gtacaacagc 660
   acgtaccgtg tggtcagcgt cctcaccgtc ctgcaccagg actggctgaa tggcaaggag 720
   tacaagtgca aggtctccaa caaagccctc ccagccccca tcgagaaaac catctccaaa 780
   gccaaagggc agccccgaga gccacaggtg tacaccctgc ccccatcccg ggatgagctg 840
   accaagaacc aggtcagcct gacctgcctg gtcaaaggct tctatcccag cgacatcgcc 900
   gtggagtggg agagcaatgg gcagccggag aacaactaca agaccacgcc tcccgtgctg 960
   gactccgacg gctccttctt cctctacagc aagctcaccg tggacaagag caggtggcag 1020
   caggggaacg tcttctcatg ctccgtgatg catgaggctc tgcacaacca ctacacgcag 1080
   aagagcctct ccctgtctcc gggtaaatga                                  1110
<210> 17
   <211> 135
   <212> PRT
   <213> homo sapiens
<400> 17
   Met Ala Trp Met Leu Leu Leu Ile Leu Ile Met Val His Pro Gly Ser
   1               5                   10                  15
   Cys Ala Leu Trp Val Ser Gln Pro Pro Glu Ile Arg Thr Leu Glu Gly
               20                  25                  30
   Ser Ser Ala Phe Leu Pro Cys Ser Phe Asn Ala Ser Gln Gly Arg Leu
           35                  40                  45
   Ala Ile Gly Ser Val Thr Trp Phe Arg Asp Glu Val Val Pro Gly Lys
       50                  55                  60
   Glu Val Arg Asn Gly Thr Pro Glu Phe Arg Gly Arg Leu Ala Pro Leu
   65                  70                  75                  80
   Ala Ser Ser Arg Phe Leu His Asp His Gln Ala Glu Leu His Ile Arg
                   85                  90                  95
   Asp Val Arg Gly His Asp Ala Ser Ile Tyr Val Cys Arg Val Glu Val
               100                 105                 110
   Leu Gly Leu Gly Val Gly Thr Gly Asn Gly Thr Arg Leu Val Val Glu
           115                 120                 125
   Lys Glu His Pro Gln Leu Gly
       130                 135
<210> 18
   <211> 369
   <212> PRT
   <213> homo sapiens
<400> 18
<210> 19
   <211> 402
   <212> DNA
   <213> homo sapiens
<400> 19
   tatgacacac ccaccctctc ggttcatcct ggacccgaag tgatctcggg agagaaggtg 60
   accttctact gccgtctaga cactgcaaca agcatgttct tactgctcaa ggagggaaga 120
   tccagccacg tacagcgcgg atacgggaag gtccaggcgg agttccccct gggccctgtg 180
   accacagccc accgagggac ataccgatgt tttggctcct ataacaacca tgcctggtct 240
   ttccccagtg agccagtgaa gctcctggtc acaggcgaca ttgagaacac cagccttgca 300
   cctgaagacc ccacctttcc tgcagacact tggggcacct accttttaac cacagagacg 360
   ggactccaga aagaccatgc cctctgggat cacactgccc ag                   402
<210> 20
   <211> 351
   <212> DNA
   <213> homo sapiens
<400> 20
   tatgacacac ccaccctctc ggttcatcct ggacccgaag tgatctcggg agagaaggtg 60
   accttctact gccgtctaga cactgcaaca agcatgttct tactgctcaa ggagggaaga 120
   tccagccacg tacagcgcgg atacgggaag gtccaggcgg agttccccct gggccctgtg 180
   accacagccc accgagggac ataccgatgt tttggctcct ataacaacca tgcctggtct 240
   ttccccagtg agccagtgaa gctcctggtc acaggcgaca ttgagaacac cagccttgca 300
   cctgaagacc ccacctttcc tgaccatgcc ctctgggatc acactgccca g          351
<210> 21
   <211> 240
   <212> DNA
   <213> homo sapiens
<400> 21
   atctaccgcc cttctgacaa ctctgtctct aagtccgtca gattctatct ggtggtatct 60
   ccagcctctg cctccacaca gaccccctgg actccccgcg acctggtctc ttcacagacc 120
   cagacccaga gctgtgtgcc tcccactgca ggagccagac aagcccctga gtctccatct 180
   accatccctg tcccttcaca gccacagaac tccacgctcc gccctggccc tgcagccccc 240
<210> 22
   <211> 134
   <212> PRT
   <213> homo sapiens
<400> 22
   Tyr Asp Thr Pro Thr Leu Ser Val His Pro Gly Pro Glu Val Ile Ser
   1               5                   10                  15
   Gly Glu Lys Val Thr Phe Tyr Cys Arg Leu Asp Thr Ala Thr Ser Met
               20                  25                  30
   Phe Leu Leu Leu Lys Glu Gly Arg Ser Ser His Val Gln Arg Gly Tyr
           35                  40                  45
   Gly Lys Val Gln Ala Glu Phe Pro Leu Gly Pro Val Thr Thr Ala His
       50                  55                  60
   Arg Gly Thr Tyr Arg Cys Phe Gly Ser Tyr Asn Asn His Ala Trp Ser
   65                  70                  75                  80
   Phe Pro Ser Glu Pro Val Lys Leu Leu Val Thr Gly Asp Ile Glu Asn
                   85                  90                  95
   Thr Ser Leu Ala Pro Glu Asp Pro Thr Phe Pro Ala Asp Thr Trp Gly
               100                 105                 110
   Thr Tyr Leu Leu Thr Thr Glu Thr Gly Leu Gln Lys Asp His Ala Leu
           115                 120                 125
   Trp Asp His Thr Ala Gln
       130
<210> 23
   <211> 117
   <212> PRT
   <213> homo sapiens
<400> 23
   Tyr Asp Thr Pro Thr Leu Ser Val His Pro Gly Pro Glu Val Ile Ser
   1               5                   10                  15
   Gly Glu Lys Val Thr Phe Tyr Cys Arg Leu Asp Thr Ala Thr Ser Met
               20                  25                  30
   Phe Leu Leu Leu Lys Glu Gly Arg Ser Ser His Val Gln Arg Gly Tyr
           35                  40                  45
   Gly Lys Val Gln Ala Glu Phe Pro Leu Gly Pro Val Thr Thr Ala His
       50                  55                  60
   Arg Gly Thr Tyr Arg Cys Phe Gly Ser Tyr Asn Asn His Ala Trp Ser
    65                  70                  75                  80
   Phe Pro Ser Glu Pro Val Lys Leu Leu Val Thr Gly Asp Ile Glu Asn
                    85                  90                  95
   Thr Ser Leu Ala Pro Glu Asp Pro Thr Phe Pro Asp His Ala Leu Trp
               100                 105                 110
   Asp His Thr Ala Gln
           115
<210> 24
   <211> 79
   <212> PRT
   <213> homo sapiens
<400> 24
   Ile Tyr Arg Pro Ser Asp Asn Ser Val Ser Lys Ser Val Arg Phe Tyr
     1               5                  10                  15
   Leu Val Val Ser Pro Ala Ser Ala Ser Thr Gln Thr Pro Trp Thr Pro
                20                  25                  30
   Arg Asp Leu Val Ser Ser Gln Thr Gln Thr Gln Ser Cys Val Pro Pro
            35                  40                  45
   Thr Ala Gly Ala Arg Gln Ala Pro Glu Ser Pro Ser Thr Ile Pro Val
        50                  55                  60
   Pro Ser Gln Pro Gln Asn Ser Thr Leu Arg Pro Gly Pro Ala Pro
    65                  70                  75
<210> 25
   <211> 360
   <212> DNA
   <213> homo sapiens
<400> 25
   atgtcttcca cactccctgc cctgctctgc gtcgggctgt gtctgagtca gaggatcagc 60
   gcccagcagc agactctccc aaaaccgttc atctgggccg agccccattt catggttcca 120
   aaggaaaagc aagtgaccat ctgttgccag ggaaattatg gggctgttga ataccagctg 180
   cactttgaag gaagcctttt tgccgtggac agaccaaaac cccctgagcg gattaacaaa 240
   gtcaaattct acatcccgga catgaactcc cgcatggcag ggcaatacag ctgcatctat 300
   cgggttgggg agctctggtc agagcccagc aacttgctgg atctggtggt aacagaaatg 360
<210> 26
   <211> 120
   <212> PRT
   <213> homo sapiens
<400> 26
   Met Ser Ser Thr Leu Pro Ala Leu Leu Cys Val Gly Leu Cys Leu Ser
    1               5                  10                  15
   Gln Arg Ile Ser Ala Gln Gln Gln Thr Leu Pro Lys Pro Phe Ile Trp
               20                  25                  30
   Ala Glu Pro His Phe Met Val Pro Lys Glu Lys Gln Val Thr Ile Cys
           35                  40                  45
   Cys Gln Gly Asn Tyr Gly Ala Val Glu Tyr Gln Leu His Phe Glu Gly
       50                  55                  60
   Ser Leu Phe Ala Val Asp Arg Pro Lys Pro Pro Glu Arg Ile Asn Lys
   65                  70                  75                  80
   Val Lys Phe Tyr Ile Pro Asp Met Asn Ser Arg Met Ala Gly Gln Tyr
                   85                  90                  95
   Ser Cys Ile Tyr Arg Val Gly Glu Leu Trp Ser Glu Pro Ser Asn Leu
               100                 105                 110
   Leu Asp Leu Val Val Thr Glu Met
           115                 120

## Description

### Field of the Invention

The invention relates to therapeutic agents for the treatment of pathologies associated with viral infections or cancer and for the imaging and monitoring of cancer. More particularly, the present invention provides compositions and methods for the treatment and detection of a variety of viral infections, by using complex agents comprising the NK cells activating proteins, NKp46 and NKp44, and functional fragments thereof, linked to therapeutic or imaging agents.

### Background of the Invention

Natural Killer (NK) cells, as well as cytotoxic T lymphocytes (CTL), are major components of the cellular mechanism by which an immune response leads to the destruction of foreign or infected tissue [Trinchieri, *et al.,* Adv. in Immunol. 47:187-376 (1989)]. In contrast to CTL, which are activated in the presence of class I MHC molecules and an appropriate specific peptide, one well defined function of NK cells is the lysis of target cells deficient in expression of MHC class I proteins. In this manner NK cells carry out immuno-surveillance for "miself" [Ljunggren *et al.*, Immunol. Today 11:7-10 (1990)], rather than for direct detection of foreign antigens.

Thus, NK cells, generally representing about 10-15% of circulating lymphocytes, bind and kill target cells including virus-infected cells and many malignant cells, in a nonspecific manner with regard to antigen and without prior immune sensitization [Herberman *et al.,* Science 214:24-27 (1981)].

Recognition of polymorphic determinants on HLA molecules by human NK cell inhibitory receptors is mediated by three types of class I MHC-binding receptors: the Ig superfamily of inhibitory receptors which includes both the NKIR proteins [Colonna, *et al.*, Science 268:405-408 (1995); Wagtmann, *et al.,* Immunity 2:439-449 (1995); D'Andre, *et al.,* J. Immunol 155:2306-2310 (1995)] and the ILT-2 protein [Colonna, *et al.,* J. Exp.Med. 186:1809-1818 (1997)], whose ligands are various HLA-A, -B and -C proteins, and the mucin-like CD94/NKG2 complex, which delivers an inhibitory signal upon binding the HLA-E protein [Borrego *et al.,* J. Exp. Med. 187:813-818 (1998); Brnud et al., Nature, 391:795-799 (1998); Lee *et al.,* Proc. Natl. Acad. Sci. USA, 95:5199-5204 (1998)]. This variety of class I MHC protein-specific receptors illustrates the importance of these molecules in modulating NK function.

However, lysis receptor(s) involved in triggering NK cell cytotoxicity against target cells are little understood. Four candidate lysis receptors were recently identified, NKp30, NKp44 [Cantoni, C. *et al.,* J. Exp. Med. 189:787-796 (1999)], NKp46 [Pessino *et al.,* J. Exp. Med. 188:953-960 (1998)] and CD16 [Mandelboim *et al.,* Proc. Natl. Acad. Sci. USA. 96:5640-5644 (1999)]. The NKp46 receptor is conceded to be the major lysis receptor involved in killing target cells as it is expressed on all NK cells, whether activated or non-activated [Pessino et al., (1998) *ibid*.]. The present invention is based on some identification and characterization of "lysis ligand(s)" for NKp44 and NKp46. With the exception of NKp44, all of these receptors are expressed on the surface of both activated and non activated NK cells and all transduce activation signals via association with CD3ζ/FcεRIγ [Bottino, C., et al., *Hum. Immunol.* 61: 1-6 (2000). In contrast, the NKp44 receptor is expressed on the surface of activating NK cells only and delivers its activating signal via the association with DAP12 [Lanier L.L., et al., *Nature.* 391: 703-707 (1998)]. The lysis ligands that are recognized by these receptors are unknown.

The findings of the present inventors show that soluble NKp44- and NKp46-Ig fusion proteins bind to the hemagglutinin (HA) of Influenza virus, and the hemagglutinin-neuraminidase (HN) of parainfluenza virus, and that binding of NKp44 and NKp46 to these viral proteins is required for lysis of cells expressing the corresponding glycoproteins. The binding requires the sialylation of NKp44 and NKp46 oligosaccharides, which is consistent with the known sialic binding capacity of the viral glycoproteins. These findings explain how NKp44- and NKp46-expressing cells can recognize Influenza and parainfluenza virus-infected target cells without a major decrease in target cell class I molecule expression. As sialic acid is utilized as a receptor for a number of other viruses, a general strategy for NK recognition of a substantial subset of viral pathogens may be suggested.

### Summary of the Invention

In a first aspect, the present invention relates to a targeting complex, capable of targeting an active substance to a target cell. This complex comprises a target recognition segment, which comprises at least NKp46, NKp30, NKp44 or a functional fragment thereof, and an active segment comprising the active substance which may be a cytotoxic moiety, an imaging moiety or an Ig fragment.

The target recognition segment is derived from NKp46 and preferably comprises at least one of domains 1 and 2 of the NKp46 molecule, more preferably both domains 1 and 2. In another particularly preferred embodiment the segment comprises domain 2 of the NKp46 molecule. Alternatively, the target recognition segment is derived from NKp44 and preferably comprises at least one of domains 1 and 2 of the NKp44 molecule, more preferably both domains 1 and 2. In another particularly preferred embodiment the segment comprises domain 2 of the NKp44 molecule.

In a specifically preferred embodiment the complex the invention is a fusion protein comprising as the active segment an Ig fragment. This Ig fragment is preferably the Fc portion of an Ig molecule.

Alternatively, the complex of the present invention is a conjugate comprising as an active segment a cytotoxic moiety. This cytotoxic moiety may be a cytotoxin or an anticellular agent, which is capable of killing and/or suppressing the growth or cell division of the target cell.

Preferred conjugates of the invention may comprise as the cytotoxin or anticellular agent a synthetic toxin or a toxin derived from plants, fungi, or bacteria. More specifically, this toxin can be selected from any one of A chain toxin, ribosome inactivating protein, α-sarcin, aspergillin, restrictocin, ribonuclease, diphtheria toxin, *Pseudomonas* exotoxin, an endotoxin or the lipid A moiety of an endotoxin.

In yet another alternative embodiment, the complex of the present invention may be a conjugate in which the active segment is an imaging moiety. The imaging moiety may be any detectable label, such as paramagnetic, radioactive and fluorogenic labels.

In a specific embodiment, the complex of the present invention may be specifically targeted to cells derived from solid as well as non-solid tumors, particularly malignant tumors.

Alternatively, the complex of the present invention is targeted to a defective or diseased cell. Such target cells may be pathogenic virus-infected cells. More specifically, the pathogenic virus may be any of variety of viruses including, but not limited to, Influenza virus, human immunodeficiency virus, Epstein-Barr virus, cytomegalovirus, Vaccinia virus and Herpes virus.

Preferably, where the target cell is a virus-infected cell, the complex of the invention has a target recognition segment which is capable of binding to a ligand expressed on the surface of said target cell, this binding being mediated by sialic acid.

A second aspect the present invention relates to an expression vector comprising DNA coding for a NKp46-Ig fusion protein. This DNA comprises a segment encoding NKp46 or functional fragments thereof, preferably a NKp46 fragment comprising at least one of domains 1 and 2 of the NKp46 molecule, more preferably both domains 1 and 2 of the NKp46 molecule. This expression vector further comprises a second segment comprises a DNA sequence encoding the Fc portion of an Ig molecule. In another preferred embodiment, the first segment may comprise the nucleic acid sequence of domain 2 alone.

In another embodiment of the present aspect, the invention relates to an expression vector comprising DNA coding for a NKp44-Ig fusion protein. This DNA comprises a segment encoding NKp44 or functional fragments thereof, preferably a NKp44 fragment comprising at least one of domains 1 and 2 of the NKp44 molecule, more preferably both domains 1 and 2. In another preferred embodiment, the first segment may comprise the nucleic acid sequence of domain 2 alone. The second segment of the DNA comprised in the expression vector of the invention comprises a DNA sequence encoding the Fc portion of an Ig molecule.

The invention also relates to a host cell transformed with the DNA or expression vectors of the invention.

A specifically preferred embodiment of the invention relates to a NKp46-Ig fusion protein. This fusion protein comprises the amino acid sequence substantially as denoted by any one of SEQ ID NO:6 and NO:14, and is encoded by the nucleic acid sequence substantially as denoted by any one of SEQ ID NO:3 and NO:12.

In yet another specifically preferred embodiment the invention relates to a NKp44-Ig fusion protein. This fusion protein comprises the amino acid sequence substantially as denoted by SEQ ID NO:10, and is encoded by the nucleic acid sequence substantially as denoted by SEQ ID NO:8.

Another aspect of the present invention relates to antibodies that specifically recognize and bind to the fusion proteins NKp46-Ig or to NKp44-Ig of the invention.

Additionally, the invention relates to an antibody that specifically recognizes and binds to an epitope on a protein, which protein is a ligand for the NK cell activating receptor NKp46 or NKp44. A specifically preferred antibody is the antibody designated as 135.7. The antibodies of the invention may be mono- or polyclonal antibodies. Further, the antibodies of the invention may be conjugated to a detectable moiety.

In a further aspect the invention relates to compositions for the treatment-of pathological conditions. The compositions of the invention comprise as active ingredient a complex comprising a target recognition segment and an active segment. The target recognition segment is capable of specifically recognizing and binding to a diseased target cell involved with said pathological condition. This target recognition segment comprises at least NKp46, NKp30, NKp44, or a biologically functional fragment thereof. The active segment of the complex may be selected from cytotoxic agents moieties and Ig fragments.

In one embodiment the composition of the present invention is intended for treating a malignant disease such as, for example, melanoma, carcinoma, sarcoma and lymphoma.

Alternatively, the compositions of the present invention are intended for treating viral infections caused by any one of Influenza virus, human immunodeficiency virus, Epstein-Barr virus, cytomegalovirus, Vaccinia virus, ECMV, MVM and Herpes virus.

The NKp46, NKp44 or their fragments, comprised in the complexes and compositions of the invention are capable of binding to a ligand expressed on the surface of the target cell. When the target cell is a virus-infected cell, the binding is mediated by sialic acid. The complex of the invention may bind to a free virus, this binding also being mediated by sialic acid.

Preferably, the target recognition segment in the complexes and compositions of the invention comprises a NKp46 fragment comprising at least one of domains 1 and 2 of the NKp46 molecule, preferably comprising both domains 1 and 2. In another preferred embodiment the fragment comprising only domain 2 of NKp46. Alternatively, the target recognition segment in the complex and compositions of the invention comprises a NKp44 fragment comprising at least one of domains 1 and 2 of the NKp44 molecule, preferably comprising both domains 1 and 2. In another preferred embodiment the fragment comprising only domain 2 of NKp44.

The active segment of the complex or compositions of the invention may be an Ig fragment, preferably the Fc portion of an Ig molecule. Alternatively, the active fragment may be a cytotoxic moiety, such as a cytotoxin or an anticellular agent capable of killing and/or suppressing the growth and/or cell division of the target cell.

The cytotoxin or anticellular agent may be a synthetic agent or a plant-derived, fungal, or bacteria-derived toxin.

More particularly, the toxin may be selected from the group consisting of A chain toxin, ribosome inactivating protein, α-sarcin, aspergillin, restrictocin, ribonuclease, diphtheria toxin, *Pseudomonas* exotoxin, an endotoxin or the lipid A moiety of an endotoxin

An alternative aspect of the present invention relates to a diagnostic composition for detecting the presence of diseased or defective cells in a sample. This diagnostic composition comprises a complex comprising a target recognition segment that is capable of specifically recognizing and binding to a diseased target cell involved with the pathological condition, and a detectable moiety. The recognition segment comprises NKp46, NKp44, NKp30 or a biologically functional fragment thereof. The detectable imaging moiety may be a paramagnetic, radioactive or fluorogenic agent.

Another aspect of the present invention relates to a method for treating a pathological condition in a subject. This method comprises the step of administering to the subject a pharmaceutically effective amount of a therapeutic agent comprising a complex having a first, target recognition segment, capable of specifically recognizing and binding to a diseased target cell involved with the pathological condition, and a second, therapeutically active segment. The target recognition segment comprises at least one of NKp46, NKp30, NKp44 or a biologically functional fragment thereof, and the therapeutically active segment may be a cytotoxic moiety or an Ig fragment. The pathological condition to be treated may be a viral infection caused by Influenza virus, human immunodeficiency virus, Epstein-Barr virus; cytomegalovirus, Vaccinia virus and Herpes virus, or a malignant disease such as melanoma, carcinoma, lymphoma and sarcoma.

In a specifically preferred embodiment, the method of the invention employs complexes in which NKp46 or NKp44 comprised in the target recognition segment are capable of binding to a ligand expressed on the surface of said target cell. When the target cell is virus infected cell this binding is mediated by sialic acid. More specifically, the NKp46 fragment comprises at least one of domains 1 and 2 of the NKp46 molecule, more preferably both domains 1 and 2. In another prefrred embodiment this fragment comprises only domain 2 of the NKp46 molecule. Alternatively, the NKp44 fragment comprises at least one of domains 1 and 2 of the NKp44 molecule, more preferably both domains 1 and 2. In another prefrred embodiment this fragment comprises only domain 2 of the NKp44 molecule.

In one alternative embodiment, the method of invention employs a complex containing as the active segment an Ig fragment. This Ig fragment is particularly the Fc portion of an Ig molecule.

In another alternative embodiment, the method of the invention comprises as an active segment a cytotoxic moiety. This cytotoxic moiety may be selected from cytotoxins or anticellular agents capable of killing and/or suppressing the growth and/or cell division of the target cell. More specifically, the cytotoxin or anticellular agent may a synthetic toxin or a plant-, fungus-, or bacteria-derived toxin.

This toxin may be selected from the group consisting of A chain toxin, ribosome inactivating protein, α-sarcin, aspergillin, restrictocin, ribonuclease, diphtheria toxin, *Pseudomomas* exotoxin, an endotoxin or the lipid A moiety of an endotoxin.

An alternative aspect of the present invention relates to a method for the diagnosis and imaging of pathologies, specifically tumors. This method comprises the steps of introducing an imaging agent into the blood stream of a subject, and detecting and quantitating the binding of the imaging agent to a NKp46 or NKp44 ligand expressed on malignant cells. The imaging agent comprises a complex having a first, target recognition segment, capable of specifically recognizing and binding to malignant cells. The recognition segment comprises at least one of NKp46, NKp30, NKp44 or a biologically functional fragment thereof. The complex also comprises a second, active segment, which is an imaging moiety, which may be a paramagnetic, radioactive or fluorogenic agent.

### Brief Description of the Figures

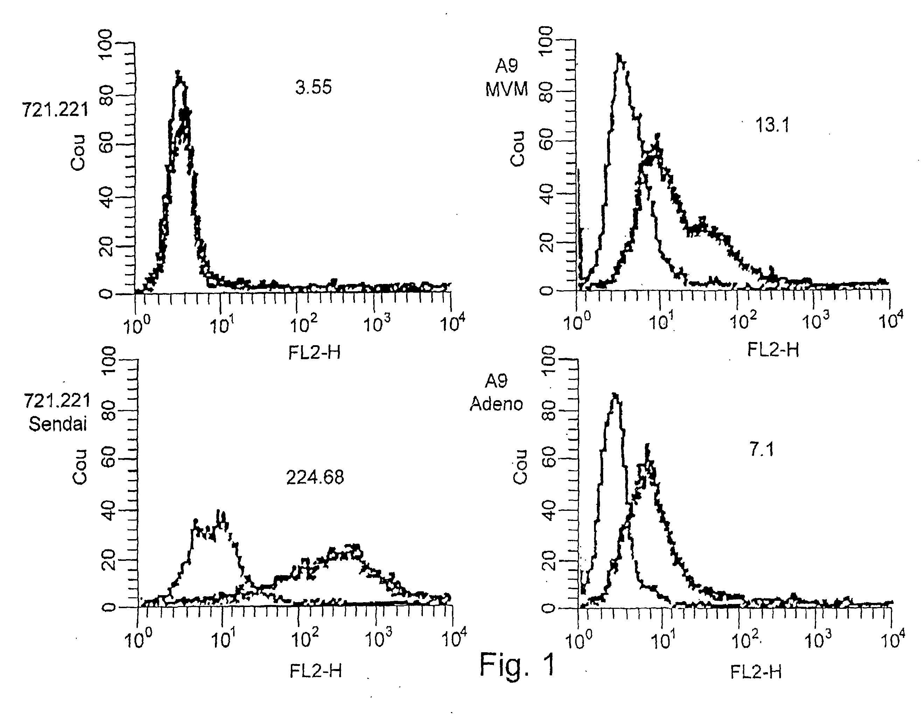

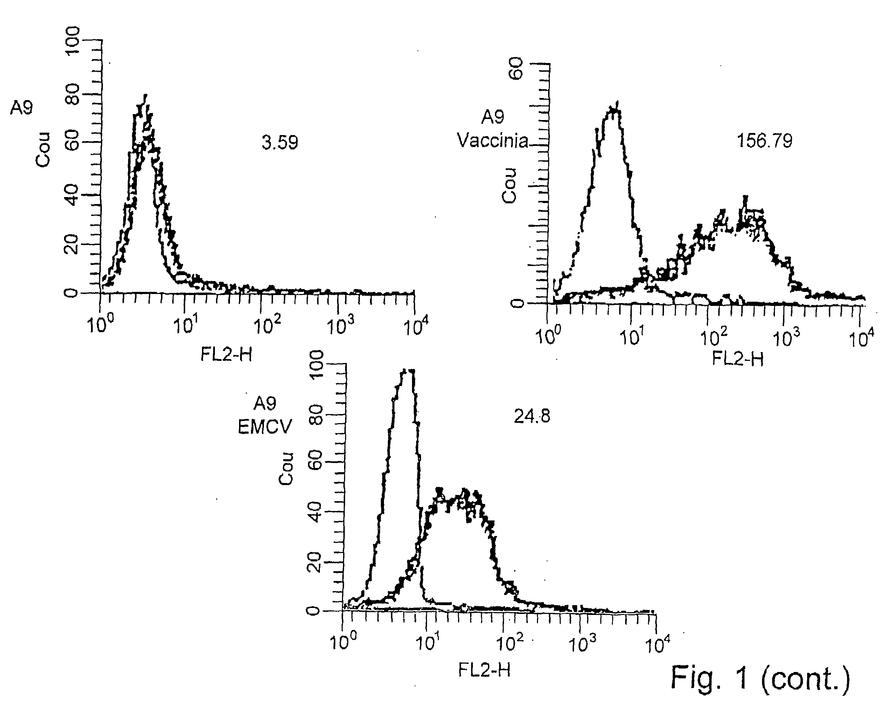

**Figure 1** - *Upregulation **of** NKp46 ligand expression post various virus infection*
   721.221 cells (10⁶/ml) were incubated overnight with 100 µl/ml of Sendai virus-containing supernatant. A9 cells (half-confluent culture flask passaged 24 hr before) were incubated for 3 hr with either MVM (1.5x10⁷ units/ml), EMCV (250 µl/ml of EMCV-containing supernatant), Adenovirus (5×10⁶units/ml) or Vaccinia (4 ml Vaccinia-containing supernatant). Following incubation of A9 cells with the different viruses, virus-containing media were removed and culture media was added. 18 to 24 hr after infection cells (infected or uninfected) were harvested, washed and stained either with the NKp46-Ig fusion protein (bold line) or with control CD99-Ig (plain line), followed by PE-conjugated anti-human Fc. Numbers inside Panels represent the MFI of the NKp46-Ig staining. Results are from a representative experiment out of two performed. Abbreviations: Cou is for counts.

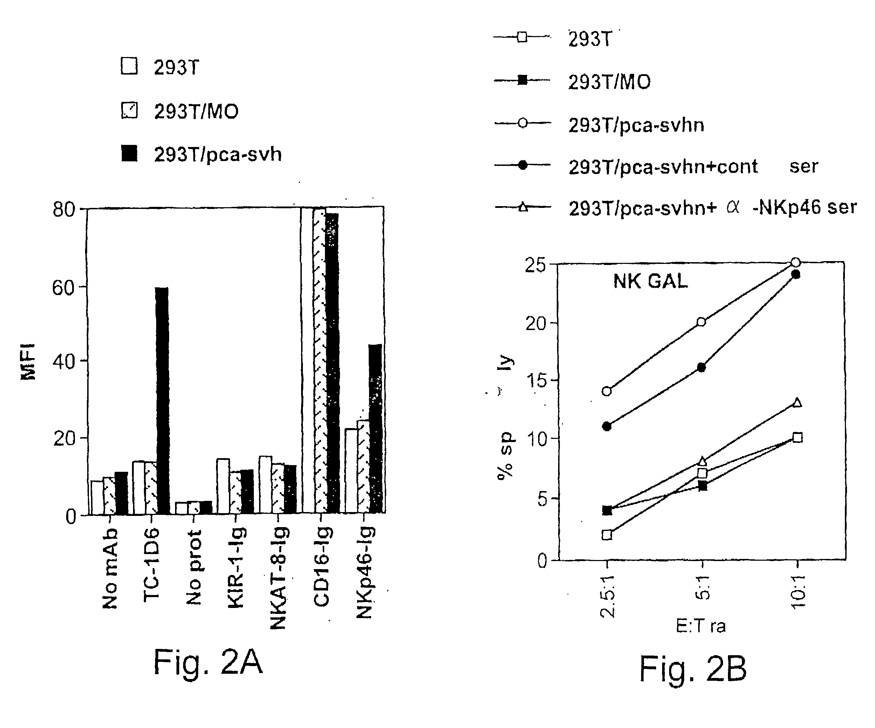

**Figure 2A-C** - *NKp46 Ig mediated enhanced lysis of 293T cells transfected witk the Sendai HN cDNA*
   **2A:** NKp46-Ig binding to 293T cells transfected with Sendai HN cDNA. 293T cells were either transiently transfected with a control (cont) PCDNA3 plasmid (293T/MOCK) or with a cDNA coding for HN of the Sendai Virus (293T/pca-svhn). 48 hr later cells were stained either with TC-1D6 mAb or with KIR-1, NKAT-8, CD16 and NKp46 Ig-fusion proteins. MFI indicates Median Fluorescence Intensity. Controls were the same cells stained either with FITC-conjugated anti-mouse (anti=α) antibodies (No mAb), or with PE-conjugated anti-human Fc antibodies (no Ig-fusion protein). Results are of a representative experiment out of three performed.
   **2B and 2C:** Enhanced lysis of 293T cells transfected with the Sendai HN cDNA is blocked by anti-NKp46 and anti-HN mAb. 48 h after transfection, 293T, 293T/MOCK and 293T/pca-svhn cells were labeled with ³⁵S-Met and washed. Labeled cells were then incubated, at the effector to target (E:T) ratios indicated, with NK GAL pre-incubated with either control serum or with anti-NKp46 serum (B). Alternatively, labeled cells were incubated with the various mAbs for 1 hr on ice, washed, and then incubated with NK GAL (C). In all experiments NK cells were pre-incubated with 50% of human serum (ser) for 1 h on ice and then washed to block Fc receptors. Results are representative experiment of three performed. Abbreviations: Prot (protein), specific lysis (sp ly), ratio (ra), MO (mock).

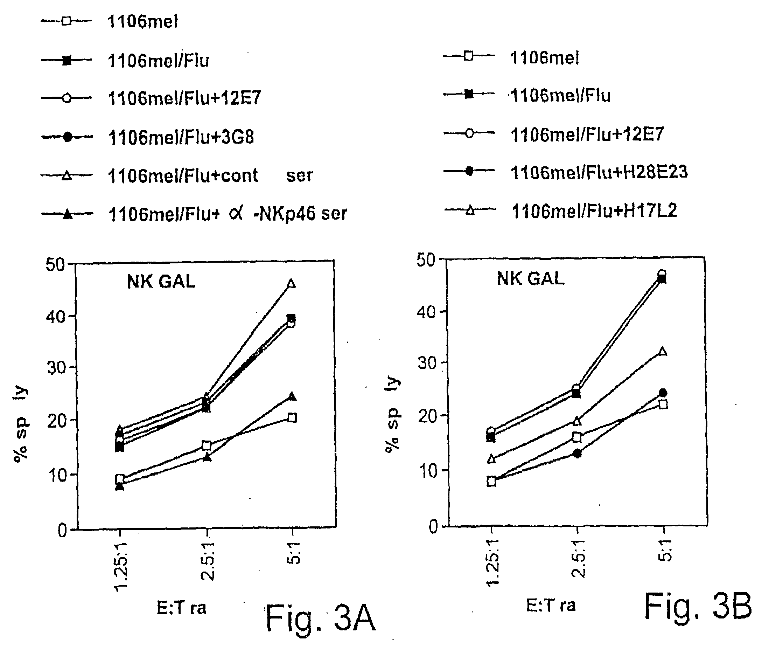

**Figure 3A-C** - *Lysis of IV-infected 1106mel cells by NK GAL and derived clones*
   **3A:** NK cells were incubated either with no antibody, with the control (cont) anti-CD99 mAb (12E7), with anti-CD16 mAb (anti=a) (3G8), with control serum (cont ser), or with anti-NKp46 serum, for 1 h on ice. NK cells were then washed and then incubated either with 1106mel cells or with IV-infected 1106mel cells at the E:T ratios (=ra) indicated.
   **3B:** IV-infected 1106mel cells were incubated with various mAb for 1 hr on ice and then incubated with NK GAL at the indicated E:T ratios. Results are representative experiment of three performed.
   **3C**: 28 NK clones were derived from the NK GAL by limiting dilution. Blocking experiments with serum containing polyclonal antibodies were performed as described in legend to Fig. 1. The E:T ratio was 5:1. The percentages indicated in the Figure are the % of clones that behaved similarly to the one NK clone presented.. Results are representative experiment of two performed. In all experiments NK cells were pre-incubated with 50% of human serum for 1 hr on ice and then washed to block Fc receptors. Abbreviations: sp ly is for specific lysis, cont (control), ser (serum) and cl is for clones.

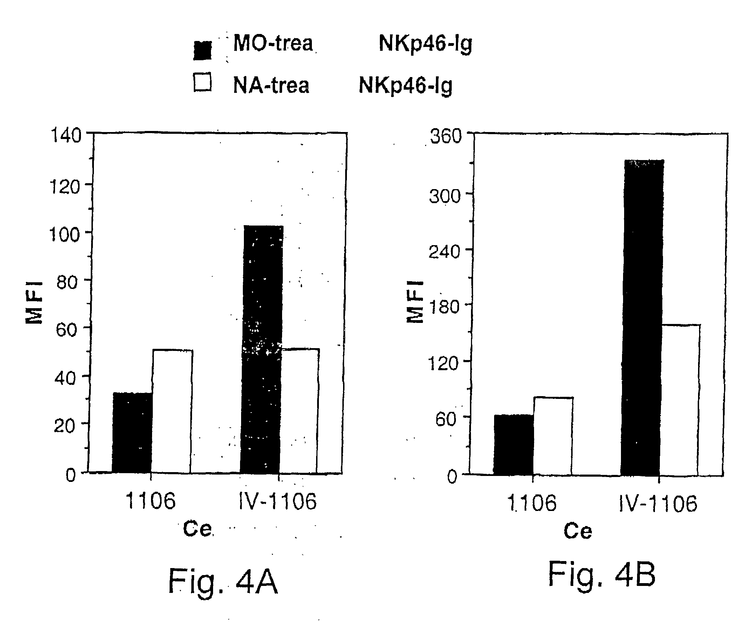

**Figure 4 -** *Effect of NA treatment on the binding of NKp46-Ig to IV-infected and non-infected 1106 cells*
   NKp46-Ig was incubated with 0.01 U of insoluble neuraminidase attached to beaded agarose (N-5254, SIGMA, St. Louis, Missouri) or with PBS (mock treated- MO-trea) for 1 h at 17°C on a roller. IV-infected, or uninfected 1106 cells (Ce) were washed, and stained either with NA-treated (trea) or mock-treated NKp46-Ig, followed by PE-conjugated anti-human Fc. MFI indicates Median Fluorescence Intensity. A and B panels are two representative experiments of eight performed. The activity of NA was confirmed by SDS-PAGE analysis of NA-treated fetuin, a highly sialylated protein.

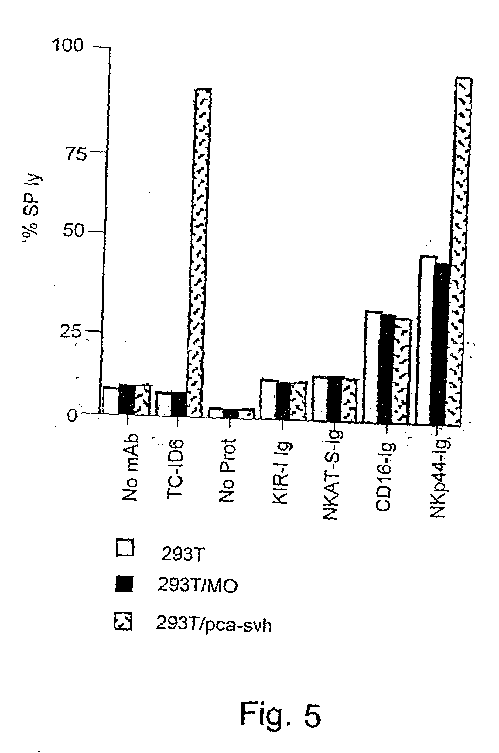

**Figure 5 -** *NKp44-Ig binding to 293T cells transfected with Sendai HN cDNA* 293T cells were either transiently transfected with a control PCDNA3 plasmid (293T/MOCK) or with a cDNA encoding for HN of the SV (293T/pca-svh) using the Fugene transfection reagent (Boehringer Mannheim). 48 hr later cells were stained either with TC-1D6 mAb or with KIR-1, NKAT-8, CD16 and NKp46 Ig-fusion proteins. MFI indicates Median Fluorescence Intensity. Controls were the same cells stained either with FITC-conjugated anti-mouse antibodies (No mAb), or with PE-conjugated anti-human Fc antibodies (no Ig-fusion protein). Results are of a representative experiment out of two performed. Abbreviations: sp ly is for specific lysis, MO (MOCK), prot (protein).

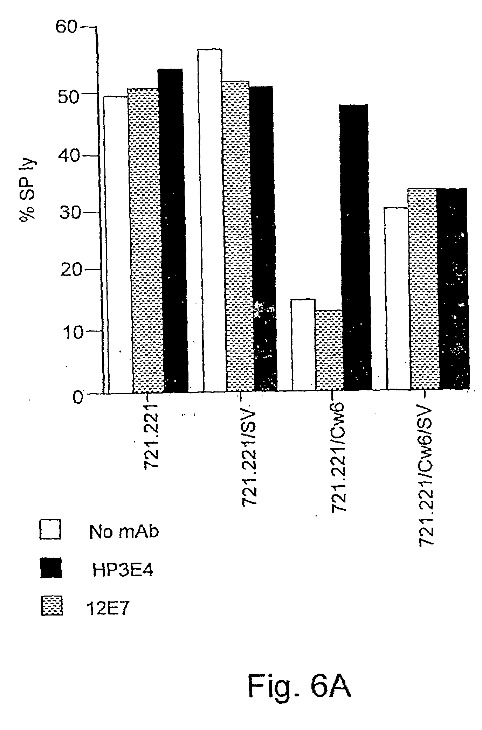

**Figure 6A-B** *SV Infection of 721.221 expressing class I MHC proteins resulted in abrogation of the inhibition*
   NK clones were derived from various donors using the autoMACS instrument (Miltenyi Biotec Inc). Clones were stained for the presence of NKp44 and NKp46 proteins using the anti-NKp44 and NKp46 serum and for the presence of NK inhibitory receptors using the HP3E4 mAb. The E: T ratio of the NK clone presented was around 3:1. Results are representative experiment of two performed.
   **6A:** shows the NK clone was first incubated with the indicated mAb for 1hr and then incubated with the labeled target cells.
   **6B**: shows labeled target cells that were first incubated with the indicated mAb for 1hr on ice and then incubated with NK cells.
   Abbreviations: sp ly is for specific lysis.
**Figure 7** *- Effect of HA blocking on the binding of NKp46-Ig and NKp44-Ig to IV-infected and non-infected 1106mel cells*
   NKp44-Ig (upper panel) or NKp46-Ig (lower panel) were incubated with or without 40 µg purified HA protein. Mixtures were next incubated with IV-infected or non-infected 1106mel cells and stained with PE-conjugated goat anti-human Fc. MFI indicates Median Fluorescence Intensity.
**Figure 8 -** *Effect of NA treatment on the binding of NKp44-Ig to IV-infected and non-infected 1106mel cells*
   MFI indicates Median Fluorescence Intensity. NKp44-Ig was incubated with 0.01U of insoluble neuraminidase attached to beaded agarose (N-5254, SIGMA, St. Louis, Missouri) or with PBS (as control) for 1 h at 17°C on a roller. IV-infected, or non-infected 1106mel cells were washed, and stained either with NA-treated or mock-treated NKp44-Ig, followed by PE-conjugated anti-human Fc. Figure shows one representative experiment of two performed. The activity of NA was confirmed by SDS-PAGE analysis of NA-treated fetuin, a highly sialylated protein. Abbreviations: N pr (no protein) and NA trea (NA treatment).

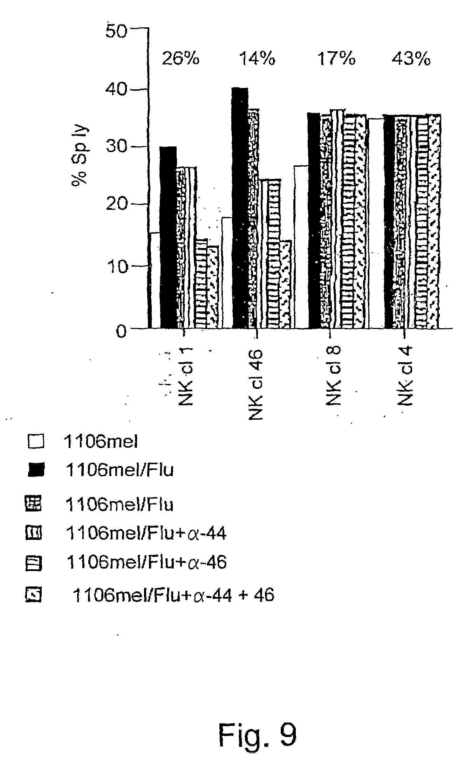

**Figure 9** - *Lysis of IV-infected 1106mel cells by NK clones*
   64 NK clones were derived from the NK line MB by limiting dilution. Blocking experiments with serum containing polyclonal antibodies were performed as described in experimental procedures. The E:T ratio was 2:1. The percentages indicated in the Figure are the % of clones that behaved similarly to the one NK clone presented. 1106mel/Flu+ C indicates incubation with control serum. Results are representative experiment of two performed. In all experiments NK cells were pre-incubated with 50% of human serum for 1hr on ice and then washed to block Fc receptors. Abbreviations: sp ly (specific lysis), cl (clon) and α (anti).

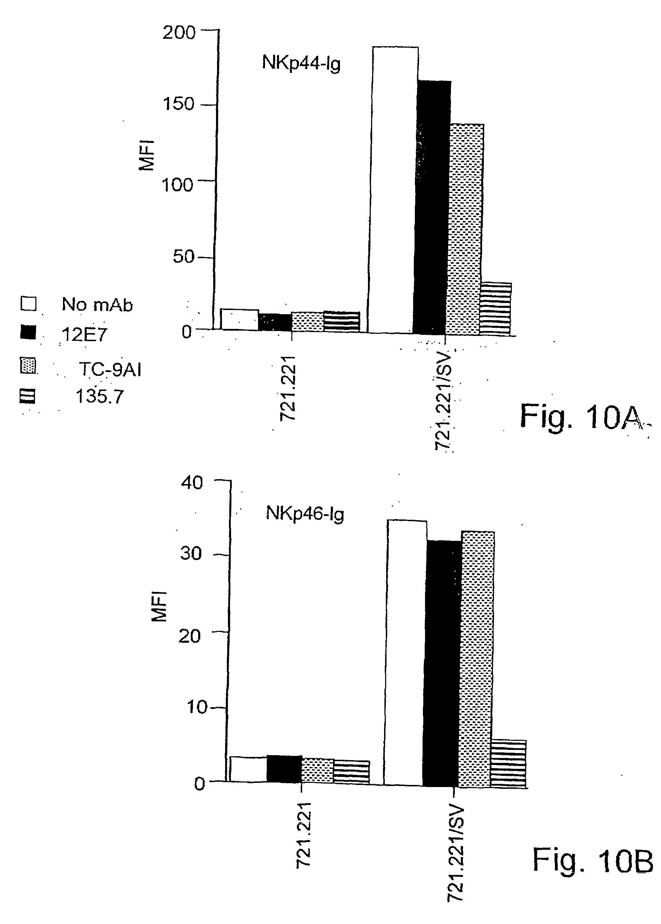

**Figure 10A-D -** *Domain 2 is responsible for the interaction with the HN viral protein of SV*
   721.221 cells were infected with 100 µl of SV supernatant. After overnight incubation, infected cells were washed and incubated on ice with the various mAbs, for 1 h. Next, cells were washed and assayed for staining with 10 µg of the appropriate Ig-fusion protein as previously described [Mandelboim, O., *et al.,* (1999) *ibid*.], MFI indicates Median Fluorescence Intensity.

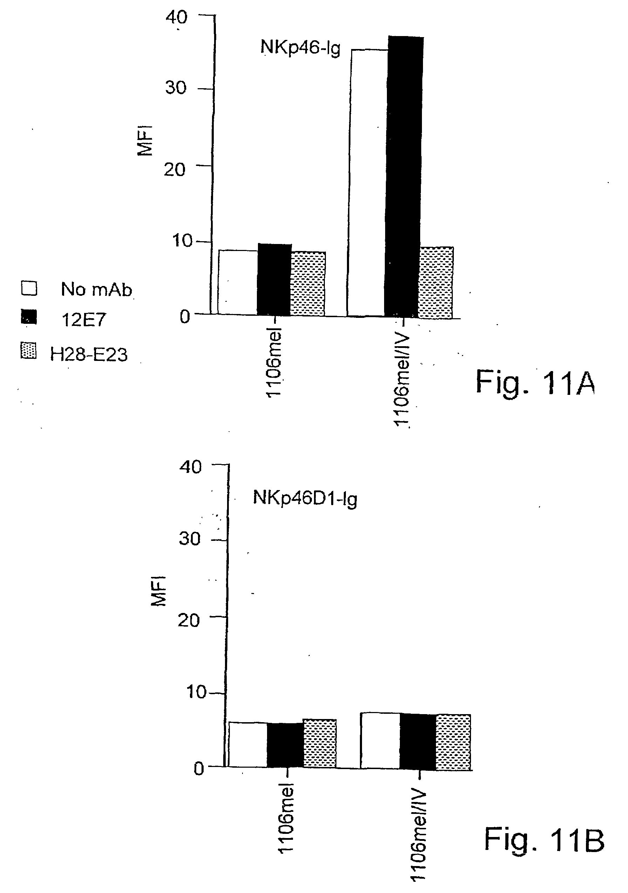

**Figure 11A-C-** *Domain 2 is responsible for the interaction with the HA viral protein of IV*
   1106mel cells were infected with 1000 u/ml of IV. After overnight incubation, infected cells were washed and incubated on ice with the various mAbs, for 1h. Next, cells were washed and assayed for staining with 10 µg of the appropriate Ig-fusion protein as previously described [Mandelboim, O., *et. al*., (1999) *ibid*.]. MFI indicates Median Fluorescence Intensity.

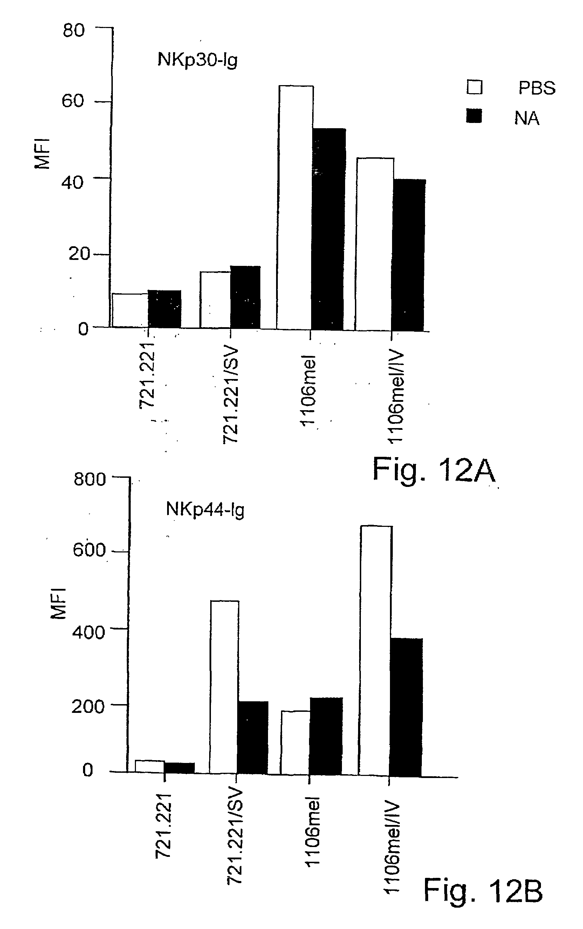

**Figure 12A-E** - *Effect of NA treatment on the binding of NKp46d2-Ig. to infected and non-infected cells*
   The different indicated fusion proteins were incubated with 0.01 U of insoluble neuraminidase (NA) attached to beaded agarose (N-5254, SIGMA, St. Louis, Missouri) or with PBS (mock treated) for 1 h at 17°C on a roller. IV-infected, or uninfected 1106 cells, as well as SV-infected, or non-infected 721.221 cells were washed, and stained either with NA-treated (trea) or PBS-treated fusion proteins, followed by PE-conjugated anti-human Fc. MFI indicates Median Fluorescence Intensity. The activity of NA was confirmed by SDS-PAGE analysis of NA-treated fetuin, a highly sialylated protein.
   **12A**: shows incubation with the NKp30-Ig fusion protein.
   **12B:** shows incubation with the NKp44-Ig fusion protein.
   **12C**: shows incubation with the NKp46-Ig fusion protein.
   **12D:** shows incubation with the NKp46D1-Ig fusion protein.
   **12E**: shows incubation with the NKp46D2-Ig fusion protein.

### Detailed Description of the Invention

A number of methods of the art of molecular biology are not detailed herein, as they are well known to the person of skill in the art. Such methods include site-directed mutagenesis, PCR cloning, expression of cDNAs, analysis of recombinant proteins or peptides, transformation of bacterial and yeast cells, transfection of mammalian cells, and the like. Textbooks describing such methods are e.g., Sambrook et al., Molecular Cloning A Laboratory Manual, Cold Spring Harbor Laboratory; ISBN: 0879693096, 1989, Current Protocols in Molecular Biology, by F. M. Ausubel, ISBN: 047150338X, John Wiley & Sons, Inc. 1988, and Short Protocols in Molecular Biology, by F. M. Ausubel et al. (eds.) 3rd ed. John Wiley & Sons; ISBN: 0471137812, 1995. These publications are incorporated herein in their entirety by reference. Furthermore, a number of immunological techniques are not in each instance described herein in detail, as they are well known to the person of skill in the art. See e.g., Current Protocols in Immunology, Coligan et al. (eds), John Wiley & Sons. Inc., New York, NY.

The present invention provides a novel approach to the treatment and/or diagnosis (imaging) of different pathologies such as tumors and pathogenic viral infections, based on recognition of different ligands expressed on tumor or viral infected cells, by the NK cells activating receptors NKp46, NKp44 and NKp30.

In diagnosis, the method of the invention will have the ability to provide an image of the tumor, for example through magnetic resonance imaging, X-ray imaging, computerized emission tomography and the like, by means of a complex of the invention comprising a detectable imaging moiety.

In therapy, complexes of the invention are designed to have a cytotoxic or otherwise anticellular effect against desired target cells, by suppressing the growth or cell division of such cells.

Thus, in a first aspect, the present invention relates to a targeting complex that specifically recognizes a ligand molecule expressed on the surface of target cells and is capable of targeting an active substance to the target cell. This complex comprises:
a. a target recognition segment comprising at least one of the NKp46, NKp30 and NKp44 proteins or a functional fragment thereof; and
b. an active segment comprising the active substance which may be a cytotoxic moiety, an imaging moiety or an Ig fragment.

In a specifically preferred embodiment the target recognition segment is a NKp46 fragment which-comprises at least one of domains 1 and 2 of the NKp46 molecule. Preferably, the target recognition segment comprises both domains 1 and 2 and has the amino acid sequence substantially as denoted by SEQ ID NO:4 or the amino acid sequence of its isoform, substantially as denoted by SEQ ID NO:13. In another preferred embodiment, the target recognition segment may comprise only domain 2 of the NKp46 molecule, as denoted by SEQ ID NO:22 and 23.

Alternatively, the target recognition segment may be a NKp44 fragment which comprises at least one of domains 1 and 2 of the NKp44 molecule. Preferably the target recognition segment comprises both domains 1 and 2 and has the amino acid sequence substantially as denoted by SEQ ID NO:9. In another preferred embodiment, the target recognition segment may comprise only domain 2 of the NKp44 molecule, as denoted by SEQ ID NO: 24. The target recognition segment may alternatively comprise the NK activating molecule NKp30, as denoted by SEQ ID NO:17.

It is to be appreciated that the recognition segment of the invention may comprise more than one unit of domain 1 and 2 or alternatively only domain 2 of any of NKp46, NKp30 and NKp44. Creation of such multiunit segment may increase the avidity of the recognition segment to the target molecule.

By "functional fragments" is meant "fragments", "variants", "analogs" or "derivatives" of the molecule. A "fragment" of a molecule, such as any of the nucleic acid or the amino acid sequence of the present invention is meant to refer to any nucleotide or amino acid subset of the molecule. A "variant" of such molecule is meant to refer to a naturally occurring molecule substantially similar to either the entire molecule or a fragment thereof. An "analog" of a molecule is a homologous molecule from the same species or from different species. The amino acid sequence of an analog or derivative may differ from the specific molecule, e.g. the NKp46, NKp30 or NKp44 molecule, used in the present invention when at least one residue is deleted inserted or substituted.

By "functional", is meant having same biological function, for example, having identical ability to recognize and/or bind the ligand.

Another specifically preferred embodiment, relates to the complex the invention being a fusion protein comprising as the active segment an Ig fragment. This Ig fragment is preferably the Fc portion of an Ig molecule, and is encoded by the amino acid sequence of SEQ ID NO:5.

The complement system is one of the major effector mechanisms of humoral immunity. It is activated principally by the binding of the first classical pathway component, C1, to the Fc portion of antigen-complexed antibody molecules. Therefore, the fusion proteins comprising NKp44, NKp30 or NKp46 and the Fc portion of an Ig molecule, can serve as target for the complement system *in vivo.*

Alternatively, the complex of the present invention is a conjugate comprising as an active segment a cytotoxic moiety. This cytotoxic moiety may be a cytotoxin or any anticellular agent, which is capable of killing and/or suppressing the growth or cell division of said target cell.

In general, for therapeutic purposes, the invention involves the use of any pharmacological agent that can be conjugated to the targeting segment of the complex of the present invention and delivered in active form to the target cells. Exemplary anticellular agents include chemotherapeutic agents, radioisotopes as well as cytotoxins. Chemotherapeutic agents include, but are not limited to, hormones such as steroids, antimetabolites such as cytosine arabinoside, fluorouracil, methotrexate or aminopterin; an anthracycline; mitomycin C; a *Vinca* alkaloid; demecolcine; etoposide; mithramycin; or an antitumor alkylating agent such as chlorambucil or melphalan.

Other embodiments may include agents such as bacterial endotoxins or the lipid A moiety of such bacterial endotoxin. In any event it is proposed that agents such as these may be successfully conjugated to the targeting segment (preferably any one of NKp46, NKp44 and NKp30 domains 1 and 2 or at least one of domains 1 and 2, preferably domain 2) of the complex of the present invention, in a manner that will allow their targeting, internalization, release or presentation to the target cells as required, using known conjugation technologies [for example, Ghose, *et al.,* Critical Reviews in Therapeutic Drug Carrier Systems, 3:256-359 (1987)].

In certain preferred embodiments, agents for therapeutic application will include generally a synthetic toxin or a plant-, fungus-, or bacteria-derived toxin, such as an A toxin, a ribosome inactivating protein, α-sarcin, aspergillin, restirictocin, a ribonuclease, diphtheria toxin, *Pseudomonas* exotoxin, an endotoxin or the lipid A moiety of an endotoin, to mention just a few examples. The most preferred toxin moiety for use in connection with the invention is diphtheria toxin.

It is contemplated that most therapeutic applications of the present invention will involve the targeting of a toxin moiety to the tumor or to the pathogenic virus-infected cells. This is due to the much greater ability of most toxins to deliver a cell killing effect as compared to other potential agents. Nevertheless, under some circumstances, such as when the target ligand of the NKp46, NKp44 or NKp30 does not internalize via a route consistent with efficient intoxication by targeted toxin-complexes, where toxins one may be substituted by chemotherapeutic agents such as antitumor drugs, other cytokines, antimetabolites, alkylating agents, hormones, and the like.

As used in the present application, the terms "cytotoxic" and "cytolytic" when used to describe the activity of the complex of the present invention (particularly when a cytotoxic moiety is selected as the active segment) are intended to be synonymous. In general, cytotoxic activity relates to killing of target cells by any of a variety of biological, biochemical, or biophysical mechanisms. Cytolysis, refers more specifically to activity in which the effector lyses the plasma membrane of the target cell, thereby destroying its physical integrity. This results in the killing of the target cell.

In yet another alternative embodiment, the complex of the present invention may be a conjugate comprising as the active segment an imaging moiety. Moreover, in the case of radioactive isotopes for therapeutic and/or diagnostic application, one might mention iodine¹³¹, iodine¹²³, technecium^{99m}, indium¹¹¹, rhenium¹⁸⁸, rhenium¹⁸⁶, galium⁶⁷, copper⁶⁷, yttrium⁹⁰, iodine¹²⁵ or astatine²¹¹. Where the aim is to provide an image of the tumor for diagnosis and monitoring purposes, one will desire to use an agent that is detectable upon imaging, such as a paramagnetic, radioactive or fluorogenic agent. Many agents are known in the art to be useful for imaging purpose. Paramagnetic ions may be, for example, ions such as chromium, manganese, iron, cobalt, nickel, copper, neodymium, samarium, holmium or erbium. Ions useful in other contexts, such as x-ray imaging, include but are not limited to lanthanum, gold, lead and bismuth.

The imaging complex of the invention may be conjugated to a detectable moiety such fluorescent compound. When the fluorescently-labeled complex is exposed to light of the proper wavelength, its presence can be detected by fluorescence. Amongst the most commonly used fluorescent labeling compounds are fluorescein, isothiocyanate, rhodamine, phycoerythrine, phycocyanin, allophycocyanin, o-phthaldehyde and fluorescamine.

The complex can also be detectably labeled using fluorescence emitting metals such as ¹⁵²E, or others of the lanthanide series. These metals can be attached to the targeting segment using such metal chelating groups as diethylenetriamine pentaacetic acid (ETPA).

The complex can also be detectably labeled by being coupled to a chemiluminescent compound. The presence of the chemiluminescent-tagged targeting segment is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used as a label in the complex of the present invention. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

It is to be appreciated that the targeting segment of the complex of the invention may be conjugated to the active segment (cytotoxic or imaging moieties), either directly or indirectly by conjugating or coupling these segments to any one of lipid backbone or carbohydrate backbone.

In a specific embodiment, the complex of the present invention may be targeted to a diseased cell.

As used to describe the present invention, "target cells" are the cells that are killed by the cytotoxic activity of the complex of the invention (wherein the active segment comprises a therapeutic, e.g., cytotoxic moiety or Ig), or cells detected by the complex of the invention (where the active segment comprises a detectable imaging moiety). The target cells express the ligand for any one of NKp46, NKp44 and NKp30 molecules and include, in particular, cells that are malignant or otherwise derived from solid as well as non-solid tumors.

As used herein to describe the present invention, "cancer", "tumor" and "malignancy" all relate equivalently to a hyperplasia of a tissue or organ. If the tissue is a part of the lymphatic or immune systems, malignant cells may include non-solid tumors of circulating cells. Malignancies of other tissues or organs may produce solid tumors. In general, the complex of the present invention as well as the composition and the methods of the present invention may be used in the treatment of non-solid and solid tumors, and for monitoring and imaging of solid tumors (wherein the selected active segment is an imaging moiety).

Alternatively, the complex of the present invention may be directed to cells that are infected by pathogenic viruses such as HIV, EBV, CMV, Vaccinia, MVM, ECMV, Herpes or Influenza virus.

More specifically, the pathogenic virus may be any one of variety of viruses including but not limited to, Influenza virus, human immunodeficiency virus, Epstein-Barr virus, cytomegalovirus, Vaccinia virus, MVM, ECMV and Herpes virus.

As used to describe the present invention, a "pathogenic virus" is a virus causing disease in a host. The pathogenic virus infects cells of the host animal and the consequence of such infection is deterioration in the health of the host. Pathogenic viruses envisioned by the present invention include, but are not limited to, HIV, EBV, CMV, Vaccinia, Herpes, MVM, ECMV and Influenza.

In a specifically preferred embodiment, where said target cell is a pathogenic virus-infected cell, the complex of the invention has a target recognition segment that is capable of binding to a ligand expressed on the surface of said viral infected target cell, this binding is mediated by sialic acid.

Alternatively, the complex of the invention may also bind to a free virus. This binding as well is mediated by sialic acid.

As shown in Examples 6 and 9, the interaction of the NKp46 and NKp44 molecules, respectively, with their ligands (HA or HN) was mediated by sialic acid. Moreover, it was shown that sialic acid is necessary but not sufficient for the interaction.

A number of mammalian sialic acid receptors have been defined [Varki, *A., et al*., FASEB J. 11:248-55 (1997)], raising the question of why these are not sufficient for binding NKp46 or NKp44. While cellular sialic acid receptors may be expressed on the target cells that were utilized, they may not be expressed in sufficient quantities to function like viral HA, which is abundantly expressed. It is also possible that the cellular lectins are sequestered or otherwise inactivated until the appropriate circumstances arise for their use.

Examples 6 and 9 describe the analysis of the binding of HA to NKp46 and NKp44. The dissociation constant of HA for sialic acids is in the mM range, too low for a monomeric interaction to account for either the stable binding of NKp46-Ig detected by flow cytometry (which requires dissociation constants less than 0.1 µM), or the potency of NKp46-Ig in blocking viral hemagglutination. This implies either a multimeric interaction of HA with NKp46 or that NKp46 interacts more intimately with HA after contact is initiated by the sialic acid binding. Even in the former case, NKp46 would need special properties to distinguish it from other cellular glycoproteins, since terminal sialic residues are ubiquitous on N-linked oligosaccharides. One possibility is that NKp46 (which is thought to have a single N-linked oligosaccharide) multimerizes in such a fashion as to enable multivalent interaction of sialic acid with a single HA complex, which as a trimer possesses three sialic acid binding sites. While recombinant NKp46-Ig and NKp44-Ig are expected to be bivalent molecules, the oligomeric state of cellular NKp46 or NKp44 is unknown.

In a second aspect, the present invention relates to an expression vector comprising a nucleic acid sequence coding for a NKp46-Ig fusion protein. This nucleic acid comprises:
a. a DNA sequence encoding a target recognition segment, which segment comprises NKp46 or functional fragments thereof. This NKp46 fragment preferably comprises at least one of domains 1 and 2 of the NKp46 molecule. Most preferably, the target recognition segment comprises both domains 1 and 2 of the NKp46 molecule and is encoded by the nucleic acid sequence substantially as denoted by SEQ ID NO:1; or by the NKp46 isoform substantially as denoted by SEQ ID NO:11, alternatively only domain 2 as denoted by any one of SEQ ID NO: 19 and NO:20, and
b. a DNA sequence encoding an active segment which is the Fc portion of an Ig molecule, said DNA having the nucleic acid sequence substantially as denoted by SEQ ID NO:2.

In an alternative embodiment the expression vector of the present invention comprises a nucleic acid sequence coding for a NKp44-Ig fusion protein. This nucleic acid sequence comprises:
a. a DNA sequence encoding a target recognition segment, which segment comprises NKp44 or functional fragments thereof. This NKp44 fragment preferably comprises at least one of domains 1 and 2 of the NKp44 molecule. Most preferably, the target segment comprises both domains 1 and 2 and is encoded by the nucleic acid sequence substantially as denoted by SEQ ID NO:7, alternatively, only domain 2 as denoted by SEQ ID NO: 21; and
b. a DNA sequence encoding an active segment which is the Fc portion of an Ig molecule, said DNA having the nucleic acid sequence substantially as denoted by SEQ ID NO:2.

An expression vector coding for the NKp30-Ig fusion protein is whithin the scope of the present invention. Such expression vector comprises the nucleic acid sequence of the NKp30 domain 1 and 2 and have the nucleic acid sequence as denoted by SEQ ID NO:15.

As used herein, the term "nucleic acid" refers to polynucleotides such as deoxyribonucleic acid (DNA), and, where appropriate, ribonucleic acid (RNA). The terms should also be understood to include, as equivalents, analogs of either RNA or DNA made from nucleotide analogs, and, as applicable to the embodiment being described, single-stranded and double-stranded polynucleotides.

The expression vector of the invention may further comprise operably linked regulatory elements. The term "operably linked" is used herein for indicating that a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein-coding regions, in the same reading frame.

Accordingly, the term control and regulatory elements includes promoters, terminators and other expression control elements. Such regulatory elements are described by Goeddel [Goeddel, Gene Expression Technology: Methods in Enzymology 185, Academic Press, San Diego, Calif. (1990)].

"Vectors", as used herein, encompass plasmids, viruses, bacteriophage, integratable DNA fragments, and other vehicles, which enable the integration of DNA fragments into the genome of the host. Expression vectors are typically self-replicating DNA or RNA constructs containing the desired gene or its fragments, and operably linked genetic control elements that are recognized in a suitable host cell and effect expression of the desired genes. These control elements are capable of effecting expression within a suitable host. Generally, the genetic control elements can include a prokaryotic promoter system or an eukaryotic promoter expression control system. This typically includes a transcriptional promoter, an optional operator to control the onset of transcription, transcription enhancers to elevate the level of RNA expression, a sequence that encodes a suitable ribosome binding site, RNA splice junctions, sequences that terminate transcription and translation and so forth. Expression vectors usually contain an origin of replication that allows the vector to replicate independently of the host cell.

A vector may additionally include appropriate restriction sites, antibiotic resistance or other markers for selection of vector containing cells. Plasmids are the most commonly used form of vector but other forms of vectors which serves an equivalent function and which are, or become, known in the art are suitable for use herein. See, e.g., Pouwels *et al.* Cloning Vectors: a Laboratory Manual (1985 and supplements), Elsevier, N.Y.; and Rodriquez, *et al.* (eds.) Vectors: a Survey of Molecular Cloning Vectors and their Uses, Buttersworth, Boston, Mass (1988), which are incorporated herein by reference.

Also, a specific embodiment of the invention relates to a host cell transformed with the expression vectors of the invention. Suitable host cells include prokaryotes, lower eukaryotes, and higher eukaryotes. Prokaryotes include gram negative and gram positive organisms, e.g., *E. coli* and *B. subtilis.* Lower eukaryotes include yeast, *S*. *cerevisiae* and Pichia, and species of the genus *Dictyostelium.* Higher eukaryotes include established tissue culture cell lines from animal cells, both of non-mammalian origin, e.g., insect cells and birds, and of mammalian origin, e.g., human and other primate, and of rodent origin.

A specifically preferred embodiment relates to a NKp46-Ig fusion protein. This fusion protein has the amino acid sequence substantially as denoted by SEQ ID NO:6 or by its isoform as denoted by SEQ ID NO:14, encoded by the nucleic acid sequence substantially as denoted by SEQ ID NO:3 and SEQ ID NO:12, respectively.

In another embodiment, the invention relates to the NKp46D2-Ig fusion protein as described in Example 11.

Another specifically preferred embodiment relates to a NKp44-Ig fusion protein. This fusion protein comprises the amino acid sequence substantially as denoted by SEQ ID NO:10, that is encoded by the nucleic acid sequence substantially as denoted by SEQ ID NO:8. In another embodiment, the invention relates to the NKp44D2-Ig fusion protein.

Alternatively, the fusion protein of the invention may comprise as the targeting segment the NKp30 molecule (denoted by SEQ ID NO:18).

A heterologous fusion protein is a fusion protein made of segments, which are naturally not normally fused in the same manner. Thus, the fusion product of the NKp46, NKp30 or NKp44 (particularly domains 1 and 2, or at least one of domains 1 and 2) molecule with the Fc portion of an Ig molecule, is a continuous protein molecule having sequences fused by a typical peptide bond, typically made as a single translation product and exhibiting properties derived from each source peptide.

Another aspect of the present invention relates to an antibody that specifically recognizes and binds to the fusion protein NKp46-Ig of the invention.

In yet another embodiment of the present aspect the invention relates to an antibody that specifically recognizes and binds to the fusion protein NKp44-Ig of the invention.

Additionally, the invention relates to an antibody that specifically recognizes and binds to an epitope on a protein, wherein said protein is a ligand for the NK cell activating receptor NKp46. As described in Example 3, in order to identify the NKp46 putative ligands, mice were immunized with SV-infected cells, and the different antibodies were examined for their ability to block binding of the NKp46-Ig to SV infected cells. One such antibody that efficiently blocked the binding of the NKp46-Ig to SV infected cells is designated 135.7.

Therefore, in a specifically preferred embodiment the invention relates to the antibody designated 135.7. This antibody specifically recognizes and binds to a NKp46 ligand. As described in Example 3, this ligand is a protein having a molecular weight of approximately 70Kd. This protein was found out to be the HN glycoprotein.

A preferred embodiment of the invention relates to the antibodies against the NKp46-Ig and the NKp44-Ig fusion proteins and against the NKp46 and the NKp44 ligands. These antibodies are selected from the group consisting of monoclonal and polyclonal antibodies, preferably monoclonal antibodies.

The generation of polyclonal antibodies against proteins is described in Chapter 2 of Current Protocols in Immunology, Wiley and Sons Inc.

Monoclonal antibodies may be prepared from B cells taken from the spleen or lymph nodes of immunized animals, in particular rats or mice, by fusion with immortalized B cells under conditions which favor the growth of hybrid cells. For fusion of murine B cells, the cell line Ag-8 is preferred.

The technique of generating monoclonal antibodies is described in many articles and textbooks, such as the above-noted Chapter 2 of Current Protocols in Immunology. Spleen or lymph node cells of these animals may be used in the same way as spleen or lymph node cells of protein-immunized animals, for the generation of monoclonal antibodies as described in Chapter 2 therein. The techniques used in generating monoclonal antibodies are further described by Kohler and Milstein, Nature 256:495-497, (1975), and in USP 4,376,110.

The term "antibody" is meant to include both intact molecules as well as fragments thereof, such as, for example, Fab and F(ab')₂, which are capable of binding antigen. Fab and F(ab')₂ fragments lack the Fc fragment of intact antibody, clear more rapidly from the circulation, and may have less nonspecific tissue binding than an intact antibody [Wahl *et al*., J. Nucl. Med. 24: 316-325, (1983)]. It will be appreciated that Fab and F(ab')₂ and other fragments of the antibodies useful in the present invention may be used for the detection and quantitation of the ligand for the complex of the invention, according to the methods disclosed herein for intact antibody molecules. Such fragments are typically produced by proteolytic cleavage, using enzymes such as papain (to produce Fab fragments) or pepsin (to produce F(ab')₂ fragments).

An antibody is said to be "capable of binding" a molecule if it is capable of specifically reacting with the molecule to thereby bind the molecule to the antibody. The term "epitope" is meant to refer to that portion of any molecule capable of being bound by an antibody that can also be recognized by that antibody. Epitopes or "antigenic determinants" usually consist of chemically active surface groupings of molecules such as amino acids or sugar side chains, and have specific three-dimensional structural characteristics as well as specific charge characteristics.

An "antigen" is a molecule or a portion of a molecule capable of being bound by an antibody, which is additionally capable of inducing an animal to produce antibody capable of binding to an epitope of that antigen. An antigen may have one or more than one epitope. The specific reaction referred to above is meant to indicate that the antigen will react, in a highly selective manner, with its corresponding antibody and not with the multitude of other antibodies which may be evoked by other antigens.

The antibodies, including fragments of antibodies, useful in the present invention, may be used to quantitatively and/or qualitatively detect the ligand for the complex of the present invention in a sample. This can be accomplished by immunofluorescence techniques employing a fluorescently or color-labeled antibody coupled with light microscopic, flow cytometric, or fluorometric detection.

Another specifically preferred embodiment relates to the antibodies of the invention conjugated to a detectable moiety. One of the ways in which an antibody in accordance with the present invention can be detectably labeled is by linking the same to an enzyme and used in an enzyme immunoassay (EIA). This enzyme, in turn, when later exposed to an appropriate substrate, will react with the substrate in such a manner as to produce a chemical moiety which can be detected, for example, by spectrophotometric, fluorometric or by visual means. Enzymes which can be used to detectably label the antibody include, but are not limited to, malate dehydrogenase, staphylococcal nuclease, delta-5-steroid isomerase, yeast alcohol dehydrogenase, α-glycerophosphate dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholin-esterase. The detection can be accomplished by colorimetric methods, which employ a chromogenic substrate for the enzyme. Detection may also be accomplished by visual comparison of the extent of enzymatic reaction of a substrate in comparison with similarly prepared standards.

Detection may be accomplished by using any of a variety of other immunoassays. For example, by radioactive labeling the antibodies or antibody fragments, it is possible to detect receptor tyrosine phosphatase (R-PTPase) through the use of a radioimmunoassay (RIA). A good description of RIA may be found in Laboratory Techniques and Biochemistry in Molecular Biology, by Work, T.S. *et al.,* North Holland Publishing Company, NY (1978) with particular reference to the chapter entitled "An Introduction to Radioimmune Assay and Related Techniques" by Chard, T., incorporated by reference herein. The radioactive isotope can be detected by such means as the use of a g counter or a scintillation counter or by autoradiography.

It is also possible to label an antibody in accordance with the present invention with a fluorescent compound, fluorescence emitting metals, a chemi- luminescent compound or a bioluminescent compound.

In a fourth aspect, the invention relates to a composition for the treatment of a pathological condition. This composition comprises as active ingredient a complex according to the invention, in which the active segment is a cytotoxic moiety and/or an Ig fragment and pharmaceutically acceptable carriers.

As used herein "pharmaceutically acceptable carrier" includes any and all solvents, dispersion media, coatings, antibacterial and antifungal agents and the like. The use of such media and agents for pharmaceutical active substances is well known in the art. Except as any conventional media or agent is incompatible with the active ingredient, its use in the therapeutic composition is contemplated.

The composition of the invention may be used in the treatment of viral infections by, for example, Influenza virus, human immunodeficiency virus, Epstein-Barr virus, cytomegalovirus, Vaccinia virus, MVM, ECMV and Herpes virus.

The composition of the invention may also be used for treating a cancer disease such as melanoma, carcinoma, sarcoma and lymphoma, preferably melanoma.

An alternative aspect of the present invention relates to a diagnostic composition for detecting the presence of abnormal cells in a sample, comprising a complex of the invention in which the active segment is a detactable imaging moiety such as a paramagnetic, radioactive or fluorogenic agent or moiety.

Another aspect of the present invention relates to a method for treating a pathological condition in a subject comprising the step of administering a pharmaceutically effective amount of a therapeutic agent to the subject, wherein said therapeutic agent comprises a complex or composition of the invention.

The pathological condition may be a viral infection caused by any one of Influenza virus, human immunodeficiency virus, Epstein-Barr virus, cytomegalovirus, Vaccinia virus, MVM, ECMV and Herpes virus.

Alternatively, the method of the invention may be used for treating a malignant disease such as melanoma, carcinoma, sarcoma and lymphoma, and preferably melanoma.

The method of the invention preferably employs a complex of the invention in which the active segment comprises an Ig fragment, particularly the Fc portion of an Ig molecule denoted by the amino acid sequence substantially as denoted by SEQ ID NO:5. Alternatively, the method of the invention employs a complex in which the active segment comprises a cytotoxic moiety. This cytotoxic moiety may be selected from cytotoxins or anticellular agents capable of killing and/or suppressing the growth and/or cell division of said target cell. More specifically, the cytotoxin or anticellular agent may be a synthetic toxin or a plant-, fungus-, or bacteria-derived toxin. For example, the toxin may be an A chain toxin, ribosome inactivating protein, α-sarcin, aspergillin, restrictocin, ribonuclease, diphtheria toxin, *Pseudomonas* exotoxin, an endotoxin or the lipid A moiety of an endotoxin.

In addition to the method of treatment, the present invention encompasses ex vivo treatment, by which cancerous cells or otherwise defective cells which express any one of NKp46, NKp44 and NKp30 on their surface are treated with the complex of the invention. In such ex vivo protocols, the biological sample may be drawn from the body of the subject, such as a human subject.

The sample may be of blood, bone marrow cells, or similar tissues or cells from an organ afflicted with a cancer. Methods for obtaining such samples are well known to the skilled workers in the fields of oncology and surgery. They include sampling blood in well-known ways, or obtaining biopsies from the bone marrow or other tissue or organ. The cancer cells (or virus-infected cells) contained in the sample may be effectively eliminated due to the cytotoxic activity of the complex of the invention. The sample may then be returned to the body of the subject from which it was obtained.

As used herein, "effective amount" means an amount necessary to achieve a selected result. For example, an effective amount of the composition of the invention useful for the treatment of said pathology.

In a preferred embodiment, the method of the invention is intended for treating a mammalian subject, preferably, a human. Therefore, by "patient" or "subject in need" is meant any mammal for which gene therapy is desired, including human bovine, equine, canine, and feline subjects, preferably, human patient.

For the *in vivo* treatment in accordance with the invention, the complex or compositions of the invention can be administered in a variety of ways. By way of non limiting example, the cells may be delivered intravenously, or into a body cavity adjacent to the location of a solid tumor, such as the intraperitoneal cavity, or injected directly into or adjacent to a solid tumor. Intravenous administration, for example, is advantageous in the treatment of leukemias, lymphomas, and comparable malignancies of the lymphatic system, as well as in the treatment of viral infections.

The pharmaceutical forms suitable for injection use include sterile aqueous solutions or dispersions and sterile powders for the extemporaneous preparation of sterile injectable solutions or dispersions. In all cases the form must be sterile and must be fluid to the extent that easy syringeability exists.

It must be stable under the conditions of manufacture and storage and must be preserved against the contaminating action of microorganisms, such as bacterium and fungi.

The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above.

In the case of sterile powders for the preparation of the sterile injectable solutions, the preferred method of preparation are vacuum-drying and freeze drying techniques which yield a powder of the active ingredient plus any additional desired ingredient from a previously sterile-filtered solution thereof.

The complex and compositions of the present invention may be administered directly to the subject to be treated or, depending on the size of the compound (cytotoxic or imaging moiety), it may be desirable to conjugate them to carrier proteins such as ovalbumin or serum albumin prior to their administration. Therapeutic formulations may be administered in any conventional dosage formulation. Formulations typically comprise at least one active ingredient, as defined above, together with one or more acceptable carriers thereof.

Each carrier should be both pharmaceutically and physiologically acceptable in the sense of being compatible with the other ingredients and not injurious to the patient. Formulations include those suitable for oral, rectal, nasal, or parenteral (including subcutaneous, intramuscular, intravenous and intradermal) administration. The formulations may conveniently be presented in unit dosage form and may be prepared by any methods well known in the art of pharmacy.

An alternative aspect of the present invention relates to a method for the diagnosis and imaging of pathologies, specifically tumors. This method comprises the steps of introducing an imaging agent into the blood stream of a subject, and detecting and quantitating the binding of the imaging agent to any one of NKp46, NKp44 or NKp30 ligands expressed on a diseased target cell. The imaging agent comprises a complex of the invention in which the active segment comprises an imaging moiety that can be, for example, a paramagnetic, radioactive or fluorogenic agent.

In a specifically preferred embodiment, the diagnostic method of the invention may be used for the diagnosis of pathological conditions such as tumors, e.g. melanomas, carcinomas, sarcomas and lymphomas.

Disclosed and described, it is to be understood that this invention is not limited to the particular examples, process steps, and materials disclosed herein as such process steps and materials may vary somewhat. It is also to be understood that the terminology used herein is used for the purpose of describing particular embodiments only and not intended to be limiting since the scope of the present invention will be limited only by the appended claims and equivalents thereof.

It must be noted that, as used in this specification and the appended claims, the singular forms "a", "an" and "the" include plural referents unless the content clearly dictates otherwise.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

### Examples

### Materials and Methods

### Cells and Viruses

*Cell lines:*
   * 721.221 - class I MHC-negative human EBV-transformed B cell line.
   * 1106mel - class I MHC-negative human melanoma cell line.
   * 293T - adenovirus-transformed, SV-large T antigen-transfected, human fibroblast kidney cell line.
   * NK cells (lines and clones) were isolated from peripheral blood lynphocyte (PBL) using the human NK cell isolation kit and the autoMACS instrument (Miltenyi Biotec Inc), NK cells were kept in culture as previously described [Mandelboim, O., *et al. J. Exp. Med.* 184:913-922 (1996)].
*Viruses:*
   Sendi virus (SV) a mouse paramyxovirus and the Influenza virus (IV) A/PR/8/34 (H1N1) were purchased from Spafas (Preston City, CT, USA).
Monoclonal antibodies
   Sendi virus specific mAb were previously described [Peterhans, E., *et al.,* Virology 128:366-376 (1983); Yewdell, J. W. *et al.,* J. Immunol. 128:2670-2675 (1982)].

Influenza virus specific mAb were previously described [Yewdell, J. W*., et al.,* J. Virol. 48:239-248 (1983)].

Anti-CD99 mAb 12E7 is a kind gift from A. Bernard (Hopital de L'Archet, Nice, France).

The anti-KIR2DL1 (NKAT1) mAb HP3E4 is a kind gift from Dr. Lopez-Botet (Hospital de la Princesa, Madrid, Spain). The hybridoma-producing mAb 3G8 was kindly given by Jay Unkeless (Mt. Sinai School of Medicine, New York, USA). The pan anti-class I mAb 147 was purchased from ExBio (Czech Republic).

The hybridoma-producing mAb 3G8 was kindly given by Jay Unkeless (Mt. Sinai School of Medicine, New York, USA).

### General methods in molecular biology:

Standard molecular biology techniques known in the art and not specifically described were generally followed as in Maniatis et al., Molecular Cloning: A Laboratory Manual, Cold Spring Harbor Laboratory, New York (1989, 1992).

### Generation of mAb that recognize 721.221 cells infected with SV

SV-infected 721.221 cells (5x10⁶) were washed and injected intraperitoneally three times into BALB/c mice at 14 day intervals. Sera were harvested from immunized mice and tested for the presence of antibodies against infected cells. Mice producing such antibodies were re-boosted, spleens harvested 4 days later, and the splenocytes fused with SP2/0 cells as previously described [Porgador, A., *et al.,* Immunity 6:715-726 (1997)].

Supernatants from wells containing growing cell fusions were first screened by ELISA for binding 721.221, SV-infected 721.221 or 1106mel cells. Supernatants positive for infected 721.221 and/or 1106mel were then screened by flow cytometry for blocking of NKp46-Ig binding to SV-infected 721.221 cells.

### Hemagglutination inhibition test

In microtitration plates 1 hemagglutination unit (HAU) of IV was incubated for 30' at 4°C in 50 µl PBS with serial two-fold dilutions of NKp46-Ig. 50 µl of 1% packed sheep RBC suspension was then added and the pattern of hemagglutination counted after 30 min at room temperature.

### Cytotoxicity assays

The cytotoxic activity of NK lines and clones against the various targets was assessed in 5-hr ³⁵S-release assays as previously described [Porgador, A., Proc Natl Acad Sci. USA 94:13140-13145 (1997)].

In experiments where mAb were included, NK cells were first incubated with 50% human serum (to prevent binding of the mAb to the various Fc receptors expressed on the surface of human NK cells) and washed. The final mAb concentration was 20 µg/ml or 1:100 dilution in cases where the mAbs are present in sera from hybridoma bearing mice. In all experiments shown, the spontaneous release was less than 25% of maximal release. Each point represents the average of duplicate values. The range of the duplicates was always within 5% of their mean.

### Ig-fusion proteins

The generation of CD16-Ig fusion protein was previously described [Mandelboim, O. *et al.,* Proc. Natl. Acad. Sci. USA 96:5640-5644 (1999)].

Sequences encoding the extracellular portions of Sequences encoding the extracellular portions of NKp30 (accession number AJ223153), NKp44 (accession number NM_004828), NKp46 (isoform used has accession number AJ006121), KIR-1 (accession number L41267) and NKAT-8 (NM_012314) were amplified by PCR from cDNA isolated from human NK clones. These PCR-generated fragments were cloned into the mammalian expression vector containing the Fc portion of human IgG1 and Ig-fusion proteins were produced as described [Mandelboim, O. *et al.*, (1999) *ibid*.]. Sequencing of the constructs confirmed that cDNA of all Ig-fusion proteins were in frame with the human Fc genomic DNA and were identical to the reported sequences.

All Ig-fusion proteins used in this work migrate as a single band on standard non-reduced SDS-PAGE gels and each was regularly assayed by SDS-PAGE to ensure the proteins had not degraded.

FACS staining procedure with Ig-fusion proteins was previously described [Mandelboim, O. *et al.*, (1999) *ibid*.].

### Production of anti-NKp46-Ig and NKp44-Ig serum

BALB/c mice were injected in their foot pads with 40 µg of NKp46-Ig, NKp44-Ig or KIR-1-Ig fusion proteins emulsified in CFA. Six weeks later mice were boosted and sera were harvested 12 days later.

For control sera, mice were immunized as above with PBS emulsified in CFA. Sera were tested for the NKp46 and NKp44 antigen specificity on YTS, KIR-1-transfected YTS cells and various NK lines and clones.

The anti-NKp44 and 46 serum was used at 1:100 dilution, a concentration at which binding was saturated as measured by flow cytometry. In agreement with the pattern of expression of NKp44 and NKp46 reported [Cantoni C, C. et al., *J*. *Exp Med.* 189: 787-796 (1999), Pessino A, S. et al., *J. Exp. Med.* 188: 953-960 (1998)], only NK cells were positively stained with the anti-NKp44 and anti-NKp46 serums and control cells 721.221, RPMI 8866, Jurkat and others, remained unstained. All NK cells (6 lines and more than 150 clones) were stained to various degrees with this serum. In agreement with the pattern of expression of NKp44 reported [Cantoni C, C. *et al. ibid* (1999)], expression of NKp44 was detected only on the surface of activated NK cells. In addition, re-directed lysis of P815 cells could be induced when the cells were coated with the anti-NKp44 and 46 serum and incubated with various NK lines and clones. The anti-NKp44 and 46 serum partially blocked the lysis of several non-infected target cells, even including lysis of non-infected 1106mel and 293T cells at high E:T ratios (greater than 20:1). The presence of cellular ligands to other NK triggering receptors [as reviewed in Bottino, C*., et al., Hum. Immunol.* 61: 1-6 (2000)] might explain the partial blocking.

### Blocking of binding of NKp46-Ig and NKp44-Ig fusion proteins to virus-infected cells

Cells (721.221 and 1106mel) were infected either with 100 µl of SV supernatant (for 721.221), or with 1000 u/ml of IV (for 1106mel). After overnight incubation, infected cells were washed and incubated on ice with the various mAbs, for 1 h. Next, cells were washed and assayed for staining with 10 µg of the appropriate Ig-fusion protein as previously described [Mandelboim, O., *et al*., (1999) *ibid*.]*.*

### Blocking of NKp44-Ig binding using purified HA.

Ten micrograms of various Ig-fusion proteins were incubated for two hrs on ice with 40 µg of purified HA protein [Brand, C. M. and Skehel, J.J. Nat. New Biol. 238:145-147 (1972)] at final volume of 100 µl PBS-BSA-Azid. These mixtures were then incubated with cells for 2 hrs on ice and stained for the presence of Ig-fusion proteins using the same staining procedures as previously described [Mandelboim, O., P. *et al., ibid* (1999)].

### Transient transfection

293T cells were either transiently transfected with a control PCDNA3 plasmid (293T/MOCK) or with a cDNA encoding for HN of the Sendai Virus (293T/pca-svhn) using the Fugene transfection reagent (Boehringer Mannheim).

### Example 1

### Detection of NKp30 NKp44, NKp46 and CD16 ligands.

The role of NKp30, NKp44 NKp46 and CD16 in NK recognition was studied by producing fusion proteins in which extracellular domains of NKp30, NKp44, NKp46 and CD16 are fused to the Fc portion of human IgG1. The extracellular domain of CD99 fused to the Fc of the human IgG1 DNA was used as control. These constructs were transiently transfected into COS-7 cells and secreted fusion proteins were purified on a protein G column. The Ig-fusion proteins were incubated with the various target cells and analyzed for binding by indirect immunostaining as previously described [Mandelboim, O.*, et al.,. ibid* (1999)]. In general, among all the Ig-fusion proteins tested, the most efficient binding was observed with the NKp44-Ig fusion protein (Table 1). The control CD99-Ig fusion protein did not bind to any of the target cells tested. Previous reports suggested that NKp46, together with the NKp44 activating receptor but not the CD16 receptor [Mandelboim, O., *et al., ibid.* (1999)], are involved in the lysis of class I negative 721.221 cells [Cantoni C, C. *et al., ibid* (1999), Biassoni R, A. *et al.,* Eur J Immunol. 29: 1014-1020 (1999), Pessino A, S. *et al.,* J. Exp. Med. 188: 953-960 (1998); Sivori S, D. *et al.,* Eur J Immunol. 29: 1656-66 (1999)].

Indeed, little staining of 721.221 cells was observed when cells were incubated either with the NKp30-Ig, NKp44-Ig or NKp46-Ig fusion proteins and no staining was observed when the 721.221 cells were incubated with the CD16-Ig fusion protein (Table 1). Similar results were obtained with another EBV transformed B cell line, RPMI 8866. These results indicate that the low binding of NKp30-Ig, NKp44-Ig and NKp46-Ig, suggesting low ligands expression for all of these proteins, is either nevertheless enough to cause lysis of these cells, or alternatively suggests the existence of another lysis ligand for other lysis receptors different from NKp44, NKp46 or CD16. Binding of NKp30-Ig, NKp44-Ig, NKp46-Ig or CD16-Ig to large T antigen transfected 293T kidney cell line, to the 1106mel-melanoma cell line and to the monkey COS-7 cell line, was observed (Table 1). Indeed, all of these target cells are sensitive, to various degrees, to NK cell mediated killing [Mandelboim, O., P. *et al., ibid.* (1999)]. The presence of ligands for NKp30 NKp44, NKp46 and CD16 on COS-7 cells suggests that the ligands for these receptors might be conserved among some primates. Little staining of NKp30 and NKp44-Ig fusion proteins to LB33MELA1 melanoma cells line was observed (Table 1); a cell line that can not be killed by NK cells (data not shown). Finally, little or no binding of either NKp30-, NKp44-, NKp46- or CD16-Ig fusion proteins was observed, either to the mouse p815 cells or to PBL derived from healthy donors (Table 1), thus indicating foremost that the ligands for the lysis receptors might be different between human and mouse and secondly that "normal" cells, derived from PBL, at least, do not express the lysis ligands for NKp30 NKp44, NKp46 and CD16.

**Table 1**

| Staining of different target cells with various Ig-fusion proteins | | | | | | |
|---|---|---|---|---|---|---|
| Cells | No protein | CD99-Ig | CD16-Ig | NKp30-I G | NKp44-I g | NKp46-1 g |
| 721.221 | 3 | 4 | 3 | 11 | 9 | 11 |
| RPMI 8866 | 3 | 4 | 4 | 10 | 9 | 9 |
| 293T | 3 | 4 | 12 | 57 | 125 | 13 |
| 1106mel | 3 | 4 | 9 | 60 | 99 | 11 |
| LB33ME LA1 | 3 | 4 | 4 | 8 | 7 | 4 |
| COS | 6 | 4 | 16 | 91 | 100 | 14 |
| P815 | 2 | 2 | 3 | 5.2 | 3 | 4 |
| PBL | 2 | 2 | 2 | 57 | 2 | 2 |
| Cells were incubated with various Ig-fusion proteins as described in the experimental procedures and stained with PE-conjugated goat anti-human Fe. MFI indicates Median Fluorescence Intensity; MFI numbers were rounded to the nearest whole numbers. Results are representative of two independent experiments. | | | | | | |

### Example 2

### NKp46-Ig fusion protein binds to virus infected cells

As previously published [Trinchieri *et al.,* Adv. in Immunol. 47:187-376 (1989)], NK cells can effectively lyse virus-infected cells, therefore the question whether infection with Sendai virus (SV) (a mouse paramyxovirus) increased the binding of NKp46-Ig was next tested. Remarkably, a 10-fold increase in the staining by NKp46-Ig was observed (Table 2). This effect is specific for NKp46 since SV infection did not alter the binding of other NK receptor Ig-fusion proteins tested (CD16-Ig, KIR-1-Ig, or NKAT-8-Ig - data not shown).

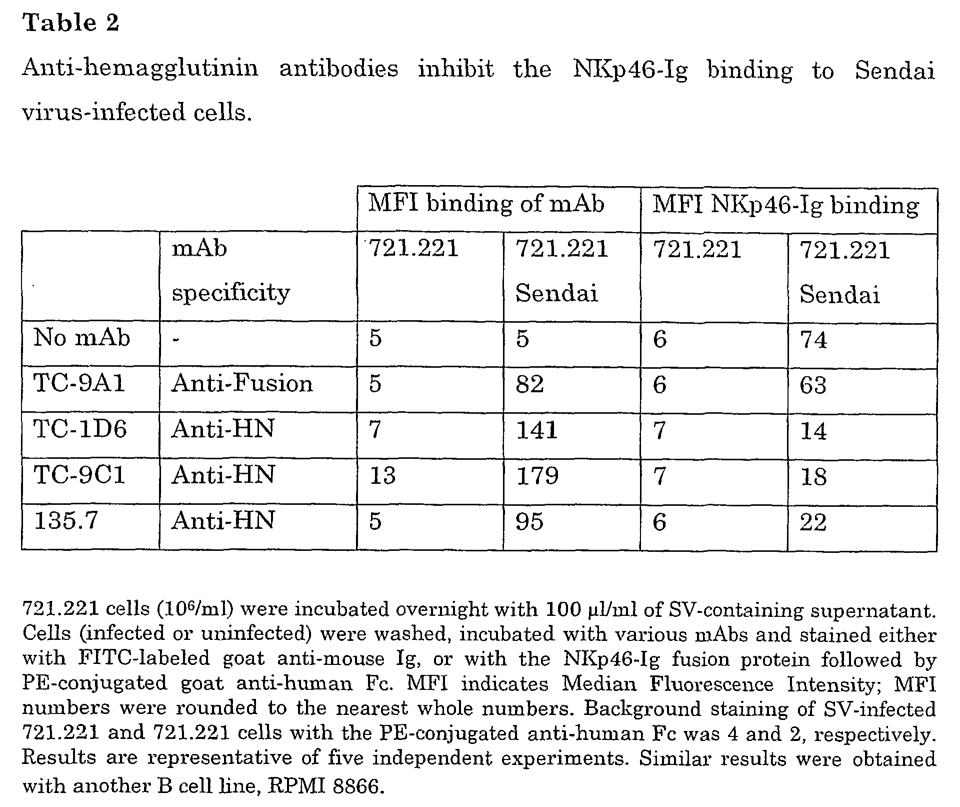

### Infection with different viruses

To find out whether the observed binding of the NKp46-Ig fusion protein to SV infected 721.221 cells was virus or cell specific, similar infection experiments were performed using the A9 fibroblasts infected with different viruses. As shown in Fig. 1, significant increase in the NKp46-Ig binding was observed when uninfected A9 cells were compared to the same cells infected with EMCV, MVM, Adeno virus or Vaccinia virus.

### Example 3

### Binding of NKp46-Ig to the SV HN glycoprotein

To identify the putative NKp46-ligand on SV-infected 721.221 cells, mice were immunized with the SV-infected 721.221 cells, and spleen derived B cell hybridoma supernatants were screened for increased staining of virus-infected cells relative to non-infected cells. The supernatants of one of the hybridoma clones tested (135.7) efficiently blocked the binding of NKp46-Ig to SV infected cells (Table 2). Therefore, mAb 135.7 may recognize either proteins encoded by SV or host cells.

ELISA assays using SV as immunoadsorbent indicated that mAb 135.7 recognizes a viral gene product (data not shown).

SDS-PAGE analysis of 135.7-reactive proteins recovered from detergent lysates of ¹²⁵I-cell surface labeled SV-infected 721.221 cells, revealed that 135.7 binds a protein with an apparent M.W. of 70 kDa, similar to the reported mobility of the HN glycoprotein.

Therefore the question whether well characterized anti-SV mAb also block the binding of NKp46-Ig to SV-infected cells, was next tested. Indeed, as shown in Table 2, NKp46-Ig binding was blocked when infected cells were first incubated with anti-HN mAb TC-1DG or TC-9C1 but not with mAb TC-9A1 specific for the other major SV glycoprotein, the fusion (F) protein.

### Example 4

### The role of NKp46 recognition of HN in lysis of HN transfected 293Tcells

Despite the HN-dependent binding of NKp46-Ig to SV infected 721.221 cells, detection of an increased susceptibility of these cells to NK mediated lysis associated with SV infection was failed (data not shown), probably because these cells are already very sensitive to NK-mediated lysis. This prevented the direct testing of the role of NKp46 recognition of HN in NK lysis.

However, this was possible using 293T cells that were transiently transfected with a plasmid encoding HN. Forty-eight h after transfection, cell surface HN expression was confirmed using the TC-1D6 mAb (Fig. 2A) or 135.7 (not shown).

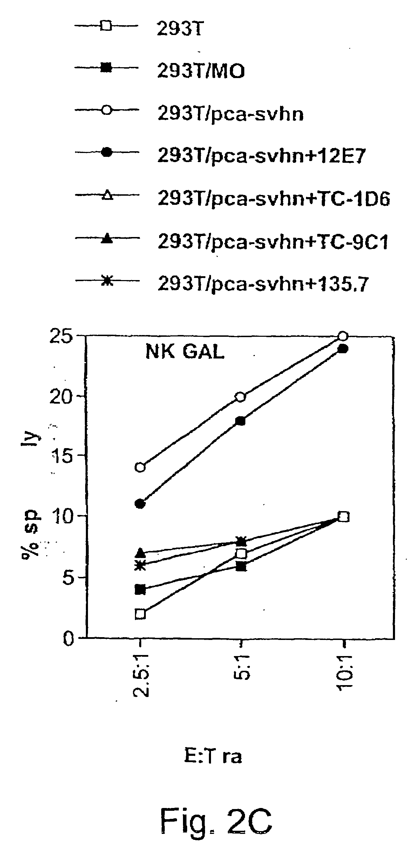

Importantly, as shown in Figure 2A, transfection resulted in a two-fold increase in NKp46-Ig staining, without enhancing the staining with other Ig-fusion proteins - KIR-1-Ig, NKAT-8-Ig or CD16-Ig. Moreover, NK-GAL, a NK line derived from healthy donor PBL, lysed HN-transfected 293T cells at least 4-fold more efficiently than non-transfected or mock-transfected cells (Fig. 2B,C).

Pre-incubation of NK GAL with a mouse antiserum raised against NKp46-Ig, resulted in inhibition of the increased killing of HN-transfected 293T cells, while incubation with a control serum had little effect (Fig. 2B). The same experiment revealed that each of three HN-specific mAb could block NK mediated lysis, while a control mAb specific for CD99 had no significant effect (Fig. 2C).

### Example 5

### NKp46 is required for the recognition of HA- expressing cells by NK cells

One of the hallmarks of NK recognition is its lack of antigen specificity. Having shown that NKp46 interacts with the SV HN both physically and functionally in NK mediated lysis, the inventors next tested whether it could interact with the Influenza virus (IV) HA. IV-infection of 1106mel cells (a class I deficient cell line) resulted in a four-fold increase in NKp46-Ig binding (Table 3). As above, the specific nature of the enhanced binding is shown by the constant binding of other Ig-fusion proteins (data not shown).

Importantly, the increased NKp46-Ig binding was completely, or partially blocked, respectively, by the HA specific mAb H28-E23 and H17-L2 (Table 3). In contrast, HN specific mAb TC-1D6, TC-9C1 or 135.7 had no effect on binding (data not shown).

Next, 1106mel cells were infected with IV. As shown in Figure 3A, this infection enhances NK GAL mediated lysis, and the enhanced killing is blocked by pre-incubation of cells with anti-NKp46 serum but not with control serum or mAb 12E7 and 3G8 specific for CD99 and CD16 respectively.

Incubation of IV-infected 1106mel cells with mAb H28-E23 resulted in complete inhibition of the increased lysis, whereas H17-L2 had a partial inhibitory effect, mirroring the results of the blocking experiment (Fig. 3B, Table 3).

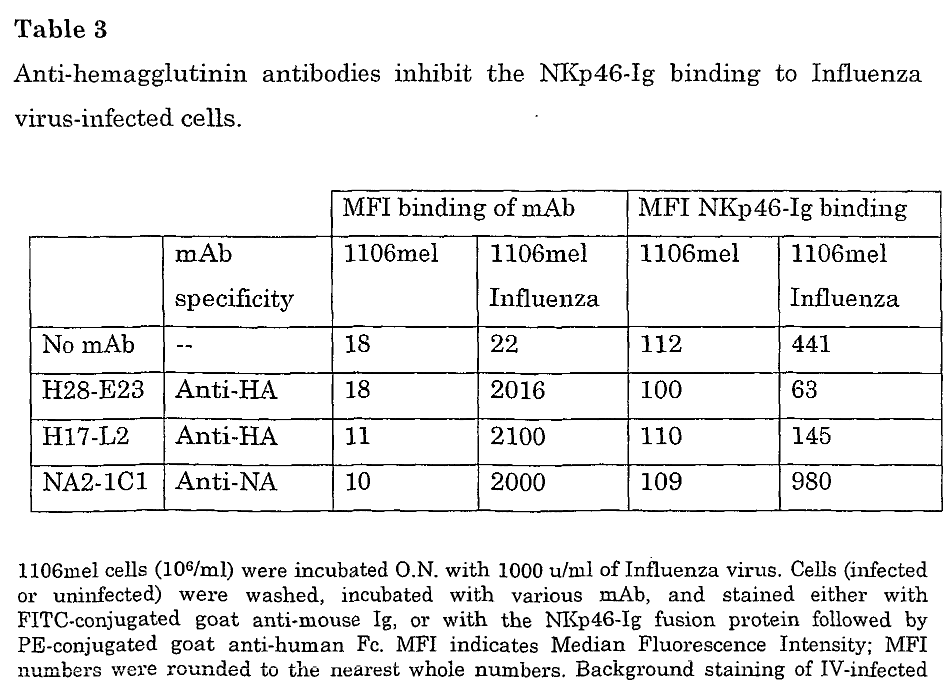

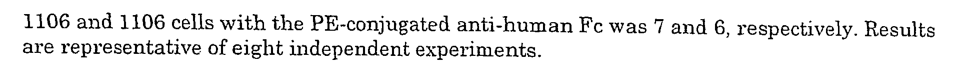

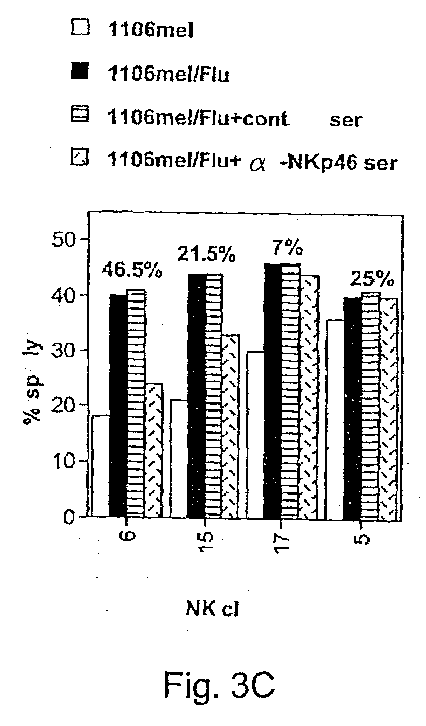

The recognition of IV-infected 1106mel cells by clones prepared from NK GAL was examined by limiting dilution. All 28 clones tested were positively stained with the anti-NKp46 serum. Twenty-one of the clones exhibited enhanced recognition of IV-infected cells relative to uninfected cells (Fig. 3C); pre-incubation of all 21 clones with the HA-specific mAb H28-E23 completely inhibited the IV-enhanced lysis (data not shown). Pre-incubation of NK clones with anti-NKp46 serum completely inhibited the IV-enhanced lysis of 13 of these clones, (e.g. clone 6, Fig. 3C), while 6 of the clones were partially inhibited (e.g. clone 15, Fig. 3C). The average MFI staining with anti-NKp46 serum for these two groups was 28.9 and 32.8 respectively. Two clones demonstrating enhanced lysis of IV-infected cells were not affected by the NKp46 antiserum (e.g. clone 17, Fig. 3C). The average MFI for anti-NKp46 staining for this group of two clones was 17.5.

Finally, no IV-associated enhancement in lysis was observed in 7 of the clones tested, (e.g. clone 5, Figure 3C) and the MFI for anti-NKp46 staining of this group was 31.3. Similar results were obtained when the same NK clones were tested with HN transfected 293T cells (data not shown).

NK clones were also generated from other NK lines that express lower amount of the NKp46 receptor (for example, an NK line with MFI of NKp46 staining of 15.5 as compared to MFI of 41.4 in NK GAL). Inhibition (either partial or complete) of the enhancement of IV-infected 1106mel lysis was observed in about third of the clones generated from this NK line, and the extent of lysis correlated with NKp46 staining (data not shown).

These findings indicate first, that NKp46 is required for the recognition of HA- and HN-expressing cells by a substantial subset of NK cells, and second, that other populations of NK cells can lyse these cells in an NKp46 independent manner.

### Example 6

### Involvement of sialic acid in the interaction of NKp46 with HA and HN

SV HN and IV HA both recognize terminal N-acetyl neuraminic acid residues (sialic acids) attached to Gal, suggesting a common mechanism for binding to NKp46. The involvement of sialic acid in the interaction of NKp46 with HA is indicated by a number of findings. First, NKp46-Ig is able to completely block IV-mediated agglutination of sheep erythrocytes at a protein concentration as low as 2 µM. Second, pre-incubation of IV-infected cells with a mAb (NA2-1C21) that blocks the enzymatic activity of IV neuraminidase (the other major IV glycoprotein expressed on the surface of infected cells and virions) significantly enhances NKp46-Ig binding to IV infected cells (Table 3). Finally, and most directly, treatment of NKp46-Ig with bacterial neuraminidase reduced its binding to IV-infected cells without reducing its binding to uninfected cells (Fig. 4).

Inasmuch as desialylation often increases interactions by reducing negative charge repulsion of receptor-ligand pairs [Varki, A., *et al.,* FASEB J. 11:248-55 (1997)], this strongly supports the direct interaction of NKp46 with the sialic binding site of HA.

These findings can be interpreted to indicate that NKp46 binds to target cells via two types of ligands: the first based on interaction of NKp46-associated sialic acid with viral sialic acid receptors, the second on a sialic acid-independent interaction with undefined cellular ligands. The former is clearly responsible for the enhanced killing of IV-infected cells by the NK cells that were studied. The contribution of the second interaction to NK activation remains to be established, and probably varies depending on the nature of the ligands expressed by the target cells and other factors as well. Whether the interaction of NKp46 with viral HA is sufficient for triggering or also requires interaction with other cellular ligands remains an important question for future studies.

The existence of NK clones that recognize IV-infected cells in a NKp46 independent manner (e.g. NK GAL clone 17, Fig. 3C) suggests the existence of other lysis receptors involved in the recognition of virus infected cells. These receptors also probably recognize HA, since the enhanced lysis associated with virus infection is completely blocked by anti-HA mAb. It is possible that the triggering of these receptors is also based on the interaction of the activating receptor with sialic acid. Given that members of at least 7 virus families utilize sialic acid as a receptor for virus entry into host cells, this suggests a general strategy for NK cell recognition of a substantial subset of viruses.

### Example 7

### Up-regulation of NKp44-Ig binding to SV-infected 721.221 cells is blocked by anti-HN mAb.

As was described herein before, the viral HA was identified as a ligand for NKp46 receptor. To test whether the NKp44 receptor can also bind to viral HA, 721.221 cells were infected with SV and tested for increased binding of NKp44-Ig. A 10-fold increase in the staining by NKp44-Ig was observed (Table 4). This effect is specific for NKp44-Ig and NKp46 (see table 2 as well), since SV infection did not alter the binding of other NK receptor Ig-fusion proteins tested (NKp30-Ig, CD16-Ig, KIR-1-Ig, or NKAT-8-Ig (data not shown). The NKp44-Ig binding was partially blocked by mAb directed against SV-HN, TC-1D6, TC-9C1 [Peterhans, E., et al., *Virology.* 128: 366-376 (1983)] or 135.7, but not with mAb TC-9A1 [Peterhans, E., *et al., ibid.* (1983)] specific for the other major SV glycoprotein, the fusion (F) protein (Table 4). This suggests that NKp44-Ig can interact with HN from SV.

### Elevation of NKp44-Ig binding to 293T cells transfected with the SV-HN cDNA

To further demonstrate direct binding of NKp44-Ig to Sendai HN, 293T cells were transiently transfected with an expression plasmid encoding Sendai HN cDNA (pca-svhn, a kind gift from Dr. Allen Portner). Forty-eight hrs after transfection, efficient HN staining was observed when using the anti-HN mAb (TC-1D6, Figure 5). Similar levels of expression were also observed when stained with 135.7 mAb, but not with control mAb (anti-HA of Influenza, data not shown). Importantly, a two-fold increase in NKp44-Ig staining of the pca-svh transfected 293T cells was also observed (Figure 5), confirming the specificity of the increase to be a ligand for NKp44. No change in the staining of all tested cells was observed using other Ig-fusion proteins (KIR-1-Ig, NKAT-8-Ig or CD16).

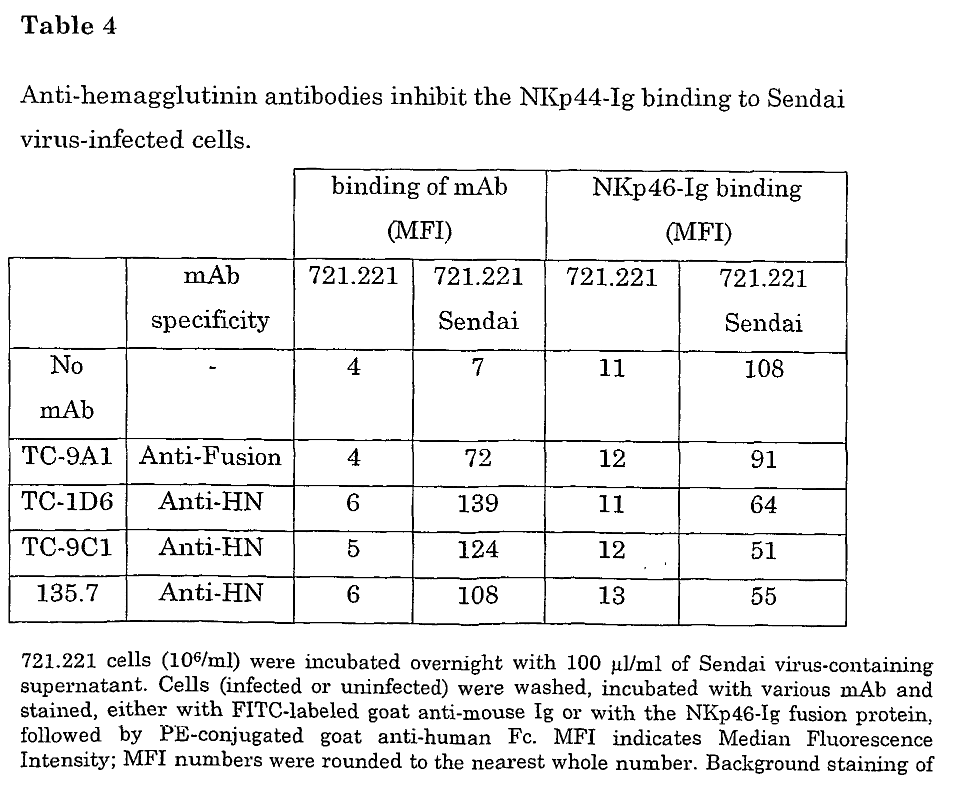

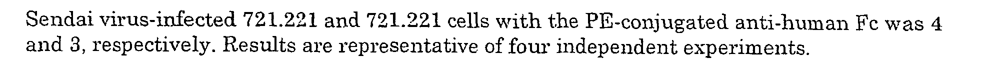

### Example 8

### SV infection of 721.221/Cw6 cells resulted in the abrogation of the inhibition mediated by NK clones expressing high levels of NKp44 proteins

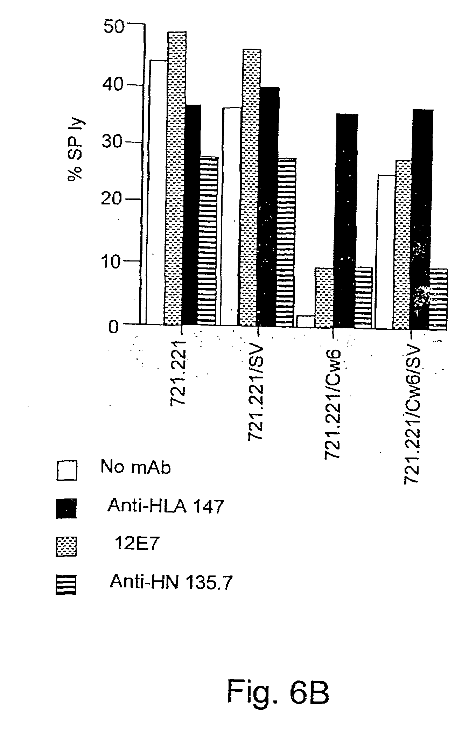

The possibility that infection of cells with SV would enhance NK-mediated lysis was next investigated. No change in the NK mediated killing was observed between the SV-infected 721.221 cells and non-infected cells (data not shown). Several explanations may account for this phenomena. Among these is the observation that NK cells efficiently lyse 721.221 cells, and it is therefore possible that the addition of another lysis ligand (SV hemagglutinin) will not result in increased killing. Therefore, the inventors next tested whether any change in the killing pattern of NK clones will be observed when 721.221 cells expressing class I MHC proteins will be infected with SV. NK clones were prepared from various donors and were first screened for inhibition of lysis mediated by 721.221 cells transfected either with HLA-Cw3, -Cw4, -Cw6 or -Cw7 class I MHC proteins. The generation of these transfectants was described by O. Mandelboim [Mandelboim, O., et al., *J. Exp. Med.* 184: 913-922 (1996)]. NK clones that were found to be inhibited by 721.221 cells expressing class I MHC proteins were next tested against the same target cells infected with SV. SV infection of 721.221 cells resulted in the abrogation of the inhibition and consequently lysis by about 75% of the NK clones tested. All of these clones expressed high levels of both NKp44 and NKp46 proteins (data not shown). One representative clone is seen in Fig. 6. NK clone 66 is inhibited by 721.221 cells expressing Cw6 (721.221/Cw6). Abolishment of the inhibition was observed when NK clone 66 was incubated with the anti-KIR2DL1 mAb HP3E4 but not when the cells were incubated with the control mAb 12E7 (Fig. 6A). Reversal of the inhibition was also observed when 721.221/Cw6 cells were infected with SV. The inhibition was made possible due to the interaction of the KIR2DL1 receptor with HLA-Cw6 as blocking of the inhibition was observed when721.221/Cw6 cells were incubated with the pan anti-class I mAb 147 (Fig. 6B). The reversal of the inhibition was dependent on the expression of HA on the infected cells as inhibition was restored when the infected cells were incubated with the anti-HA mAb 135.7 (Fig. 6B). Similar results were obtained when NK clone 66 was incubated with 721.221/Cw4 cells or with other NK clones expressing the KIR2DL2 receptor inhibited by 721.221/Cw3 or 721.221/Cw7 (data not shown). About 25% of the NK clones tested that expresses theNKp44 protein at low levels showed no change in the inhibition pattern when target cells were infected with SV. Thus, the interaction of NKp44 with the HA of SV is probably needed to overcome the inhibition mediated by SV-infected target cells expressing class I MHC protein.

### Example 9

### Purified HA Blocks the binding of the fusion proteins NKp46-Ig binding and NKp44-Ig to virus infected cells

The involvement of both NKp44 and NKp46 in the killing of the infected 721.221 cells (wild type and transfectants) can no be directly tested in this system using the anti-NKp44 and NKp46 serums as the lysis of 721.221 cells is NKp44 and NKp46 dependent [Cantoni C, C. *et al., ibid* (1999), Biassoni R, A. *et al., ibid* (1999), Pessino A, S. *et al., ibid* (1998), Sivori S, D. *et al., ibid* (1999)]. Therefore, the possibility that the NKp44-Ig fusion protein can bind the hemagglutinin of other viruses using different cell types was next tested. 1106mel cells (a cell line which is only moderately lysed by CD16 positive human NK cells [Mandelboim, O.*, et al., ibid* (1999)] were infected with IV and stained for elevated NKp44-Ig staining. Increased NKp44-Ig staining (about four fold) was observed when the 1106mel cells were infected with IV (Table 5). This NKp44-Ig staining was specific, as no increase in the binding of other Ig-fusion proteins, including NKp30 CD16-Ig, KIR-1-Ig and NKAT-8-Ig, to the IV-infected 1106mel cells was observed (data not shown). Importantly, the increased NKp44-Ig binding was completely blocked by H28-E23 mAb and H17-L2 mAb (Table 5). Both mAb are directed against the HA of Influenza virus. The addition of mAb directed against the HN from SV (TC-1D6, TC-9C1 and 135.7) had no effect (data not shown).

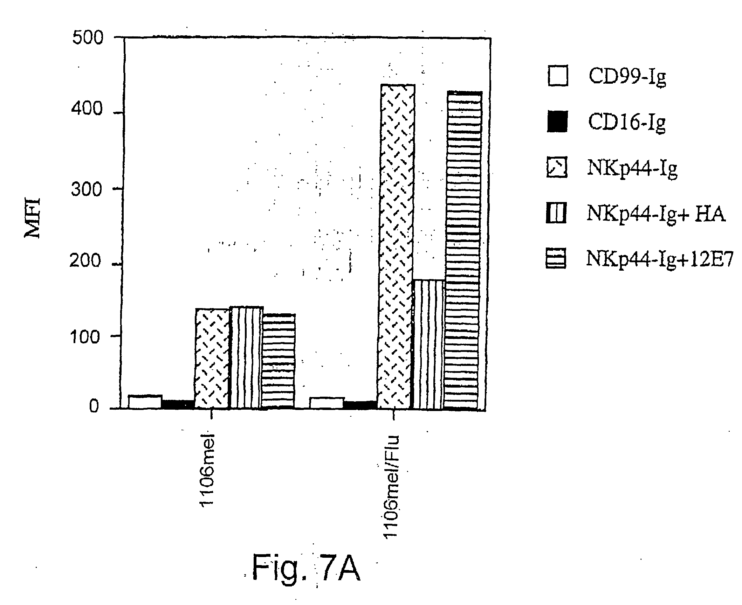

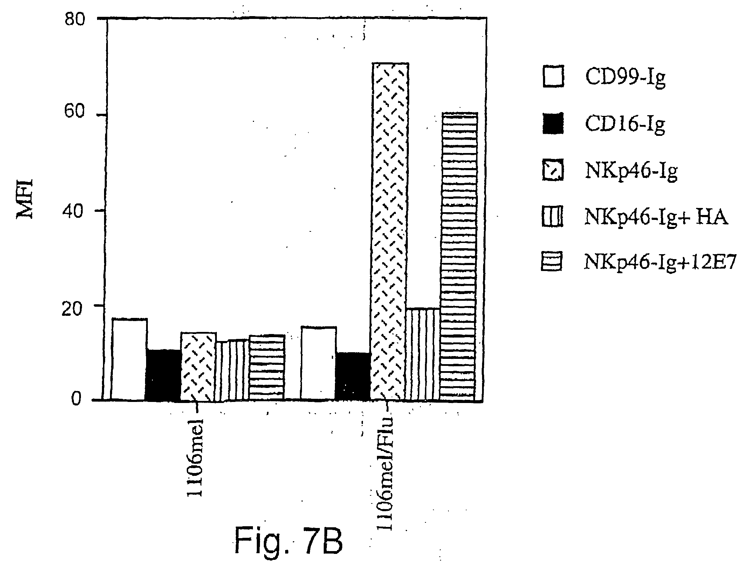

To find out whether purified HA can block the binding of NKp44 or NKp46 to infected cells, ten micrograms of various Ig-fusion proteins were incubated with 40 µg of purified HA protein at final volume of 100 µl in PBS-BSA-Azid, for two hrs on ice as described elsewhere [Brand, C. M *et al.* Nat. New Biol. 238:145-147 (1972)]. Fig. 7 shows incubation of NKp44-Ig (A) or NKp46-Ig (B) with or without 40 µg purified HA protein (no blocking of NKp46-Ig or NKp44-Ig binding was evident when less than 40 µg of purified HA protein were used). The mixtures were next incubated for 2 hrs on ice with IV-infected or non-infected 11061mel cells and stained with PE-conjugated goat anti-human Fc for the presence of Ig-fusion proteins using the same staining procedures as previously described [Mandelboim, O. *et al. ibid* (1999)]. Fig. 7 shows one representative experiment out of two performed. Similar results were obtained when SV-infected 721.221 cells were used. These results indicate direct interaction of the viral protein HA and the natural killer cells activating receptors NKp44 and NKp46.

Moreover, direct interactions between the NKp46-Ig and NKp44-Ig and the hamagglutinin protein in an ELISA assay (not shown).

### Binding of NKp44-Ig to cells infected with Influenza virus is dependent on the sialylation of NKp44

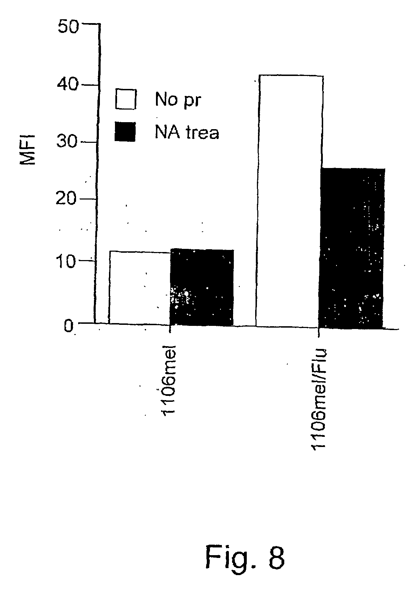

SV HN and IV HA both recognize terminal N-acetyl neuraminic acid residues (sialic acids) attached to Gal, suggesting that binding to NKp44 might occur via the sialic acid residues expressed on NKp44 similarly to the binding of HA to NKp46 (described in Example 6). Indeed, pre-incubation of IV-infected cells with a mAb (NA2-1C21) that blocks the enzymatic activity of IV neuraminidase (the other major IV glycoprotein expressed on the surface of infected cells) significantly enhances NKp44-Ig binding to IV infected cells (Table 5). In addition, NKp44-Ig did not stain target cells infected with measles (data not shown) whose HA does not bind sialic acid [Maisner, A. and Herrler, G. *Virology* 210: 479-481 (1995)]. Finally, and most directly, treatment of NKp44-Ig with bacterial neuraminidase reduced its binding to IV-infected cells without reducing its binding to uninfected cells (Fig. 8). Treatment of NKp44-Ig did not affect staining of non-infected 1106mel cells, measured by flow cytometry (Fig. 8), nor altered the integrity of the protein tested by SDS/PAGE analysis (data not shown). As desialylation often increases interactions by reducing negative charge repulsion of receptor-ligand pairs [Varki, A., FASEB J. 11: 248-55 (1997)], this strongly supports the direct interaction of NKp44 with the sialic binding site of HA.

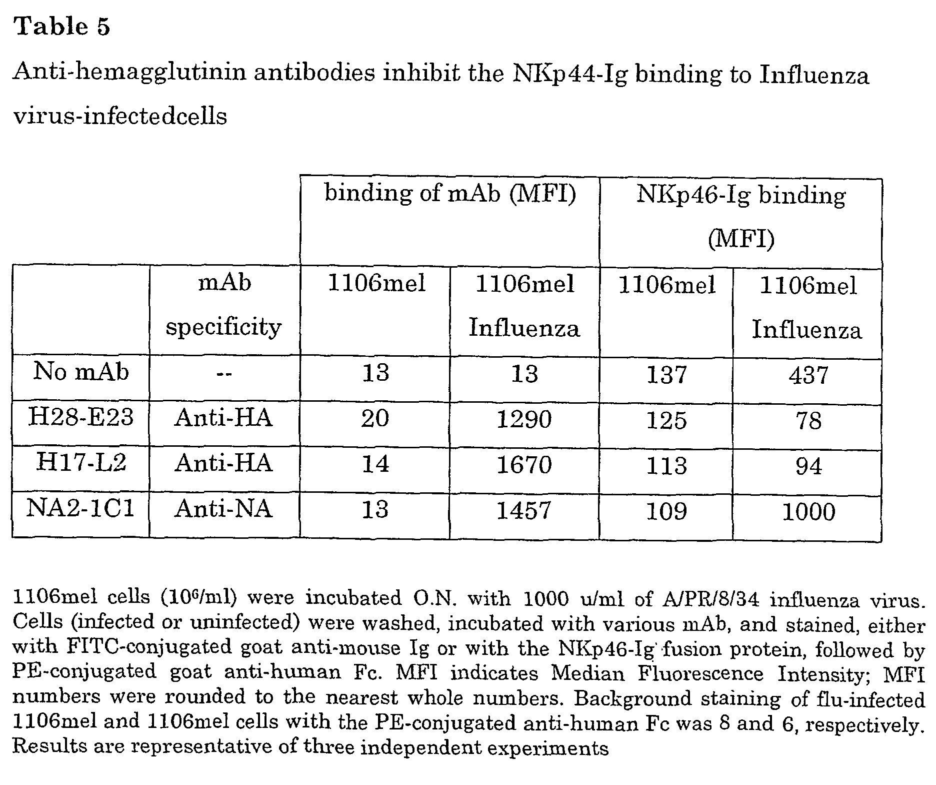

### Example 10

### The enhancement of lysis of IV-infected 1106mel cells is blocked by polyclonal mAb to NKp44

The above results suggested that NKp44 can bind the hemagglutinin of both IV and SV. As demonstrated in Example 5, IV infection of 1106mel cells resulted in enhancement of lysis of 1106mel cells that were completely blocked by mAb to HA. However, when anti-NKp46 serum was included in the assays, several killing phenotypes (complete inhibition, partial inhibition or no inhibition) could be observed among the NK clones tested. One possible explanation was the existence of another receptor able to bind HA. The lysis of IV-infected 1106mel was therefore assayed using NK clones and combinations of anti-NKp44 and anti-NKp46 serums. NK clones were prepared from PBL derived from donor MB by limiting dilution. All clones tested (64 in total) were positively stained with both anti-NKp44 and anti-NKp46 serum. Increased killing of the TV-infected1106me1 cells (1106mel/Flu) was observed in 57% of the NK clones tested (Fig. 9). Complete inhibition of the enhancement in 1106mel/Flu lysis was observed in 26% of NK clones tested that were either pre-incubated with anti-NKp46 serum or with the combination of both anti-NKp44 and NKp46 serums (e.g., clone 1, Fig. 9). 14% of the clones tested showed partial inhibition when both anti NKp44 and NKp46 serums were used independently and complete inhibition when both anti-NKp44 and anti-NKp46 serums were combined (e.g., clone 46, Fig. 9). The enhancement in 1106mel/Flu lysis could not be blocked in 17% of the clones tested (for example, clone 8, Fig. 9). Finally, no enhancement in lysis was observed in 43% of the clones tested obtained from this donor, (e.g., clone 4, Fig. 9). The percentage of such cells can vary considerably among different donors, as was showed herein before. Complete inhibition of the enhancement of 1106me1/Flu cells was never achieved when only the anti-NKp44 serum was included in the assay (data not shown). When efficient lysis of 1106mel was observed at higher E:T ratio, incubation of the cells either with anti-CD16 mAb or with anti-NKp44 and NKp46 serum resulted in partial inhibition of lysis (data not shown). When all antibodies were combined, efficient inhibition of 1106mel lysis was observed (data not shown).

Thus, similarly to the NKp46 receptor, NKp44 can bind to the hemagglutinin of both Sendai and Influenza viruses and this binding, results in triggering of NK cell-lysis of the infected cells. The reason for why 43% of the clones tested here showed no increased killing of 1106mel/Flu cells is not completely understood. One possible explanation is that these clones might express receptors to other proteins that are either up- or down-regulated due to the infection and are important in regulating NK killing.

### Example 11

### Identification of Domain 2 of the NKp46 as responsible for interacting with the viral HA

The role of different domains of the NKp46 molecule in the interaction with the viral HA was studied by producing different deleted fusion proteins.

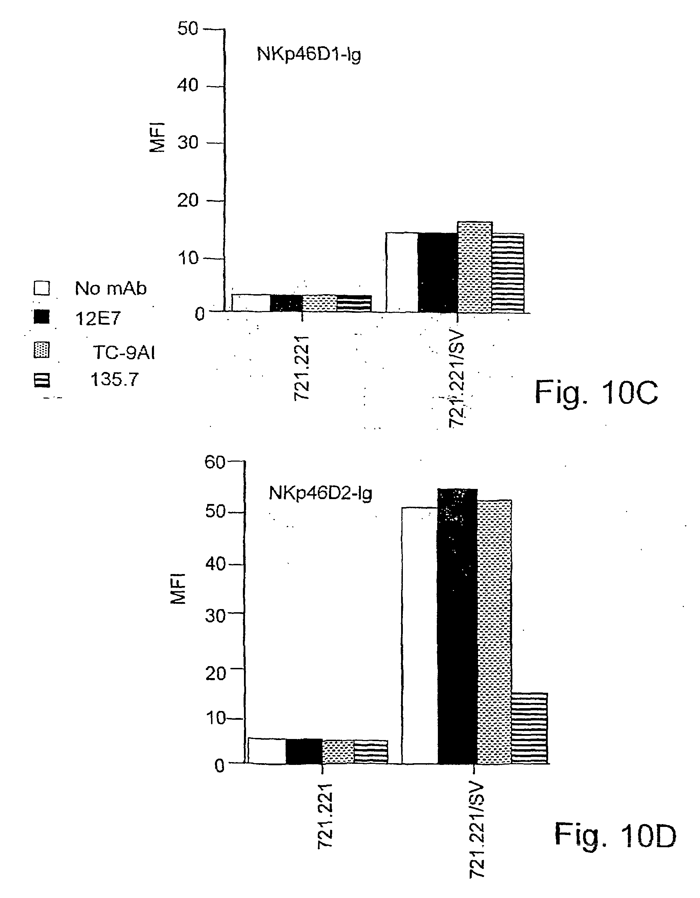

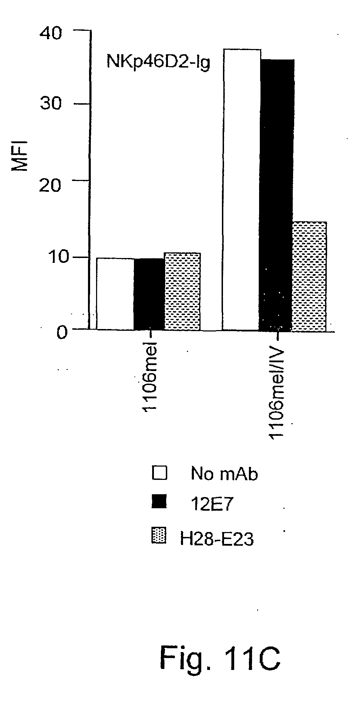

The first NKp46 extracellular domain (from a.a. #1-120- as denoted by SEQ ID NO: 26) as well as the second extracellular domain (from a.a. #121-234 - as denoted by SEQ ID NO: 22), were fused to the Fc portion of human IgG1, creating the NKp46D1-Ig and the NKp46D2-Ig, respectively. These constructs were transiently transfected into COS-7 cells and secreted fusion proteins were purified on a protein G column. To test whether binding to the viral HA is mediated particularly by one of those two domains, both deletion fusion proteins as well as the control full fusion protein NKp46-Ig and the NKp44-Ig were examined for binding to virus infected cells. Therefore 721.221 cells were infected with SV and tested for increased binding of NKp46D1-Ig or NKp46D2-Ig. About 10-fold increase in the staining by NKp46D2-Ig fusion protein was observed (Fig. 10), whereas SV infection of 721.221 cells did not enhance any significant binding of the NKp46D1-Ig fusion protein. This observed NKp46D2-Ig binding was significantly blocked by the 135.7 mAb directed against SV-HN, but not with mAb TC-9A1 [Peterhans, E., et al., *ibid* (1983)] specific for the other major SV glycoprotein, the fusion (F) protein or by the control 12E7 antobody (Fig. 10D). These results indicate that the HA interacting portion in NKp46 is the Domain 2. Similar experiments were performed in 1106mel infected with Influenza virus showed that domain 2 of the NKp46 is the domain responsible for the observed interaction with the viral HA (Fig. 11). Thus, NKp46D2 but not NKp46D1 can bind to viral-infected cells and furthermore, this binding can be blocked with anti-HN mAb.

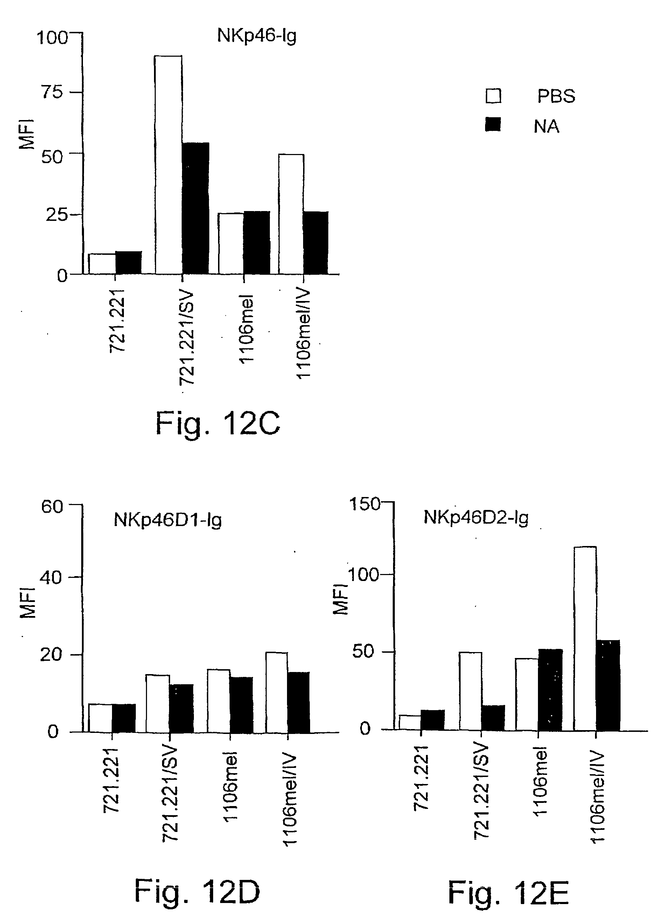

As described in Examples 6 and 9 herein, binding of SV HN and IV HA to NKp46 and NKp44, respectively, occurs via the sialic acid residues expressed on both receptors. In order to find out whether the observed binding of NKp46D2 to the viral proteins is also mediated by sialic residues, the fusion proteins were treated with bacterial neuraminidase prior to their incubation with the infected cells (SV infected 721.221 and IV infected 1106 cells). As shown in Fig. 12, removal of the sialic acid residues using neuroaminidase (NA), significantly reduced the binding capacity of the fusions proteins. These results indicate that the binding of NKp46D2-Ig to the IV-infected 1106mel cells or to the SV-infected 721.221 cells is sialic acid dependent.

### Example 12

### Expression of various lysis ligands, and particularly the NKp30 on human melanoma cells

It has been previously reported that treatment of melanoma patients with Tumor Infiltrated Lymphocytes (TIL) resulted in the emergence of class I MHC loss variants in 40% of the melanoma patients [Restifo, N.P., *et al.,* J. Natl. Cancer Inst. 88:100-108 (1996)]. This is due to downregulation of the β2-microglobulin expression in the tumor cells. The same tumor lines were later shown to be sensitive to various degrees for NK cell mediated killing [Porgador, A., *et al*., Proc Natl Acad Sci. 94:13140-13145 (1997)]. In addition, it was also shown that one of these melanoma lines, the 1106mel, was inefficiently killed by many of the CD16-negative NK clones tested [Mandelboim, O., *et al*., Proc. Natl. Acad. Sci. 96:5640-5644 (1999)].

The role of NKp30, NKp44, NKp46 and CD16 receptors in NK recognition of various melanoma cells deficient in class I MHC expression (except from LB33melA1, used as control) was studied by performing experiments using the NKp30 NKp44, NKp46 and CD16 Ig fusion proteins of the invention. cDNA encoding the extracellular domains of CD99 fused to the human IgG1 DNA was used as control. The Ig-fusion proteins were incubated with the various melanoma cells and analyzed for binding by indirect immunostaining as previously described [Mandelboim, O.*, ibid.* (1999)]. In general, the highest staining of the melanoma cells was observed with the NKp30-Ig and NKp44-Ig fusion proteins (Table 6). Little staining of all Ig-fusion proteins was observed with LB33melA1 cells, a cell line that is hardly killed by NK cells (data not shown). All other cell lines that can be killed by NK cells were stained to various degrees with the Ig-fusion proteins (Table 6).

**Table 6**

| Binding of different fusion proteins to melanoma cell lines | | | | | |
|---|---|---|---|---|---|
| Melanoma cell lines | CD99Ig MFI | CD16Ig MFI | NKp30Ig MFI | NKp44Ig MFI | NKp46Ig MFI |
| L33melA1 | 0.0 | 0.20 | 2.71 | 3.28 | 0.52 |
| L33melB1 | 0.0 | 1.14 | 8.76 | 6.41 | 1:45 |
| 1106me1 | 0.0 | 3.70 | 39.1 | 15.22 | 3.05 |
| FO-1 | 0.0 | 2.44 | 12.32 | 13.47 | 2.26 |
| 1259mel | 0.0 | 1.24 | 13.81 | 11.63 | 6.01 |
| 1047mel | 0.0 | 1.16 | 12.42 | 24.35 | 2.44 |
| 1612mH | 0.0 | 0.0 | 4.49 | 20.15 | 9.75 |
| 1612mel | 0.0 | 0.1 | 2.53 | 15.28 | 2.71 |

**Table 7**

| Sequence listing | | |
|---|---|---|
| SEQ ID NO | Description | amino acids / nucleotides |
| 1 | NKp46 cDNA (isoform a) | nucleotides |
| 2 | Fc Portion of IgG - cDNA | nucleotides |
| 3 | NKp46- Ig (isoform a) fusion protein - cDNA | nucleotides |
| 4 | NKp46 (isoform a) | amino acids |
| 5 | Fc Portion of IgG | amino acids |
| 6 | NKp46 -Ig (isoform a) fusion protein | amino acids |
| 7 | NKp44 cDNA | nucleotides |
| 8 | NKp44 -Ig fusion protein cDNA | nucleotides |
| 9 | NKp44 | amino acids |
| 10 | NKp44 -Ig fusion protein | amino acids |
| 11 | NKp46 cDNA (isoform b) | nucleotides |
| 12 | NKp46 -Ig (isoform b) fusion protein - cDNA | nucleotides |
| 13 | NKp46 (isoform b) | amino acids |
| 14 | NKp46 -Ig (isoform b) fusion protein | amino acids |
| 15 | NKp30 cDNA | nucleotides |
| 16 | NKp30 -Ig fusion protein - cDNA | nucleotides |
| 17 | NKp30 | amino acids |
| 18 | NKp30 -Ig fusion protein | amino acids |
| 19 | NKp46D2 (isoform a) | nucleotides |
| 20 | NKp46D2 (isoform b) | nucleotides |
| 21 | NKp44D2 | nucleotides |
| 22 | NKp46D2 (isoform a) | amino acids |
| 23 | NKp46D2 (isoform b) | amino acids |
| 24 | NKp44D2 | amino acids |
| 25 | NKp46DI (isoform a) | nucleotides |
| 26 | NKp46D1 (isoform a) | amino acids |

## Claims

1. A targeting complex capable of targeting specifically an active substance to abnormal target cell types selected from the group consisting of cells derived from malignant tumors, cells derived from solid tumors, cells derived from non-solid turmors, a defective or diseased cell, and a virus-infected cell, said complex comprising:
(a) a target recognition segment comprising a receptor specific to NK cells, the receptor activating lysis of the target, said receptor selected from the group consisting of NKp46, NKp44, NKp30 or a functional fragment thereof; and
(b) an active segment comprising the active substance, said active substance being selected from the group consisting of: a cytotoxic moiety; an imaging moiety; and an lg fragment

2. The targeting complex according to claim 1, wherein the target recognition segment comprises NKp46 or a functional fragment thereof.

3. The complex of claim 2, wherein said NKp46 fragment comprises at least one of domains 1 and 2 of the NKp46 molecule.

4. The complex of claim 3, wherein
(a) said NKp46 fragment comprises domains 1 and 2 of the NKp46 molecule and has the amino acid sequence substantially as denoted by any one of SEQ ID NO:4 and SEQ ID NO:13; or
(b) said NKp46 fragment comprises domain 2 of the NKp46 molecule and has the amino acid sequence substantially as denoted by any one of SEQ ID NO:22 and SEQ ID NO:23.

5. The complex of claim 1, wherein said NKp44 fragment comprises at least one of domains 1 and 2 of the NKp44 molecule.

6. The complex of claim 5, wherein
(a) said NKp44 fragment comprises domains 1 and 2 of the NKp44 molecule and has the amino acid sequence substantially as denoted by SEQ ID NO:9; or
(b) said NKp44 fragment comprises domain 2 of the NKp44 molecule and has the amino acid sequence substantially as denoted by SEQ ID NO:24.

7. The complex of any one of claims 1 to 6, wherein said complex is a fusion protein comprising as said active segment an Ig fragment, and said Ig fragment is the Fc portion of an Ig molecule, encoded by the amino acid sequence substantially as denoted by SEQ ID NO:5.

8. The complex of any one of claims 2 to 4, wherein said complex is a fusion protein comprising as said active segment an Ig fragment, and said Ig fragment is the Fc portion of an Ig molecule, encoded by the amino acid sequence substantially as denoted by SEQ ID NO:5.

9. The complex of any one of claims 1 to 6, wherein
(a) said complex is a conjugate comprising as said active segment a cytotoxic moiety, selected from cytotoxins or anticellular agents capable of killing and/or suppressing the growth or cell division of said target cell; or
(b) said complex is a conjugate comprising as a said active segment an imaging moiety, which imaging moiety is selected from the group consisting of paramagnetic, radioactive and fluorogenic agents.

10. The complex of any one of claims 2 to 4, wherein
(a) said complex is a conjugate comprising as said active segment a cytotoxic moiety, selected from cytotoxins or anticellular agents capable of killing and/or suppressing the growth or cell division of said target cell; or
(b) said complex is a conjugate comprising as a said active segment an imaging moiety, which imaging moiety is selected from the group consisting of paramagnetic, radioactive and fluorogenic agents.

11. The conjugate of any one of claims 9 and 10, wherein said cytotoxin or anticellular agent is any one of synthetic and plant-, fungus-, or bacteria-derived toxin.

12. The conjugate of claim 11, wherein said toxin is selected from the group consisting of A chain toxin, ribosome inactivating protein, α-sarcin, aspergillin, restrictocin, ribonudease, diphteria toxin, *Pseudomonas* exotoxin, an endotoxin or the lipid A moiety of an endotoxin.

13. The complex of any one of claims 1 and 2, wherein said target cell is a diseased cell.

14. The complex of claim 13, wherein said diseased cell is a cancer cell.

15. The complex of claim 14, wherein said cancer cell is selected from the group consisting of carcinomas, melanomas, lymphomas and sarcomas.

16. The complex of any one of claims 1 and 2, wherein said target cell is a pathogenic virus-infected cell.

17. The complex of claim 16, wherein said pathogenic virus is any one of Influenza virus, human immunodeficiency virus, Epstein-Barr virus, cytomegalovirus, Vaccinia virus, MVM, ECMV and Herpes virus.

18. The complex of claim 17, wherein said target recognition segment is capable of binding to a ligand expressed on the surface of said target cell, said binding being sialic acid mediated.

19. An expression vector comprising a DNA coding for NKp44-Ig or NKp46-Ig fusion protein as recited in claim 7 or 8, said protein comprising:
(a) a target recognition segment which comprises NKp44 or NKp46 or functional fragments thereof; and
(b) an active segment which is the Fc portion of an Ig molecule.

20. The expression vector of claim 19, wherein said NKp46 fragment comprises at least one of domains 1 and 2 of the NKp46 molecule.

21. The expression vector of claim 20, wherein
(a) said NKp46 fragment comprises domains 1 and 2 of the NKp46 molecule encoded by the nucleic acid sequence substantially as denoted by any one of SEQ ID NO:1 and SEQ ID NO:11 and the Fc portion of an Ig molecule is encoded by the nucleic acid sequence substantially as denoted by SEQ ID NO:2;
(b) said NKp46 fragment comprises domain 2 of the NKp46 molecule encoded by the nucleic acid sequence substantially as denoted by any one of SEQ ID NO:19 and SEQ ID NO:20 and the Fc portion of an Ig molecule is encoded by the nucleic acid sequence substantially as denoted by SEQ ID NO:2.

22. The expression vector of claim 19, wherein said NKp44 fragment comprises at least one of domains 1 and 2 of the NKp44 molecule.

23. The expression vector of claim 22, wherein
(a) said NKp44 fragment comprises domains 1 and 2 of the NKp44 molecule encoded by the nucleic acid sequence substantially as denoted by SEQ ID NO:7 and the Fc portion of an Ig molecule is encoded by the nucleic acid sequence substantially as denoted by SEQ ID NO:2; or
(b) said NKp44 fragment comprises domain 2 of the NKp44 molecule encoded by the nucleic acid sequence substantially as denoted by SEQ ID NO:21 and the Fc portion of an lg molecule is encoded by the nucleic acid sequence substantially as denoted by SEQ ID NO:2.

24. A host cell transformed with the expression vector of any one of claims 19 to 23.

25. A NKp46-Ig fusion protein encoded by a vector according to any of claims 19 to 21, comprising the amino acid sequence substantially as denoted by any one of SEQ ID NO:6, SEQ ID NO:14, encoded by the nucleic acid sequence substantially as denoted by SEQ ID NO:3 and SEQ ID NO:12, respectively.

26. A NKp44-Ig fusion protein encoded by a vector according to any of claims 19, 22 or 23, comprising the amino acid sequence substantially as denoted by SEQ ID NO:10, encoded by the nucleic acid sequence substantially as denoted by SEQ ID NO:8:

27. An antibody that specifically recognizes and binds to the fusion protein of claim 25 or 26.

28. A composition for the treatment of a pathological condition comprising as an active ingredient a complex comprising:
(a) a target recognition segment capable of specifically recognizing and binding to a diseased target cell involved with said pathological condition, said recognition segment comprising one of NKp46, NKp44, NKp30 or a biologically functional fragment thereof; and
(b) an active segment selected from the group consisting of a cytotoxic moiety and an Ig fragment.

29. The composition according to claim 28, wherein said recognition segment comprises NKp46 or a biologically functional fragment thereof.

30. The composition of any one of claims 28 and 29, wherein said pathological condition is a cancer disease selected from the group comprising of carcinomas, melanomas, lymphomas and sarcomas.

31. The composition of any one of claims 28 and 29, wherein said pathological condition is a viral infection caused by any one of Influenza virus, human immunodeficiency virus, Epstein-Barr virus, cytomegalovirus, Vaccinia virus, MVM, ECMV and Herpes virus.

32. The composition of claim 31, wherein the target recognition segment comprising NKp46 or functional fragments thereof is capable of binding to a ligand expressed on the surface of said target cell, said binding being sialic acid mediated.

33. The composition of claim 31, wherein the target recognition segment comprising NKp44 or functional fragments thereof is capable of binding to a ligand expressed on the surface of said target cell, said binding being sialic acid mediated.

34. The composition of any one of claims 28 to 32, wherein said NKp46 fragment comprises at least one of domains 1 and 2 of the NKp46 molecule.

35. The composition of claim 34, wherein
(a) said NKp46 fragment comprises domains 1 and 2 of the NKp46 molecule and has the amino acid sequence substantially as denoted by any one of SEQ ID NO:4 and SEQ ID NO:13; or
(b) said NKp46 fragment comprises domain 2 of the NKp46 molecule and has the amino acid sequence substantially as denoted by any one of SEQ ID NO:22 and SEQ ID NO:23.

36. The composition of any one of claims 28, 30, 31 and 33, wherein said NKp44 fragment, comprises at least one of domains 1 and 2 of the NKp44 molecule.

37. The composition of claim 36, wherein
(a) said NKp44 fragment comprises domains 1 and 2 of the NKp44 molecule and has the amino acid sequence substantially as denoted by SEQ ID NO:9; or
(b) said NKp44 fragment comprises domain 2 of the NKp44 molecule and has the amino acid sequence substantially as denoted by SEQ ID NO:24.

38. The composition of any one of claims 35 to 37, wherein said active segment is an Ig fragment which is the Fc portion of an Ig molecule encoded by the amino acid sequence substantially as denoted by SEQ ID NO:5.

39. The composition of claim 28, wherein said active segment is an Ig fragment which is the Fc portion of an lg molecule encoded by the amino acid sequence substantially as denoted by SEQ ID NO:5.

40. The composition of any one of claims 35 to 37, wherein said active segment is a cytotoxic moiety, selected from cytotoxins or anticellular agents capable of killing and/or suppressing the growth and/or cell division of said target cell.

41. The composition of claim 28, wherein said active segment is a cytotoxic moiety, selected from cytoxins or anticellular agents capable of killing and/or suppressing the growth and/or cell division of said target cell.

42. The composition of any one of claims 40 and 41, wherein said cytotoxin or anticellular agent is any one of synthetic and plant-, fungus-, or bacterium-derived toxin.

43. The composition of claim 42, wherein said toxin is selected from the group consisting of A chain toxin, ribosome inactivating protein, α-sarcin, aspergillin, restrictocin, ribonuclease, diphteria toxin, *Pseudomonas* exotoxin, an endotoxin or the lipid A moiety of an endotoxin.

44. A diagnostic compositon for detecting the presence of diseased cells in a sample comprising a complex comprising:
(a) target cell involved with said pathological condition, said recognition segment comprising one of NKp46, NKp44, NKp30 or a biologically functional fragment thereof; and
(b) a detectable imaging segment which is an imaging moiety selected from the group consisting of paramagnetic; radioactive and fluorogenic agent.

45. The diagnostic composition according to claim 44, wherein said recognition segment comprises NKp46 or a biologically functional fragment thereof

46. The diagnostic composition of claim 45, wherein said NKp46 fragment comprises at least one of domains 1 and 2 of the NKp46 molecule.

47. The diagnostic composition of claim 46, wherein
(a) said NKp46 fragment comprises domains 1 and 2 of the NKp46 molecule and has the amino acid sequence substantially as denoted by anyone of SEQ ID NO:4 and SEQ ID NO:13; or
(b) said NKp46 fragment comprises domain 2 of the NKp46 molecule and has the amino acid sequence substantially as denoted by any one of SEQ ID NO:22 and SEQ ID NO:23.

48. The diagnostic composition of claim 44, wherein said NKp44 fragment comprises at least one of domains 1 and 2 of the NKp44 molecule.

49. The diagnostic composition of claim 48, wherein
(a) said NKp44 fragment comprises domains 1 and 2 of the NKp44 molecule and has the amino acid sequence substantially as denoted by SEQ ID NO:9; or
(b) said NKp44 fragment comprises domain 2 of the NKp44 molecule and has the amino acid sequence substantial as denoted by SEQ ID NO:24.

50. The diagnostic composition of any one of claims 44 to 49, wherein the target recognition segment is capable of binding to a ligand expressed on the surface of said target cell.

51. The diagnostic composition of claim 50, wherein said target is a cancer cell.

52. The diagnostic composition of claim 51, wherein said cancer is a cancer disease selected from the group consisting of carcinomas, melanomas, lymphomas and sarcomas.

53. The use of a complex or pharmaceutical agent comprising the same, said complex comprising:
(a) a first target recognition segment capable of specifically recognizing and binding to a diseased target cell involved with said pathological condition, said recognition segment comprising one of NKp46, NKp44, NKp30 or a biologically functional fragment thereof; and
(b) an active segment selected from the group consisting of a cytotoxic moiety and an Ig fragment
for the preparation of a pharmaceutical composition for the treatment of a pathological condition in a subject, wherein the pathological condition is selected from the groups consisting of:
(i) a cancer disease selected from the group consisting of carcinomas, melanomas, lymphomas and sarcomas; and
(ii) a viral infection caused by any one of Influenza virus, human immunodeficiency virus, Epstein-Barr virus, cytomegalovirus, Vaccinia virus, MVM, ECMV and Herpes virus.

54. The use of claim 53, wherein said complex comprises:
(a) a first target recognition segment capable of specifically recognizing and binding to a diseased target cell involved with said pathological condition, said recognition segment comprising NKp46 or a biologically functional fragment thereof; and
(b) an active segment selected from the group consisting of cytotoxic moiety and an Ig fragment.

55. The use of any of claims 53 or 54, wherein the target recognition segment NKp46 or functional fragments thereof is capable of binding to a ligand expressed on the surface of said target cell, said binding being sialic acid mediated.

56. The use of claim 53, wherein the target recognition segment NKp44 or functional fragments thereof is capable of binding to a ligand expressed on the surface of said target cell, said binding being sialic acid mediated.

57. The use according to any one of claims 53 to 55, wherein said NKp46 fragment comprises at least one of domains 1 and 2 of the NKp46 molecule.

58. The use according to claim 57, wherein
(a) said NKp46 fragment comprises domains 1 and 2 of the NKp46 molecule and has the amino acid sequence substantially as denoted by any one of SEQ ID NO:4 and SEQ ID NO:13; or
(b) said NKp46 fragment comprises domain 2 of the NKp46 molecule and has the amino acid sequence substantially as denoted by any one of SEQ ID NO:22 and SEQ ID NO:23.

59. The use according to claim 53 or 56, wherein said NKp44 fragment comprises at least one of domains 1 and 2 of the NKp44 molecule.

60. The use according to claim 59, wherein
(a) said NKp44 fragment, comprises domains 1 and 2 of the NKp44 molecule and has the amino acid sequence substantially as denoted by SEQ ID NO:9.
(b) said NKp44 fragment, comprises domain 2 of the NKp44 molecule and has the amino acid sequence substantially as denoted by SEQ ID NO:24.

61. The use of any one of claims 58 and 60, wherein said active segment is an lg fragment which is the Fc portion of an lg molecule encoded by the amino acid sequence substantially as denoted by SEQ ID NO:5.

62. The use of any one of claims 58 and 60, wherein said active segment is a cytotoxic moiety, selected from cytotoxins or anticellular agents capable of killing and/or suppressing the growth and/or cell division of said target cell.

63. The use of claim 62, wherein said cytotoxin or anticellular agent is any one of synthetic, and plant-, fungus-, or bacterium-derived toxin.

64. The use of claim 63, wherein said toxin is selected from the group consisting of A chain toxin, ribosome inactivating protein, α-sarcin, aspergillin, restrictocin, ribonuclease, diphteria toxin, Pseudomonas exotoxin, an endotoxin or the lipid A moiety of an endotoxin.

65. Use of an imaging agent comprising a complex having:
(a) a first target recognition segment, capable of specifically recognizing and binding to said malignant cells, said recognition segment comprising one of NKp46, NKp30, NKp44 or a biologically functional fragment thereof; and
(b) an active segment which is an imaging moiety, said imaging moiety being selected from the group consisting of paramagnetic, radioactive and fluorogenic agents
for the preparation of a diagnostic composition for the diagnosis and imaging of malignant cells in a subject, wherein said diagnosis and imaging comprises the introduction of said imaging agent into the blood-stream of said subject, and detecting and quantitating the binding of said imaging agent to any one of NKp46, NKp30 and NKp44 ligands expressed on target malignant cells.

66. The use of an imaging agent according to claim 65, wherein said recognition segment comprises NKp46 or a biologically functional fragment thereof and wherein the binding of said imaging agent to NKp46 ligands expressed on target malignant cells is detected and quantified.

67. The use of claim 66, wherein said NKp46 fragment comprises at least one of domains 1 and 2 of the NKp46 molecule:

68. The use of claim 67, wherein
(a) said NKp46 fragment comprises domains 1 and 2 of the NKp46 molecule and has the amino acid sequence substantially as denoted by any one of SEQ ID NO:4 and SEQ ID NO:13; or
(b) said NKp46 fragment comprises domain 2 of the NKp46 molecule and has the amino acid sequence substantially as denoted by any one of SEQ ID NO:22 and SEQ ID NO:23.

69. The use of claim 65, wherein said NKp44 fragment comprises at least one of domains 1 and 2 of the NKP44 molecule.

70. The use of claim 69, wherein
(a) said NKp44 fragment comprises domains 1 and 2 of the NKp44 molecule and has the amino acid sequence substantially as denoted by SEQ ID NO:9; or
(b) said NKp44 fragment comprises domain 2 of the NKp44 molecule and has the amino acid sequence substantially as denoted by SEQ ID NO:24.

71. The use of any one of claims 65 to 70, wherein said malignant cells are selected from the group consisting of carcinomas, melanomas, lymphomas and sarcomas.

## Patentansprüche

1. Zielgerichteter Komplex, fähig zum spezifischen Dirigieren eines Wirkstoffs zu entarteten Zielzelltypen, ausgewählt aus der Gruppe bestehend aus Zellen abgeleitet von malignen Tumoren, Zellen abgeleitet von soliden Tumoren, Zellen abgeleitet von nicht-soliden Tumoren, einer defekten oder erkrankten Zelle und einer virusinfizierten Zelle, wobei der Komplex umfasst:
(a) ein Zielerkennungssegment, umfassend einen für NK-Zellen spezifischen Rezeptor, wobei der Rezeptor die Lyse der Zielzelle aktiviert, wobei der Rezeptor ausgewählt ist aus der Gruppe bestehend aus NKp46, NKp44, NKp30 oder einem funktionellen Fragment davon; und
(b) ein aktives Segment, umfassend den Wirkstoff, wobei der Wirkstoff ausgewählt ist aus der Gruppe bestehend aus einer cytotoxischen Einheit, einer bildgebenden Einheit und einem Ig-Fragment.

2. Zielgerichteter Komplex nach Anspruch 1, wobei das Zielerkennungssegment NKp46 oder ein funktionelles Fragment davon umfasst.

3. Komplex nach Anspruch 2, wobei das NKp46-Fragment mindestens eine der Domänen 1 und 2 des NKp46-Moleküls umfasst.

4. Komplex nach Anspruch 3, wobei
(a) das NKp46-Fragment die Domänen 1 und 2 des NKp46-Moleküls umfasst und eine Aminosäuresequenz besitzt, die im Wesentlichen wie in einer der SEQ ID NO: 4 und SEQ ID NO: 13 dargestellt ist; oder
(b) das NKp46-Fragment die Domäne 2 des NKp46-Moleküls umfasst und eine Aminosäuresequenz besitzt, die im Wesentlichen wie in einer der SEQ ID NO: 22 und SEQ ID NO: 23 dargestellt ist.

5. Komplex nach Anspruch 1, wobei das NKp44-Fragment mindestens eine der Domänen 1 und 2 des NKp44-Moleküls umfasst.

6. Komplex nach Anspruch 5, wobei
(a) das NKp44-Fragment die Domänen 1 und 2 des NKp44-Moleküls umfasst und eine Aminosäuresequenz besitzt, die im Wesentlichen wie in SEQ ID NO: 9 dargestellt ist; oder
(b) das NKp44-Fragment die Domäne 2 des NKp44-Moleküls umfasst und eine Aminosäuresequenz besitzt, die im Wesentlichen wie in SEQ ID NO: 24 dargestellt ist.

7. Komplex nach einem der Ansprüche 1 bis 6, wobei der Komplex ein Fusionsprotein ist, umfassend als das aktive Segment ein Ig-Fragment, und wobei das Ig-Fragment der Fc-Anteil eines Ig-Moleküls ist, codiert durch die Aminosäuresequenz, die im Wesentlichen wie in SEQ ID NO: 5 dargestellt ist.

8. Komplex nach einem der Ansprüche 2 bis 4, wobei der Komplex ein Fusionsprotein ist, umfassend als das aktive Segment ein Ig-Fragment, und wobei das Ig-Fragment der Fc-Anteil eines Ig-Moleküls ist, codiert durch eine Aminosäuresequenz, die im Wesentlichen wie in SEQ ID NO: 5 dargestellt ist.

9. Komplex nach einem der Ansprüche 1 bis 6, wobei
(a) der Komplex ein Konjugat ist, umfassend als das aktive Segment eine cytotoxische Einheit, ausgewählt aus Cytotoxinen oder anti-zellulären Agentien, die fähig sind zum Abtöten und/oder Unterdrücken des Wachstums oder der Zellteilung der Zielzelle; oder
(b) der Komplex ein Konjugat ist, umfassend als das aktive Segment eine bildgebende Einheit, wobei die bildgebende Einheit ausgewählt ist aus der Gruppe bestehend aus paramagnetischen, radioaktiven und fluorogenen Agentien.

10. Komplex nach einem der Ansprüche 2 bis 4, wobei
(a) der Komplex ein Konjugat ist, umfassend als das aktive Segment eine cytotoxische Einheit, ausgewählt aus Cytotoxinen oder anti-zellulären Agentien, die fähig sind zum Abtöten und/oder Unterdrücken des Wachstums oder der Zellteilung der Zielzelle; oder
(b) der Komplex ein Konjugat ist, umfassend als das aktive Segment eine bildgebende Einheit, wobei die bildgebende Einheit ausgewählt ist aus der Gruppe bestehend aus paramagnetischen, radioaktiven und fluorogenen Agentien.

11. Konjugat nach einem der Ansprüche 9 und 10, wobei das Cytotoxin oder anti-zelluläre Agens ein synthetisches oder ein aus Pflanzen, Pilzen oder Bakterien gewonnenes Toxin ist.

12. Konjugat nach Anspruch 11, wobei das Toxin ausgewählt ist aus der Gruppe bestehend aus A-Ketten-Toxin, Ribosom-inaktivierendem Protein, α-Sarcin, Aspergillin, Restrictocin, Ribonuclease, Diphtherie-Toxin, Pseudomonas-Exotoxin, einem Endotoxin oder der Lipid A-Einheit eines Endotoxins.

13. Komplex nach einem der Ansprüche 1 und 2, wobei die Zielzelle eine erkrankte Zelle ist.

14. Komplex nach Anspruch 13, wobei die erkrankte Zelle eine Krebszelle ist.

15. Komplex nach Anspruch 14, wobei die Krebszelle ausgewählt ist aus der Gruppe bestehend aus Karzinomen, Melanomen, Lymphomen und Sarkomen.

16. Komplex nach einem der Ansprüche 1 und 2, wobei die Zielzelle eine durch ein pathogenes Virus infizierte Zelle ist.

17. Komplex nach Anspruch 16, wobei das pathogene Virus eines ist aus Influenzavirus, menschlichem Immunschwächevirus, Epstein-Barr-Virus, Cytomegalievirus, Vacciniavirus, MVM, ECMV und Herpesvirus.

18. Komplex nach Anspruch 17, wobei das Zielerkennungssegment fähig ist zur Bindung an einen Liganden, der auf der Oberfläche der Zielzelle exprimiert wird, wobei die Bindung Sialinsäure-vermittelt ist.

19. Expressionsvektor, umfassend eine DNA, die ein NKp44-Ig- oder ein NKp46-Ig-Fusionsprotein gemäß Anspruch 7 oder 8 codiert, wobei das Protein umfasst:
(a) ein Zielerkennungssegment, welches NKp44 oder NKp46 oder funktionelle Fragmente davon umfasst; und
(b) ein aktives Segment, welches der Fc-Anteil eines lg-Moleküls ist.

20. Expressionsvektor nach Anspruch 19, wobei das NKp46-Fragment mindestens eine der Domänen 1 und 2 des NKp46-Moleküls umfasst.

21. Expressionsvektor nach Anspruch 20, wobei
(a) das NKp46-Fragment die Domänen 1 und 2 des NKp46-Moleküls umfasst, codiert durch eine Nucleinsäuresequenz, die im Wesentlichen wie in einer der SEQ ID NO: 1 und SEQ ID NO: 11 dargestellt ist und wobei der Fc-Anteill eines lg-Moleküls codiert ist durch eine Nucleinsäuresequenz, die im Wesentlichen wie in SEQ ID NO: 2 dargestellt ist;
(b) das NKp46-Fragment die Domäne 2 des NKp46-Moleküls umfasst, codiert durch die Nucleinsäuresequenz, die im Wesentlichen wie in einer der SEQ ID NO: 19 und SEQ ID NO: 20 dargestellt ist und wobei der Fc-Anteil eines lg-Moleküls codiert wird durch die Nucleinsäuresequenz, die im Wesentlichen wie in SEQ ID NO: 2 dargestellt ist.

22. Expressionsvektor nach Anspruch 19, wobei das NKp44-Fragment mindestens eine der Domänen 1 und 2 des NKp44-Moleküls umfasst.

23. Expressionsvektor nach Anspruch 22, wobei
(a) das NKp44-Fragment die Domänen 1 und 2 des NKp44-Moleküls umfasst, codiert durch die Nucleinsäuresequenz, die im Wesentlichen wie in SEQ ID NO: 7 dargestellt ist, und wobei der Fc-Anteil eines Ig-Moleküls codiert ist durch die Nucleinsäuresequenz, die im Wesentlichen wie in SEQ ID NO: 2 dargestellt ist, oder
(b) das NKp44-Fragment die Domäne 2 des NKp44-Moleküls umfasst, codiert durch die Nucleinsäuresequenz, die im Wesentlichen wie in SEQ ID NO: 21 dargestellt ist, und wobei der Fc-Anteil des Ig-Moleküls codiert ist durch die Nucleinsäuresequenz, die im Wesentlichen wie in SEQ ID NO: 2 dargestellt ist.

24. Wirtszelle, transformiert mit einem Expressionsvektor nach einem der Ansprüche 19 bis 23.

25. NKp46-lg-Fusionsprotein, codiert durch einen Vektor nach einem der Ansprüche 19 bis 21, umfassend die Aminosäuresequenz, die im Wesentlichen wie in einer der SEQ ID NO: 6 und SEQ ID NO: 14 dargestellt ist, bzw. codiert durch eine Nucleinsäuresequenz, die im Wesentlichen wie in SEQ ID NO: 3 und SEQ ID NO: 12 dargestellt ist.

26. NKp44-lg-Fusionsprotein, codiert durch einen Vektor nach einem der Ansprüche 19, 22 oder 23, umfassend die Aminosäuresequenz, die im Wesentlichen wie in SEQ ID NO: 10 dargestellt ist, codiert durch die Nucleinsäuresequenz, die im Wesentlichen wie in SEQ ID NO: 8 dargestellt ist.

27. Antikörper, der spezifisch das Fusionsprotein nach Anspruch 25 oder 26 erkennt und bindet.

28. Zusammensetzung zur Behandlung eines pathologischen Zustands, umfassend als Wirkstoff einen Komplex, der umfasst:
(a) ein Zielerkennungssegment, das fähig ist zur spezifischen Erkennung und Bindung an eine erkrankte Zielzelle, die beteiligt ist an dem pathologischen Zustand, wobei das Erkennungssegment NKp46, NKp44, NKp30 oder ein biologisch funktionelles Fragment davon umfasst; und
(b) ein aktives Segment, ausgewählt aus der Gruppe bestehend aus einer cytotoxischen Einheit und einem Ig-Fragment.

29. Zusammensetzung nach Anspruch 28, wobei das Erkennungssegment NKp46 oder ein biologisch funktionelles Fragment davon umfasst.

30. Zusammensetzung nach einem der Ansprüche 28 und 29, wobei der pathologische Zustand eine Krebserkrankung ist, ausgewählt aus der Gruppe aus Karzinomen, Melanomen, Lymphomen und Sarkomen.

31. Zusammensetzung nach einem der Ansprüche 28 und 29, wobei der pathologische Zustand eine virale Infektion ist, ausgelöst durch eines aus Influenzavirus, menschlichem Immunschwächevirus, Epstein-Barr-Virus, Cytomegalievirus, Vaccinavirus, MVM, ECMV und Herpesvirus.

32. Zusammensetzung nach Anspruch 31, wobei das Zielerkennungssegment, welches NKp46 oder funktionelle Fragmente davon umfasst, fähig ist zur Bindung an einen Liganden, der auf der Oberfläche der Zielzelle exprimiert wird, wobei die Bindung Sialinsäure-vermittelt ist.

33. Zusammensetzung nach Anspruch 31, wobei das Zielerkennungssegment, welches NKp44 oder funktionelle Fragmente davon umfasst, fähig ist zur Bindung an einen Liganden, der auf der Oberfläche der Zielzelle exprimiert wird, wobei die Bindung Sialinsäure-vermittelt ist.

34. Zusammensetzung nach einem der Ansprüche 28 bis 32, wobei das NKp46-Fragment mindestens eine der Domänen 1 und 2 des NKp46-Moleküls umfasst.

35. Zusammensetzung nach Anspruch 34, wobei
(a) das NKp46-Fragment die Domänen 1 und 2 des NKp46-Moleküls umfasst und eine Aminosäuresequenz besitzt, die im Wesentlichen wie in einer der SEQ 10 NO: 4 und SEQ ID NO: 13 dargestellt ist;
(b) das NKp46-Fragment die Domäne 2 des NKp46-Moleküls umfasst und eine Aminosäuresequenz besitzt, die im Wesentlichen wie in einer der SEQ ID NO: 22 und SEQ ID NO: 23 dargestellt ist.

36. Zusammensetzung nach einem der Ansprüche 28, 30, 31 und 33, wobei das NKp44-Fragment mindestens eine der Domänen 1 und 2 des NKp44-Moleküls umfasst.

37. Zusammensetzung nach Anspruch 36, wobei
(a) das NKp44-Fragment die Domänen 1 und 2 des NKp44-Moleküls umfasst und eine Aminosäuresequenz besitzt, die im Wesentlichen wie in SEQ ID NO: 9 dargestellt ist; oder
(b) das NKp44-Fragment die Domäne 2 des NKp44-Moleküls umfasst und eine Aminosäuresequenz besitzt, die im Wesentlichen wie in SEQ ID NO: 24 dargestellt ist.

38. Zusammensetzung nach einem der Ansprüche 35 bis 37, wobei das aktive Segment ein Ig-Fragment ist, welches der Fc-Anteil eines Ig-Moleküls ist, codiert durch eine Aminosäuresequenz, die im Wesentlichen wie in SEQ ID NO: 5 dargestellt ist.

39. Zusammensetzung nach Anspruch 28, wobei das aktive Fragment ein Ig-Fragment ist, welches der Fc-Anteil eines Ig-Moleküls ist, codiert durch eine Aminosäuresequenz, die im Wesentlichen wie in SEQ ID NO: 5 dargestellt ist.

40. Zusammensetzung nach einem der Ansprüche 35 bis 37, wobei das aktive Segment eine cytotoxische Einheit ist, ausgewählt aus Cytotoxinen oder anti-zellulären Agentien, die fähig sind zum Abtöten und/oder Unterdrücken des Wachstums und/oder der Zellteilung der Zielzelle.

41. Zusammensetzung nach Anspruch 28, wobei das aktive Segment eine cytotoxische Einheit ist, ausgewählt aus Cytotoxinen und anti-zellulären Agentien, die fähig sind zum Abtöten und/oder Unterdrücken des Wachstums und/oder der Zellteilung der Zielzelle.

42. Zusammensetzung nach einem der Ansprüche 40 und 41, wobei das Cytotoxin oder anti-zelluläre Agens ein synthetisches oder ein aus Pflanzen, Pilzen oder Bakterien gewonnenes Toxin ist.

43. Zusammensetzung nach Anspruch 42, wobei das Toxin ausgewählt ist aus der Gruppe bestehend aus A-Ketten-Toxin, Ribosom-inaktivierendem Protein, α-Sarcin, Aspergillin, Restrictocin, Ribonuclease, Diphtherie-Toxin, Pseudomonas-Exotoxin, einem Endotoxin oder der Lipid A-Einheit eines Endotoxins.

44. Diagnostische Zusammensetzung zum Nachweis der Anwesenheit von erkrankten Zellen in einer Probe, umfassend einen Komplex, der umfasst:
(a) eine Zielzelle, die beteiligt ist an dem pathologischen Zustand, wobei das Erkennungssegment eines von NKp46, NKp44, NKp30 oder ein biologisch funktionelles Fragment davon umfasst; und
(b) ein nachweisbares bildgebendes Segment, welches eine bildgebende Einheit ist, ausgewählt aus der Gruppe bestehend aus paramagnetischen, radioaktiven und fluorogenen Agentien.

45. Diagnostische Zusammensetzung nach Anspruch 44, wobei das Erkennungssegment NKp46 oder ein biologisch funktionelles Fragment davon umfasst.

46. Diagnostische Zusammensetzung nach Anspruch 45, wobei das NKp46-Fragment mindestens eine der Domänen 1 und 2 des NKp46-Moleküls umfasst.

47. Diagnostische Zusammensetzung nach Anspruch 46, wobei
(a) das NKp46-Fragment die Domänen 1 und 2 des NKp46-Moleküls umfasst und eine Aminosäuresequenz besitzt, die im Wesentlichen wie in einer der SEQ ID NO: 4 und SEQ ID NO: 13 dargestellt ist; oder
(b) das NKp46-Fragment die Domäne 2 des NKp46-Moleküls umfasst und eine Aminosäuresequenz besitzt, die im Wesentlichen wie in einer der SEQ ID NO: 22 und SEQ ID NO: 23 dargestellt ist.

48. Diagnostische Zusammensetzung nach Anspruch 44, wobei das NKp44-Fragment mindestens eine der Domänen 1 und 2 des NKp44-Moleküls umfasst.

49. Diagnostische Zusammensetzung nach Anspruch 48, wobei
(a) das NKp44-Fragment die Domänen 1 und 2 des NKp44-Moleküls umfasst und eine Aminosäuresequenz besitzt, die im Wesentlichen wie in SEQ ID NO: 9 dargestellt ist; oder
(b) das NKp44-Fragment die Domäne 2 des NKp44-Moleküls umfasst und eine Aminosäuresequenz besitzt, die im Wesentlichen wie in SEQ ID NO: 24 dargestellt ist.

50. Diagnostische Zusammensetzung nach einem der Ansprüche 44 bis 49, wobei das Zielerkennungssegment fähig ist zur Bindung an einen Liganden, der auf der Oberfläche der Zielzelle exprimiert wird.

51. Diagnostische Zusammensetzung nach Anspruch 50, wobei die Zielzelle eine Krebszelle ist.

52. Diagnostische Zusammensetzung nach Anspruch 51, wobei die Krebserkrankung ausgewählt ist aus der Gruppe bestehend aus Karzinomen, Melanomen, Lymphomen und Sarkomen.

53. Verwendung eines Komplexes oder eines pharmazeutischen Wirkstoffs umfassend denselben, wobei der Komplex umfasst:
(a) ein erstes Zielerkennungssegment, das fähig ist zum spezifischen Erkennen und Binden an eine erkrankte Zelle, die beteiligt ist am pathologischen Zustand, wobei das Erkennungssegment eines aus NKp46, NKp44, NKp30 oder einem biologisch funktionellen Fragment davon umfasst; und
(b) ein aktives Segment, ausgewählt aus der Gruppe bestehend aus einer cytotoxischen Einheit und einem Ig-Fragment,
für die Herstellung eines Arzneimittels zur Behandlung eines pathologischen Zustands in einem Individuum, wobei der pathologische Zustand ausgewählt ist aus der Gruppe bestehend aus:
(i) einer Krebserkrankung, ausgewählt aus der Gruppe bestehend aus Karzinomen, Melanomen, Lymphomen und Sarkomen; und
(ii) einer Viruserkrankung ausgelöst durch eines aus Influenzavirus, menschlichem Immunschwächevirus, Epstein-Barr-Virus, Cytomegalievirus, Vacciniavirus, MVM, ECMV und Herpesvirus.

54. Verwendung nach Anspruch 53, wobei der Komplex umfasst:
(a) ein erstes Zielerkennungssegment, das fähig ist zum spezifischen Erkennen und Binden an eine erkrankte Zelle, die beteiligt ist am pathologischen Zustand, wobei das Erkennungssegment NKp46 oder ein funktionelles Fragment davon umfasst; und
(b) ein aktives Segment, ausgewählt aus der Gruppe bestehend aus einer cytotoxischen Einheit und einem Ig-Fragment.

55. Verwendung nach Anspruch 53 oder 54, wobei das Zielerkennungssegment NKp46 oder ein funktionelles Fragment davon ist, das fähig ist zur Bindung an einen Liganden, der auf der Oberfläche der Zielzelle exprimiert wird, wobei die Bindung Sialinsäure-vermittelt ist.

56. Verwendung nach Anspruch 53, wobei das Zielerkennungssegment NKp44 oder ein funktionelles Fragment davon ist, das fähig ist zur Bindung an einen Liganden, der auf der Oberfläche der Zielzelle exprimiert wird, wobei die Bindung Sialinsäure-vermittelt ist.

57. Verwendung nach einem der Ansprüche 53 bis 55, wobei das NKp46-Fragment mindestens eine der Domänen 1 und 2 des NKp46-Moleküls umfasst.

58. Verwendung nach Anspruch 57, wobei
(a) das NKp46-Fragment die Domänen 1 und 2 des NKp46-Moleküls umfasst und eine Aminosäuresequenz besitzt, die im Wesentlichen wie in einer der SEQ ID NO: 4 und SEQ ID NO: 13 dargestellt ist; oder
(b) das NKp46-Fragment die Domäne 2 des NKp46-Moleküls umfasst und eine Aminosäuresequenz besitzt, die im Wesentlichen wie in einer der SEQ ID NO: 22 und SEQ ID NO: 23 dargestellt ist.

59. Verwendung nach Anspruch 53 oder 56, wobei das NKp44-Fragment mindestens eine der Domänen 1 und 2 des NKp44-Moleküls umfasst.

60. Verwendung nach Anspruch 59, wobei
(a) das NKp44-Fragment die Domänen 1 und 2 des NKp44-Moleküls umfasst und eine Aminosäuresequenz besitzt, die im Wesentlichen wie in SEQ ID NO: 9 dargestellt ist; oder
(b) das NKp44-Fragment die Domäne 2 des NKp44-Moleküls umfasst und eine Aminosäuresequenz besitzt, die im Wesentlichen wie in SEQ ID NO: 24 dargestellt ist.

61. Verwendung nach Anspruch 58 und 60, wobei das aktive Segment ein Ig-Fragment ist, welches der Fc-Anteil eines lg-Moleküls ist, codiert durch eine Aminosäuresequenz, die im Wesentlichen wie in SEQ ID NO: 5 dargestellt ist.

62. Verwendung nach Anspruch 58 und 60, wobei das aktive Segment eine cytotoxische Einheit ist, ausgewählt aus Cytotoxinen und anti-zellulären Agentien, die fähig sind zum Abtöten und/oder Unterdrücken des Wachstums und/oder der Zellteilung der Zielzelle.

63. Verwendung nach Anspruch 62, wobei das Cytotoxin oder anti-zelluläre Agens ein synthetisches oder ein aus Pflanzen, Pilzen, oder Bakterien gewonnenes Toxin ist.

64. Verwendung nach Anspruch 63, wobei das Toxin ausgewählt ist aus der Gruppe bestehend aus A-Ketten-Toxin, Ribosom-inaktivierendem Protein, α-Sarcin, Aspergillin, Restrictocin, Ribonuclease, Diphtherie-Toxin, Pseudomonas-Exotoxin, einem Endotoxin oder der Lipid A-Einheit eines Endotoxins.

65. Verwendung eines bildgebenden Agens, umfassend einen Komplex mit:
(a) einem ersten Zielerkennungssegment, das fähig ist zum spezifischen Erkennen und Binden an die malignen Zellen, wobei das Erkennungssegment eines aus NKp46, NKp44, NKp30 oder einem biologisch funktionellen Fragment davon umfasst; und
(b) einem aktiven Segment, welches ein bildgebendes Segment ist, welches ausgewählt ist aus der Gruppe bestehend aus paramagnetischen, radioaktiven und fluorogenen Agentien,
für die Herstellung einer diagnostischen Zusammensetzung zur Diagnose und Bildgebung von malignen Zellen in einem Individuum, wobei die Diagnose und Bildgebung das Einführen des bildgebenden Agens in den Blutstrom des Individuums, den Nachweis und die Quantifizierung der Bindung des bildgebenden Agens an einen aus NKp46-, NKp30- und NKp44-Liganden, die auf den malignen Zielzellen exprimiert werden, umfasst.

66. Verwendung eines bildgebenden Agens nach Anspruch 65, wobei das Erkennungssegment NKp46 oder ein biologisch funktionelles Fragment davon umfasst, wobei die Bindung des bildgebenden Agens an NKp46-Liganden, die auf malignen Zielzellen exprimiert werden, nachgewiesen und quantifiziert wird.

67. Verwendung nach Anspruch 66, wobei das NKp46-Fragment mindestens eine der Domänen 1 und 2 des NKp46-Moleküls umfasst.

68. Verwendung nach Anspruch 67, wobei
(a) das NKp46-Fragment die Domänen 1 und 2 des NKp46-Moleküls umfasst und eine Aminosäuresequenz besitzt, die im Wesentlichen wie in einer der SEQ ID NO: 4 und SEQ ID NO: 13 dargestellt ist; oder
(b) das NKp46-Fragment die Domäne 2 des NKp46-Moleküls umfasst und eine Aminosäuresequenz besitzt, die im Wesentlichen wie in einer der SEQ ID NO: 22 und SEQ ID NO: 23 dargestellt ist.

69. Verwendung nach Anspruch 65, wobei das NKp44-Fragment mindestens eine der Domänen 1 und 2 des NKp44-Moleküls umfasst.

70. Verwendung nach Anspruch 69, wobei
(a) das NKp44-Fragment die Domänen 1 und 2 des NKp44-Moleküls umfasst und eine Aminosäuresequenz besitzt, die im Wesentlichen wie in SEQ ID NO: 9 dargestellt ist; oder
(b) das NKp44-Fragment die Domäne 2 des NKp44-Moleküls umfasst und eine Aminosäuresequenz besitzt, die im Wesentlichen wie in SEQ ID NO: 24 dargestellt ist.

71. Verwendung nach einem der Ansprüche 65 bis 70, wobei die malignen Zellen ausgewählt sind aus der Gruppe bestehend aus Karzinomen, Melanomen, Lymphomen und Sarkomen.

## Revendications

1. Complexe de ciblage capable de cibler spécifiquement une substance active vers des types cellulaires cibles anormaux choisis dans le groupe constitué par des cellules dérivées de tumeurs malignes, des cellules dérivées de tumeurs solides, des cellules dérivées de tumeurs non-solides, une cellule déficiente ou malade, et une cellule infectée par un virus, ledit complexe comprenant :
(a) un segment de reconnaissance de la cible comprenant un récepteur spécifique des cellules NK, le récepteur activant la lyse de la cible, ledit récepteur étant choisi dans le groupe constitué par NKp46, NKp44, NKp30 ou un fragment fonctionnel de ceux-ci ; et
(b) un segment actif comprenant la substance active, ladite substance active étant choisie dans le groupe constitué par : une partie cytotoxique ; un groupement d'imagerie ; et un fragment d'Ig.

2. Complexe de ciblage selon la revendication 1, dans lequel le segment de reconnaissance de la cible comprend NKp46 ou un fragment fonctionnel de celui-ci.

3. Complexe de la revendication 2, dans lequel ledit fragment de NKp46 comprend au moins un des domaines 1 et 2 de la molécule NKp46.

4. Complexe de la revendication 3, dans lequel
(a) ledit fragment de NKp46 comprend les domaines 1 et 2 de la molécule NKp46 et a la séquence d'acides aminés essentiellement comme décrit par l'une des séquences SEQ ID NO :4 et SEQ ID NO:13 ; ou
(b) ledit fragment de NKp46 comprend le domaine 2 de la molécule NKp46 et a la séquence d'acides aminés essentiellement comme décrit par l'une des séquences SEQ ID NO :22 et SEQ ID NO :23.

5. Complexe de la revendication 1, dans lequel ledit fragment de NKp44 comprend au moins un des domaines 1 et 2 de la molécule NKp44.

6. Complexe de la revendication 5, dans lequel
(a) ledit fragment de NKp44 comprend les domaines 1 et 2 de la molécule NKp44 et a la séquence d'acides aminés essentiellement comme décrit par la séquence SEQ ID NO :9 ; ou
(b) ledit fragment de NKp44 comprend le domaine 2 de la molécule NKp44 et a la séquence d'acides aminés essentiellement comme décrit par la séquence SEQ ID NO :24.

7. Complexe de l'une quelconque des revendications 1 à 6, dans lequel ledit complexe est une protéine de fusion comprenant, à titre dudit segment actif, un fragment d'Ig, et ledit fragment d' Ig est la partie Fc d'une molécule d' Ig, codée par la séquence d'acides aminés essentiellement comme décrit par la séquence SEQ ID NO :5.

8. Complexe de l'une quelconque des revendications 2 à 4, dans lequel ledit complexe est une protéine de fusion comprenant, à titre dudit segment actif, un fragment d'Ig, et ledit fragment d'Ig est la partie Fc d'une molécule d'Ig, codée par la séquence d'acides aminés essentiellement comme décrit par la séquence SEQ ID NO :5.

9. Complexe de l'une quelconque des revendications 1 à 6, dans lequel
(a) ledit complexe est un conjugué comprenant, à titre dudit segment actif, une partie cytotoxique, choisie parmi des cytotoxines ou agents anticellulaires capables de tuer et/ou de supprimer la croissance ou la division cellulaire de ladite cellule cible ; ou
(b) ledit complexe est un conjugué comprenant, à titre dudit segment actif, un groupement d'imagerie, ledit groupement d'imagerie étant choisi dans le groupe constitué par les agents paramagnétiques, radioactifs et fluorogéniques.

10. Complexe de l'une quelconque des revendication 2 à 4, dans lequel
(a) ledit complexe est un conjugué comprenant, à titre dudit segment actif, une partie cytotoxique, choisie parmi des cytotoxines ou agents anticellulaires capables de tuer et/ou de supprimer la croissance ou la division cellulaire de ladite cellule cible ; ou
(b) ledit complexe est un conjugué comprenant, à titre dudit segment actif, un groupement d'imagerie, ledit groupement d'imagerie étant choisi dans le groupe constitué par les agents paramagnétiques, radioactifs et fluorogéniques.

11. Conjugué de l'une quelconque des revendications 9 et 10, dans lequel ledit agent cytotoxique ou anticellulaire est une toxine quelconque dérivée de plantes, de champignons ou de bactéries ou une toxine synthétique.

12. Conjugué de la revendication 11, dans lequel ladite toxine est choisie dans le groupe constitué par la toxine à chaîne A, la protéine d'inactivation du ribosome, l'α-sarcine, l'aspergilline, la restrictocine, la ribonucléase, la toxine de la diphtérie, l'exotoxine de *Pseudomonas,* une endotoxine ou la partie lipide A d'une endotoxine.

13. Complexe de l'une quelconque des revendications 1 et 2, dans lequel ladite cellule cible est une cellule malade.

14. Complexe de la revendication 13, dans lequel ladite cellule malade est une cellule cancéreuse.

15. Complexe de la revendication 14, dans lequel ladite cellule cancéreuse est choisie dans le groupe constitué des carcinomes, mélanomes, lymphomes et sarcomes.

16. Complexe de l'une quelconque des revendications 1 et 2, dans lequel ladite cellule cible est une cellule infectée par un virus pathogène.

17. Complexe de la revendication 16, dans lequel ledit virus pathogène est l'un quelconque parmi le virus de la grippe, de l'immunodéficience humaine, d'Epstein-Barr, le cytomégalovirus, le virus de la vaccine, MVM, ECMV et le virus de l'Herpes.

18. Complexe de la revendication 17, dans lequel ledit segment de reconnaissance de la cible est capable de se lier à un ligand exprimé à la surface de ladite cellule cible, ladite liaison étant médiée par l'acide sialique.

19. Vecteur d'expression comprenant un ADN codant une protéine de fusion NKp44-Ig ou NKp46-Ig telle qu'indiquée dans la revendication 7 ou 8, ladite protéine comprenant :
(a) un segment de reconnaissance de la cible qui comprend NKp44 ou NKp46 ou des fragments fonctionnels de ceux-ci ; et
(b) un segment actif qui est la partie Fc d'une molécule d'Ig.

20. Vecteur d'expression de la revendication 19, dans lequel ledit fragment de NKp46 comprend au moins un des domaines 1 et 2 de la molécule NKp46.

21. Vecteur d'expression de la revendication 20, dans lequel
(a) ledit fragment de NKp46 comprend les domaines 1 et 2 de la molécule NKp46 codée par la séquence d'acide nucléique essentiellement comme décrit par l'une des séquences SEQ ID NO:1 et SEQ ID NO:11 et la partie Fc d'une molécule d'Ig est codée par la séquence d'acide nucléique essentiellement comme décrit par la séquence SEQ ID NO :2 ;
(b) ledit fragment de NKp46 comprend le domaine 2 de la molécule NKp46 codée par la séquence d'acide nucléique essentiellement comme décrit par l'une des séquences SEQ ID NO :19 et SEQ ID NO :20 et la partie Fc d'une molécule d'Ig est codée par la séquence d'acide nucléique essentiellement comme décrit par la séquence SEQ ID NO :2.

22. Vecteur d'expression de la revendication 19, dans lequel ledit fragment de NKp44 comprend au moins un des domaines 1 et 2 de la molécule NKp44.

23. Vecteur d'expression de la revendication 22, dans lequel
(a) ledit fragment de NKp44 comprend les domaines 1 et 2 de la molécule NKp44 codée par la séquence d'acide nucléique essentiellement comme décrit par la séquence SEQ ID NO :7 et la partie Fc d'une molécule d'Ig est codée par la séquence d'acide nucléique essentiellement comme décrit par la séquence SEQ ID NO:2 ; ou
(b) ledit fragment de NKp44 comprend le domaine 2 de la molécule NKp44 codée par la séquence d'acide nucléique essentiellement comme décrit par la séquence SEQ ID NO :21 et la partie Fc d'une molécule d'Ig est codée par la séquence d'acide nucléique essentiellement comme décrit par la séquence SEQ ID NO :2.

24. Cellule hôte transformée par le vecteur d'expression de l'une quelconque des revendications 19 à 23.

25. Protéine de fusion NKp46-Ig codée par un vecteur selon l'une des revendications 19 à 21, comprenant la séquence d'acides aminés essentiellement comme décrit par l'une des séquences SEQ ID NO :6 et SEQ ID NO :14, codées par la séquence d'acide nucléique essentiellement comme décrit par les séquences SEQ ID NO :3 et SEQ ID NO :12, respectivement.

26. Protéine de fusion NKp44-Ig codée par un vecteur selon l'une des revendications 19, 22 ou 23, comprenant la séquence d'acides aminés essentiellement comme décrit par la séquence SEQ ID NO:10, codée par la séquence d'acide nucléique essentiellement comme décrit par la séquence SEQ ID NO :8.

27. Anticorps qui reconnaît spécifiquement et se lie à la protéine de fusion de la revendication 25 ou 26.

28. Composition pour le traitement d'un état pathologique comprenant, comme ingrédient actif, un complexe comprenant :
(a) un segment de reconnaissance de la cible capable de reconnaître spécifiquement et de se lier à une cellule cible malade impliquée dans ledit état pathologique, ledit segment de reconnaissance comprenant NKp46, NKp44, NKp30 ou un fragment biologiquement fonctionnel de celles-ci ; et
(b) un segment actif choisi dans le groupe constitué par une partie cytotoxique et un fragment d'Ig.

29. Composition selon la revendication 28, dans laquelle ledit segment de reconnaissance comprend NKp46 ou un fragment biologiquement fonctionnel de celui-ci.

30. Composition de l'une des revendications 28 et 29, dans laquelle ledit état pathologique est un cancer choisi dans le groupe comprenant des carcinomes, mélanomes, lymphomes et sarcomes.

31. Composition de l'une des revendications 28 et 29, dans laquelle ledit état pathologique est une infection virale causée par l'un quelconque parmi le virus de la grippe, de l'immunodéficience humaine, d'Epstein-Barr, le cytomégalovirus, le virus de la vaccine, MVM, ECMV et le virus de l'Herpes.

32. Composition de la revendication 31, dans laquelle le segment de reconnaissance de la cible comprenant NKp46 ou un fragment fonctionnel de celui-ci est capable de se lier à un ligand exprimé à la surface de ladite cellule cible, ladite liaison étant médiée par l'acide sialique.

33. Composition de la revendication 31, dans laquelle le segment de reconnaissance de la cible comprenant NKp44 ou un fragment fonctionnel de celui-ci est capable de se lier à un ligand exprimé à la surface de ladite cellule cible, ladite liaison étant médiée par l'acide sialique.

34. Composition de l'une des revendications 28 à 32, dans laquelle ledit fragment de NKp46 comprend au moins un des domaines 1 et 2 de la molécule NKp46.

35. Composition de la revendication 34, dans laquelle
(a) ledit fragment de NKp46 comprend les domaines 1 et 2 de la molécule NKp46 et a la séquence d'acides aminés essentiellement comme décrit par l'une des séquences SEQ ID NO :4 et SEQ ID NO : 13 ; ou
(b) ledit fragment de NKp46 comprend le domaine 2 de la molécule NKp46 et a la séquence d'acides aminés essentiellement comme décrit par l'une des séquences SEQ ID NO :22 et SEQ ID NO :23

36. Composition de l'une des revendications 28, 30, 31 et 33, dans laquelle ledit fragment de NKp44, comprend au moins un des domaines 1 et 2 de la molécule NKp44.

37. Composition de la revendication 36, dans laquelle
(a) ledit fragment de NKp44 comprend les domaines 1 et 2 de la molécule NKp44 et a la séquence d'acides aminés essentiellement comme décrit par la séquence SEQ ID NO :9 ; ou
(b) ledit fragment de NKp44 comprend le domaine 2 de la molécule NKp44 et a la séquence d'acides aminés essentiellement comme décrit par la séquence SEQ ID NO :24.

38. Composition de l'une des revendications 35 à 37, dans laquelle ledit segment actif est un fragment d'Ig qui est une partie Fc d'une molécule d'Ig codée par la séquence d'acides aminés essentiellement comme décrit par la séquence SEQ ID NO :5.

39. Composition de la revendication 28, dans laquelle ledit segment actif est un fragment d'Ig qui est une partie Fc d'une molécule d'Ig codée par la séquence d'acides aminés essentiellement comme décrit par la séquence SEQ ID NO :5.

40. Composition de l'une des revendications 35 à 37, dans laquelle ledit segment actif est une partie cytotoxique, choisie parmi les cytotoxines ou agents anticellulaires capables de tuer et/ou de supprimer la croissance et/ou la division cellulaire de ladite cellule cible.

41. Composition de la revendication 28, dans laquelle ledit segment actif est une partie cytotoxique, choisie parmi les cytotoxines ou agents anticellulaires capables de tuer et/ou de supprimer la croissance et/ou la division cellulaire de ladite cellule cible.

42. Composition de l'une des revendications 40 et 41, dans laquelle ledit agent cytotoxique ou anticellulaire est une toxine quelconque dérivée de plantes, de champignons ou de bactéries ou une toxine synthétique.

43. Composition de la revendication 42, dans laquelle ladite toxine est choisie dans le groupe constitué par la toxine à chaîne A, la protéine d'inactivation du ribosome, l'α-sarcine, l'aspergilline, la restrictocine, la ribonucléase, la toxine de la diphtérie, l'exotoxine de *Pseudomonas,* une endotoxine ou la partie lipide A d'une endotoxine.

44. Composition diagnostique pour détecter la présence de cellules malades dans un échantillon comprenant un complexe comprenant :
(a) une cellule cible impliquée dans ledit état pathologique, ledit segment de reconnaissance comprenant l'une des molécule NKp46, NKp44, NKp30 ou un fragment biologiquement fonctionnel de celles-ci ; et
(b) un segment d'imagerie détectable qui est un groupement d'imagerie choisi dans le groupe constitué des agents paramagnétiques, radioactifs et fluorogéniques.

45. Composition diagnostique selon la revendication 44, dans laquelle ledit segment de reconnaissance comprend NKp46 ou un fragment biologiquement fonctionnel de celui-ci

46. Composition diagnostique de la revendications 45, dans laquelle ledit fragment de NKp46 comprend au moins l'un des domaines 1 et 2 de la molécule NKp46.

47. Composition diagnostique de la revendication 46, dans laquelle
(a) ledit fragment de NKp46 comprend les domaines 1 et 2 de la molécule NKp46 et a la séquence d'acides aminés essentiellement comme décrit par l'une des séquences SEQ ID NO :4 et SEQ ID NO:13 ; ou
(b) ledit fragment de NKp46 comprend le domaine 2 de la molécule NKp46 et a la séquence d'acides aminés essentiellement comme décrit par l'une des séquences SEQ ID NO :22 et SEQ ID NO :23.

48. Composition diagnostique de la revendication 44, dans laquelle ledit fragment de NKp44, comprend au moins l'un des domaines 1 et 2 de la molécule NKp44.

49. Composition diagnostique de la revendication 48, dans laquelle
(a) ledit fragment de NKp44 comprend les domaines 1 et 2 de la molécule NKp44 et a la séquence d'acides aminés essentiellement comme décrit par la séquence SEQ ID NO :9 ; ou
(b) ledit fragment de NKp44 comprend le domaine 2 de la molécule NKp44 et a la séquence d'acides aminés essentiellement comme décrit par la séquence SEQ ID NO:24.

50. Composition diagnostique de l'une des revendications 44 à 49, dans laquelle le segment de reconnaissance de la cible est capable de se lier à un ligand exprimé à la surface de ladite cellule cible.

51. Composition diagnostique de la revendication 50, dans laquelle ladite cible est une cellule cancéreuse.

52. Composition diagnostique de la revendication 51, dans lequel ledit cancer est un cancer choisi dans le groupe constitué des carcinomes, mélanomes, lymphomes et sarcomes.

53. Utilisation d'un complexe ou d'un agent pharmaceutique contenant celui-ci, ledit complexe comprenant :
(a) un premier segment de reconnaissance de la cible capable de reconnaître spécifiquement et de se lier à une cellule cible malade impliquée dans ledit état pathologique, ledit segment de reconnaissance comprenant l'une des molécules NKp46, NKp44, NKp30 ou un fragment biologiquement fonctionnel de ceux-ci ; et
(b) un segment actif choisi dans le groupe constitué par une partie cytotoxique et un fragment d'Ig
pour la préparation d'une composition pharmaceutique pour le traitement d'un état pathologique chez un sujet, dans laquelle l'état pathologique est choisi dans le groupe constitué par :
(i) un cancer choisi dans le groupe constitué des carcinomes, mélanomes, lymphomes et sarcomes ; et
(ii) une infection virale causée par l'un quelconque parmi le virus de la grippe, de l'immunodéficience humaine, d'Epstein-Barr, le cytomégalovirus, le virus de la vaccine, MVM, ECMV et le virus de l'Herpes.

54. Utilisation selon la revendication 53, dans laquelle ledit complexe comprend :
(a) un premier segment de reconnaissance de la cible capable de reconnaître spécifiquement et de se lier à une cellule cible malade impliquée dans ledit état pathologique, ledit segment de reconnaissance comprenant NKp46 ou un fragment biologiquement fonctionnel de celui-ci ; et
(b) un segment actif choisi dans le groupe constitué par une partie cytotoxique et un fragment d'Ig.

55. Utilisation selon l'une des revendications 53 ou 54, dans laquelle le segment de reconnaissance de la cible NKp46 ou un fragment fonctionnel de celui-ci est capable de se lier à un ligand exprimé à la surface de ladite cellule cible, ladite liaison étant médiée par l'acide sialique.

56. Utilisation selon la revendication 53, dans laquelle le segment de reconnaissance de la cible NKp44 ou un fragment fonctionnel de celui-ci est capable de se lier à un ligand exprimé à la surface de ladite cellule cible, ladite liaison étant médiée par l'acide sialique.

57. Utilisation selon l'une quelconque des revendications 53 à 55, dans laquelle ledit fragment de NKp46 comprend au moins l'un des domaines 1 et 2 de la molécule NKp46.

58. Utilisation selon la revendication 57, dans laquelle
(a) ledit fragment de NKp46 comprend les domaines 1 et 2 de la molécule NKp46 et a la séquence d'acides aminés essentiellement comme décrit par l'une des séquences SEQ ID NO :4 et SEQ ID NO :13 ; ou
(b) ledit fragment de NKp46 comprend le domaine 2 de la molécule NKp46 et a la séquence d'acides aminés essentiellement comme décrit par l'une des séquences SEQ ID NO :22 et SEQ ID NO :23.

59. Utilisation selon l'une des revendications 53 ou 56, dans laquelle ledit fragment de NKp44 comprend au moins l'un des domaines 1 et 2 de la molécule NKp44.

60. Utilisation selon la revendication 59, dans laquelle
(a) ledit fragment de NKp44 comprend les domaines 1 et 2 de la molécule NKp44 et a la séquence d'acides aminés essentiellement comme décrit par la séquence SEQ ID NO :9.
(b) ledit fragment de NKp44 comprend le domaine 2 de la molécule NKp44 et a la séquence d'acides aminés essentiellement comme décrit par la séquence SEQ ID NO :24.

61. Utilisation selon l'une des revendications 58 et 60, dans laquelle ledit segment actif est un fragment d'Ig qui est une partie Fc d'une molécule d'Ig codée par la séquence d'acides aminés essentiellement comme décrit par la séquence SEQ ID NO :5.

62. Utilisation selon l'une des revendications 58 et 60, dans laquelle ledit segment actif est une partie cytotoxique, choisie parmi les cytotoxines ou agents anticellulaires capables de tuer et/ou de supprimer la croissance et/ou la division cellulaire de ladite cellule cible.

63. Utilisation selon la revendication 62, dans laquelle ledit agent cytotoxique ou anticellulaire est une toxine quelconque dérivée de plantes, de champignons ou de bactéries ou une toxine synthétique.

64. Utilisation selon la revendication 63, dans laquelle ladite toxine est choisie dans le groupe constitué par la toxine à chaîne A, la protéine d'inactivation du ribosome, l'α-sarcine, l'aspergilline, la restrictocine, la ribonucléase, la toxine de la diphtérie, l'exotoxine de *Pseudomonas,* une endotoxine ou la partie lipide A d'une endotoxine.

65. Utilisation d'un agent d'imagerie comprenant un complexe ayant :
(a) un premier segment de reconnaissance, capable de reconnaître spécifiquement et de se lier aux dites cellules malignes, ledit segment de reconnaissance comprenant l'une des molécules NKp46, NKp44, NKp30 ou un fragment biologiquement fonctionnel de celles-ci ; et
(b) un segment actif qui est une groupement d'imagerie, ledit groupement d'imagerie étant choisi dans le groupe constitué par les agents paramagnétiques, radioactifs et fluorogéniques
pour la préparation d'une composition diagnostique pour le diagnostique et la visualisation de cellules malignes chez un sujet, dans laquelle lesdits diagnostique et visualisation comprennent l'introduction dudit agent d'imagerie dans la circulation sanguine dudit sujet, et la détection et la quantification de la liaison dudit agent d'imagerie à l'un des ligands de NKp46, NKp30 et NKp44 exprimés sur les cellules malignes cibles.

66. Utilisation d'un agent d'imagerie selon la revendication 65, dans laquelle ledit segment de reconnaissance comprend NKp46 ou un fragment biologiquement fonctionnel de celui-ci et dans laquelle la liaison dudit agent d'imagerie aux ligands de NKp46 exprimés sur les cellules malignes cibles est détecté et quantifié.

67. Utilisation selon la revendication 66, dans laquelle ledit fragment de NKp46 comprend au moins un des domaines 1 et 2 de la molécule NKp46.

68. Utilisation selon la revendication 67, dans laquelle
(a) ledit fragment de NKp46 comprend les domaines 1 et 2 de la molécule NKp46 et a la séquence d'acides aminés essentiellement comme décrit par l'une des séquences SEQ ID NO :4 et SEQ ID NO:13 ; ou
(b) ledit fragment de NKp46 comprend le domaine 2 de la molécule NKp46 et a la séquence d'acides aminés essentiellement comme décrit par l'une des séquences SEQ ID NO :22 et SEQ ID NO :23.

69. Utilisation selon la revendication 65, dans laquelle ledit fragment de NKp44 comprend au moins l'un des domaines 1 et 2 de la molécule NKp44.

70. Utilisation selon la revendication 69, dans laquelle
(a) ledit fragment de NKp44 comprend les domaines 1 et 2 de la molécule NKp44 et a la séquence d'acides aminés essentiellement comme décrit par la séquence SEQ ID NO :9 ; ou
(b) ledit fragment de NKp44 comprend le domaine 2 de la molécule NKp44 et a la séquence d'acides aminés essentiellement comme décrit par la séquence SEQ ID NO :24.

71. Utilisation selon l'une des revendications 65 à 70, dans laquelle lesdites cellules malignes sont choisies dans le groupe constitué des carcinomes, mélanomes, lymphomes et sarcomes.
